# EUROPEAN PATENT APPLICATION

(11) **EP 4 851 289 A1**
(43) Date of publication of application: **09.12.2026**
(21) Application number: 25749195.1
(22) Date of filing: 31.01.2025
(51) Int. Cl.: C12N 15/113, A61K 31/7088, A61K 48/00, A61P 35/00, A61P 25/28, A61P 43/00

(54) **COMPOUND FOR REGULATING ACTIVITY OR EXPRESSION OF MTOR AND USE THEROF**

(30) Priority: 30.01.2024 KR 20240014189; 14.03.2024 KR 20240036041
(71) Applicant: Sovargen Co., Ltd., Daejeon 34051 (KR)
(72) Inventor: PARK, Sang Min, Daejeon 34051 (KR); CHUN, Hein, Daejeon 34051 (KR); LEE, Seung Jin, Daejeon 34051 (KR); JU, Young Hee, Daejeon 34051 (KR); GONG, Byeong Uk, Daejeon 34051 (KR); KOOK, Jiae, Daejeon 34051 (KR); PARK, Eun Jung, Daejeon 34051 (KR); AHN, Sunyoung, Daejeon 34051 (KR); CHOE, Eun Ji, Daejeon 34051 (KR); SHIN, Eun Ji, Daejeon 34051 (KR); HA, Ji Sun, Daejeon 34051 (KR); LEE, Sangmee, Daejeon 34051 (KR); LEE, Chansong, Daejeon 34051 (KR); OH, Eun Ju, Daejeon 34051 (KR)
(74) Representative: Vossius & Partner Patentanwälte Rechtsanwälte mbB
(86) International application number: PCT/KR2025/001593
(87) International publication number: WO 2025/165154

(57) **Abstract**

The present invention relates to a compound for modulating the expression level and/or activity of an mTOR gene and a composition comprising the same, wherein the compound and composition are useful for preventing, treating, or alleviating diseases, disorders, and conditions related to mTOR.

## Description

### [Technical Field]

The present invention relates to RNA-targeted therapeutics for modulating the expression level and/or activity of the mTOR gene, and more particularly to compounds and compositions comprising the same, wherein said compounds and compositions are useful for preventing, ameliorating or treating diseases, disorders, and conditions associated with mTOR.

### [Background Art]

The mammalian (or mechanistic) target of rapamycin (mTOR) pathway plays an important role in regulating a variety of biological processes that are central to cellular life, aging, and death. Given that mTOR is ubiquitously distributed across diverse cell types throughout the body, the mTOR pathway controls multiple anabolic and catabolic processes in distinct organs/tissues including, but not limited to, the liver, lymphocytes, white and brown adipose tissue, skeletal muscle, and brain. Accordingly, impaired mTOR activity, particularly hyperactivation thereof, has been associated with a broad spectrum of human diseases including cancer, type 2 diabetes, cardiovascular pathology, neurological disease, and aging.

mTOR is a 289 kDa protein kinase encoded in humans by the MTOR gene (chromosome 1p36.2). MTOR is an intracellular serine/threonine kinase that plays a critical role in mediating appropriate physiological function in vivo. MTOR forms complexes with other proteins to mediate intracellular signal transduction; depending on the identity of the constituent proteins, these complexes are classified as MTOR complex 1 (MTORC1) or MTOR complex 2 (MTORC2). MTORC1 contains RAPTOR (regulatory-associated protein of mTOR) as a component and plays an important role in cell growth and proliferation by regulating ribosome biogenesis and protein expression. MTORC2 contains RICTOR (rapamycin-insensitive companion of mTOR) as a component and plays an important role in intracellular cytoskeletal remodeling and cell migration. The mTOR-containing complexes also differ with respect to their upstream regulators, substrate specificity, functional output, and sensitivity to rapamycin. Sensitivity to rapamycin represents another key feature that distinguishes the mTORC1 and mTORC2 complexes. Rapamycin does not directly inhibit the catalytic (kinase) activity of mTOR; instead, it binds to the immunophilin FKBP12 (FK506-binding protein of 12 kDa), a protein that binds to the mTOR FKBP-rapamycin binding domain (FRB). Although the FRB domain is present in both mTORC1 and mTORC2 complexes, it is exposed only in mTORC1 because Rictor blocks access of the FKBP12-rapamycin complex to the FRB domain of mTORC2. Consequently, mTORC2 is relatively resistant to the effects of rapamycin both in vitro and in vivo.

### [Detailed Description of the Invention]

### [Technical Problem]

An embodiment of the present invention relates to RNA-targeted therapeutics for modulating an activity and/or expression level of MTOR, including, for example, RNAi agents, antisense oligonucleotides, miRNAs, and the like.

An embodiment of the present invention relates to a compound for modulating an activity and/or expression level of MTOR, and a pharmaceutical composition, a method, or use for preventing, ameliorating, and/or treating diseases, disorders, and conditions associated with MTOR, comprising the compound as active ingredient.

A further embodiment of the present invention relates to a composition, a method, or use for modulating the activity and/or expression level of MTOR by using a compound for modulating the activity and/or expression level of MTOR.

The compounds for modulating an activity and/or expression level of MTOR may be oligomeric compounds and/or RNAi agents, wherein the oligomeric compounds may be antisense oligonucleotides, and wherein the RNAi agents may include, but are not limited to, ssRNAi agents, siRNA, shRNA, and miRNA.

### [Technical Solution]

An embodiment of the present invention relates to a compound for modulating an activity and/or expression level (amount) of MTOR, and a pharmaceutical composition or method for preventing, ameliorating and/or treating diseases, disorders, and conditions associated with MTOR, comprising said compound as an active ingredient. Additionally, an embodiment of the present invention relates to a compound for modulating the activity and/or expression level (amount) of MTOR, and a pharmaceutical composition or method for preventing, ameliorating, and/or treating diseases, disorders, and conditions mediated by MTOR, comprising said compound as an active ingredient.

A specific embodiment of the present invention relates to a method for preventing, ameliorating, and/or treating diseases, disorders, and conditions associated with MTOR, comprising a step of administering a compound for modulating the activity and/or expression level of MTOR to an individual or patient in need thereof. As used herein, the term "expression" refers to a process by which a polynucleotide produces a gene product, e.g., RNA or polypeptide (or protein, etc.).

More specifically, the present invention relates to a pharmaceutical composition or a method for preventing, ameliorating, and/or treating diseases, disorders, and conditions caused by the hyperactivation or overexpression of MTOR.

In the present disclosure, "individual," "patient," or "subject" means a human or non-human animal selected for treatment or therapy, and non-human animals may include, but are not limited to, mammals such as monkeys, rats, mice, rabbits, guinea pigs, and the like.

A specific embodiment of the present invention relates to a compound for modulating the activity and/or expression level (amount) of MTOR, an oligomer compound and/or RNAi agent. An embodiment provides a compound, a method, and a composition for inhibiting expression of MTOR mRNA and protein, inhibiting the activity of MTOR protein, or reducing levels of mRNA and protein. In specific embodiments, the compound for modulating the activity and/or expression level (amount) of MTOR may be an inhibitor that reduces or inhibits an activity and/or expression level (amount) of MTOR.

Hyperactivation of the MTOR signaling pathway can occur due to mutations in the MTOR gene itself or genes upstream of MTOR, or due to a disruption in the homeostasis of other signaling pathways. For example, focal cortical dysplasia (FCD), a representative disease of intractable pediatric epilepsy, is classified to a disease of FMCD (Focal Malformation of Cortical Development) together with unilateral hemimegalencephaly (HME), and the genetic cause of these diseases has been well known due to brain-specific somatic mutations that induce hyperactivation of the MTOR signaling pathway.

In particular, the mutations in the MTOR gene itself, which account for approximately 60% of the various gene variations found in patients with FCD type II disease, can alter the structure of the MTOR protein, leading to the hyperactivation of both MTORC1 and MTORC2. It is well known from prior literature that both of the MTORC1 and MTORC2 complexes can cause intractable FMCD epilepsy.

The MTOR-targeting antisense oligonucleotide (ASO) of the present invention reduces the expression level of MTOR by degrading the mRNA of MTOR gene. This reduces the expression level of MTOR itself, which is a common component of the two complexes, thereby inducing inhibition of the activity of both MTORC1 and MTORC2 complexes, and thus can be used as a therapeutic drug for intractable FMCD epilepsy. In particular, MTOR proteins modified by mutations may respond differently to MTOR inhibitors of the small molecule compound class, but the MTOR antisense oligonucleotide of the present invention can overcome this.

The compound according to the present invention may be used for a disease, disorder, and condition associated with MTOR, or more specifically a disease, a disorder, and a condition caused by the hyperactivation or overexpression of MTOR, and the hyperactivation or overexpression of MTOR may be caused by a gene alteration of the mTOR gene or genes in a signaling pathway including it, and such gene alterations include both germline alterations and somatic alterations.

Examples of the diseases, disorders, or pathological conditions may include tumors, neurological disorders, and kidney diseases. The neurological disorders include neurodevelopmental disorders and neurodegenerative disorders. The neurodevelopmental disorders may include mTORpathies, epilepsy and seizure-related disorders, and the like. The mTORpathies include focal cortical dysplasia (FCD) caused by somatic cell mutations, such as FCD type II, and hemimegalencephaly (HME), as well as Tuberous Sclerosis Complex (TSC), megalencephaly (MEG), Cowden Syndrome, PIK3CA-Related Overgrowth Spectrum (PROS), Bannayan-Riley-Ruvalcaba Syndrome (BRRS), Lhermitte-Duclos Disease (LDD), MCAP (Megalencephaly-Polydactyly-Polymicrogyria-Hydrocephalus), MPPH (Megalencephaly-Polymicrogyria-Hydrocephalus), Smith-Kingsmore Syndrome (SKS), Polyhydramnios, Megalencephaly and Symptomatic Epilepsy Syndrome (PMSE), and Familial Intellectual Disability-Macrocephaly Syndrome, and Proteus Syndrome. The neurodevelopmental disorders include Fragile X-Syndrome (FXS) and Autism Spectrum Disorder (ASD), etc. Specifically, the neurodevelopmental disorders include Malformations of Cortical Development (MCDs) which examples of MCDs are tuberous sclerosis complex (TSC), Focal Cortical Dysplasia (FCD) such as Focal Cortical Dysplasia Type II (FCDII), HME, and the like, and symptoms related to MCD include epilepsy, seizures, etc.

The epilepsy and seizure-related disorders include Familial Focal Epilepsy with Variable Foci (FFEVF), Autosomal Dominant Nocturnal Epilepsy (ADNFLE), Familial Mesial Temporal Lobe Epilepsy (FMTLE), Temporal Lobe Epilepsy (TLE), and Infantile-Onset Developmental and Epileptic Encephalopathies (DEE).

The neurodegenerative diseases include Alzheimer's disease (AD), Parkinson's disease (PD), Huntington's disease (HD), amyotrophic lateral sclerosis (ALS), etc.

The tumors include solid tumors and neuro-oncological tumors, and solid tumors include lung cancer, colorectal cancer, gastric cancer, renal cell carcinoma, bladder cancer, prostate cancer, and breast cancer. The above neuro-oncological tumors include ganglioma, glioblastoma, dysembryoplastic neuroepithelial tumor (DNET), subependymal giant cell astrocytomas (SGCAs/SEGAs), and the like.

The kidney diseases may include kidney transplantation, polycystic kidney disease (PKD), renal carcinoma, and diabetic nephropathy. Specifically, the examples are autosomal dominant polycystic kidney disease (ADPKD) or autosomal recessive polycystic kidney disease (ARPKD), which is a genetic disease characterized by the formation of kidney cysts and is known to be caused by mutations in two genes of PKD1 and PKD2 encoding polycystin-1 (PC-1) and polycystin-2 (PC-2), respectively. Additionally, ARPKD is known to occur due to mutations in the PKHD1 gene. Preclinical results have been reported showing that MTOR inhibitors delay cyst growth and preserve kidney function in patients with PKD. Accordingly, the present compound can be used for kidney diseases associated with MTOR overactivation or overexpression, such as polycystic kidney disease or its symptoms, such as delaying cyst growth, preserving kidney function, etc.

The compound for modulating the activity and/or expression level of MTOR in the present invention is, specifically, a compound useful for modulating the activity of mTOR complex 1 (mTORC1) and/or mTOR complex 2 (mTORC2). In particular, the compound of the present invention targets the MTOR gene and modulates the activity and/or expression of the MTOR protein, which is common to both mTORC1 and mTORC2, thereby enabling the regulation of both mTORC1 and mTORC2. In particular, mutations in the MTOR gene itself, which account for approximately 60% of the various gene variations found in patients with FCD type II disease, induce the altered structure of the MTOR protein, thereby leading to the hyperactivation of both mTORC1 and mTORC2. Since both the mTORC1 and mTORC2 complexes can cause FMCD intractable epilepsy, the compound of the present invention has the advantage of being able to act on all FMCD diseases caused by various genetic variations.

As used in the present disclosure, "target nucleic acid" and "target RNA" refer to a nucleic acid for which an antisense compound is designed to exert an effect. "Target RNA" refers to an RNA transcript and includes pre-mRNA and mRNA, unless otherwise specified. In the present disclosure, "RNA" refers to an RNA transcript and includes both pre-mRNA and mature mRNA unless otherwise specified.

As used in the present disclosure, "target region" refers to a portion of the target nucleic acid to which an oligomeric compound is designed to hybridize thereto. Here, the "target nucleic acid" and "target RNA" are MTOR. In the present disclosure, the MTOR gene may be a human MTOR gene sequence, and include a known reference transcript or a variant thereof, which are available in various gene databases. Specifically, the MTOR nucleic acid sequence, e.g., the human MTOR nucleic acid sequence, can be identified by the NCBI (National Center for Biotechnology Information) reference sequence Nucleotide ID NG_033239.1, and the genomic location is 11,106,535 to 11,262,551 on chromosome 1 (corresponding to nucleotides 11,106,535 to 11,262,551 of the NCBI reference sequence Nucleotide ID NC_000001.11 or Ensembl transcript ID: ENST00000361445.9). The coordinates used herein refer to the coordinates of the genome reference assembly GRCh38 (Genome Reference Consortium Human Build 38), known as Hg38 (Human Genome Build 38).

The present invention relates to RNA-targeted therapeutic agents for modulating the activity and/or expression levels of MTOR, and includes, for example, RNAi agents, antisense oligonucleotides, miRNAs, etc. Specifically, the compound of the present invention includes, but is not limited to, an antisense oligonucleotide and an RNA interference agent (RNAi agent) for MTOR nucleic acid. The RNA-targeted therapeutic agent may be an oligonucleotide comprising 8 to 80 nucleosides or RNAi agent having a sequence complementary to the human MTOR nucleic acid sequence.

The RNAi agent refers to an antisense agent that acts partially via RISC or Ago2 to regulate a target nucleic acid and/or a protein encoded by the target nucleic acid. RNAi agents include, but are not limited to, double-stranded siRNA, single-stranded RNAi (ssRNAi), and microRNAs including microRNA mimics. RNAi agents are not intended to include antisense oligonucleotides.

An embodiment of the RNAi agent may be a double-stranded RNAi agent, which may be siRNA and may include an antisense strand complementary to a target gene and a sense strand complementary to the antisense strand, and may optionally include one or more lipophilic moiety conjugated to one or more internal positions of one strand, optionally via a linker or carrier. The sense and antisense strands may each be 19 to 25 nucleotides in length, but are not limited thereto.

Specifically, the targeted nucleobase sequence of MTOR gene (Ensembl Transcript ID: ENST00000361445.9) for complementary hybridization may be GAATGTTGACCAATGCTAT (SEQ No. 464), GGTCTGAGTTTAAGGTCTA (SEQ No. 465), or GGTCTGAGTTTAAGGTCTA (SEQ No. 465), and examples of the sense sequence and antisense sequence of the siRNA acting on these target sequence regions may be the nucleobase sequences described in SEQ Nos. 456 to 463, but are not limited thereto.

The lipophilic moiety may be an aliphatic, alicyclic, or polyalicyclic compound, and specifically lipid, cholesterol, retinoic acid, cholic acid, adamantane acetic acid, 1-pyrene butyric acid, dihydrotestosterone, 1,3-bis-O(hexadecyl)glycerol, geranyloxyhexianol, hexadecylglycerol, borneol, menthol, 1,3-propanediol, heptadecyl group, palmitic acid, myristic acid, O3-(oleoyl)ritocholic acid, O3-(oleoyl)cholenic acid, dimethoxytrityl, or phenoxazine, or may contain functional group selected from the group consisting of a saturated or unsaturated C4-C30 hydrocarbon chain, hydroxyl, amine, carboxylic acid, sulfonate, phosphate, thiol, azide, and alkyne. More specifically, the lipophilic moiety may contain saturated or unsaturated C6-C18 hydrocarbon chains, and details thereof may be found WO2019217459A1, etc.

The MTOR inhibitor of the present invention may comprise a conjugate group and/or a terminal group. In specific examples, the MTOR inhibitor modulates the amount and/or activity of the target nucleic acid. The conjugate moiety modifies one or more properties of the attached oligonucleotide, including but not limited to pharmacodynamics, pharmacokinetics, stability, binding, absorption, tissue distribution, cell distribution, cell absorption, charge, and removal. In specific examples, the conjugate moiety imparts new properties to the attached oligonucleotide. As used in the present disclosure, "conjugate group" refers to a group of atoms directly attached to the oligonucleotide. The conjugate group includes a conjugate moiety and a conjugate linker that attaches the conjugate moiety to the oligonucleotide.

In the present disclosure, the term "inhibiting expression or activity" indicates a reduction or inhibition of expression or activity compared to expression or activity in untreated or control samples, and does not necessarily indicate the complete elimination of expression or activity overall. Specifically, the antisense oligomer or RNAi agent according to the present invention may be capable of reducing MTOR expression levels to 70% or less, 65% or less, 60% or less, or 50% or less.

In specific examples, oligomer compounds and oligomer duplexes hybridize to target nucleic acid and can generate at least one antisense activity; these oligomer compounds and oligomer duplexes are antisense compounds. In specific examples, antisense compounds have antisense activity when they reduce or inhibit the amount or activity of target nucleic acid by more than 25% in a standard cell assay. In specific examples, antisense compounds selectively affect one or more target nucleic acids.

In a specific example, the MTOR inhibitor may reduce the level of activity or expression of MTOR RNA or MTOR Protein by about 5% or more, about 10% or more, about 15% or more, about 20% or more, about 25% or more, about 30% or more, about 35% or more, about 40% or more, about 45% or more, about 50% or more, about 55% or more, about 60% or more, about 65% or more, about 70% or more, about 75% or more, about 80% or more, about 85% or more, about 90% or more, or about 95% or more, based on the control group before treatment and the control group not treated with the MTOR inhibitor, which is set at 0%. Alternatively, the activity or expression level of MTOR RNA or MTOR Protein after treatment with the MTOR inhibitor may be about 95% or less, about 90% or less, about 85% or less, about 80% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, or about 5% or less, based on the control group before treatment and the control group not treated with the MTOR inhibitor, which is set on 100%.

The MTOR inhibitor of the present invention may be an antisense oligonucleotide, and an antisense oligonucleotide means an oligonucleotide having a nucleic acid sequence complementary to a target nucleic acid or a region or segment thereof. In specific examples, the antisense oligonucleotide is capable of specifically hybridizing to a target nucleic acid or a region or segment thereof. As used herein, "antisense oligonucleotide" means an oligonucleotide comprising an oligonucleotide portion of an antisense compound capable of hybridizing to a target nucleic acid and having at least one antisense activity. The terms "antisense oligomer," "antisense compound," "antisense oligonucleotide," or "antisense RNase H oligonucleotide" are used interchangeably and each has a base-pair forming portion connected by nucleobase linkage that causes the base-pair portion to hybridize. In these embodiments, the region of the oligonucleotide has a nucleobase sequence complementary to the target nucleobase sequence. In a specific example, a region or the entire length of the nucleobase sequence of the oligonucleotide is complementary to the target gene or target region and has sequence identity of least 50%, at least 60%, at least 70%, at least 80%, at least 85%, at least 90%, at least 95%, or at least 100%. The term "antisense RNase H oligonucleotide" refers to an oligonucleotide comprising a region that is complementary to a target sequence and comprises at least one chemical modification suitable for RNase H-mediated nucleic acid reduction.

In the present disclosure, a compound for modulating the activity and/or expression level (amount) of MTOR, e.g., an oligonucleotide, includes a pharmaceutically acceptable salt thereof, wherein the "pharmaceutically acceptable salt" means a physiologically and pharmaceutically acceptable salt of a compound, e.g., an oligomer compound or an oligonucleotide, i.e., a salt that retains the desired biological activity of the parent compound and does not cause undesirable toxicological effects.

An embodiment relates to an oligomer compound comprising a modified oligonucleotide composed of 8 to 80 linked nucleosides, wherein the nucleic acid sequence of the modified oligonucleotide has at least 80% sequence identity to a sequence complementary to an equal length portion of the MTOR nucleic acid sequence, and the modified oligonucleotide comprises at least one modification selected from the group consisting of a modified sugar moiety, a modified internucleoside linkage and a modified nucleobase.

Another embodiment relates to an oligomer compound comprising a modified oligonucleotide composed of 8 to 80 linked nucleosides, wherein the nucleic acid sequence of the modified oligonucleotide comprises 12, 13, 14, 15, 16, 17, 18, 19, or 20 contiguous nucleobases among the nucleic acid sequences of SEQ ID NOs 1 to 439, and the modified oligonucleotide comprises at least one modification selected from the group consisting of a modified sugar moiety, a modified internucleoside linkage and a modified nucleobase.

An "oligomer compound" means a compound comprising a single oligonucleotide and, optionally, one or more additional feature, e.g., a conjugate group or a terminal group. An "oligonucleotide" means a polymer of linked nucleosides, each of which is independently modified or unmodified. Unless otherwise specified, the oligonucleotide comprises at least one subunit set selected from the group consisting of 8 to 80, 8 to 70, 8 to 60, 8 to 50, 8 to 40, 8 to 30, 8 to 25, 8 to 22, 8 to 20, 10 to 80, 10 to 70, 10 to 60, 10 to 50, 10 to 40, 10 to 30, 10 to 25, 10 to 22, 10 to 20, 12 to 80, 12 to 70, 12 to 60, 12 to 50, 12 to 40, 12 to 30, 12 to 25, 12 to 22, 12 to 20, 14 to 80, 14 to 70, 14 to 60, 14 to 50, 14 to 40, 14 to 30, 14 to 25, 14 to 22, 14 to 20, 16 to 80, 16 to 70, 16 to 60, 16 to 50, 16 to 40, 16 to 30, 16 to 25, 16 to 22, and 16 to 20 subunits.

The length of an oligonucleotide can be increased or decreased without removing activity. For example, in the literature by Woolf et al. (Proc. Natl. Acad. Sci. USA 89:7305-7309, 1992), a series of oligonucleotides with nucleotide lengths of 13 to 25 were tested for their ability to induce cleavage of target RNA in an oocyte injection model. An oligonucleotide of 25 nucleobases in length having 8 or 11 mismatched bases near the terminus of the oligonucleotide can direct specific cleavage of the target RNA, albeit to a lesser extent than an oligonucleotide containing no mismatches.

As used in the present disclosure, in the context of oligonucleotides, "contiguous" refers to nucleosides, nucleobases, sugar moieties, or linkages between nucleosides that are immediately contiguous to each other (internucleoside linkage). For example, " contiguous nucleobases" means nucleobases that are immediately contiguous to each other in a sequence.

As an exemplary oligomer compound of the present invention, nucleobase sequences that bind complementarily to human MTOR sequences are shown in Tables 1 to 4 below, and the start point and end point in Tables 1 to 4 below represent the base positions of MTOR nucleic acid (Ensembl Transcript ID: ENST00000361445.9) provided by Ensembl.

**[Table 1]**

| Compound ID | SEQ ID NO | Start point | End point | Sequence (5' to 3') |
|---|---|---|---|---|
| 534001 | 1 | 155,944 | 155,963 | TGGTGTCTAGACATGGCTAC |
| 534002 | 2 | 155,943 | 155,962 | GGTGTCTAGACATGGCTACA |
| 534003 | 3 | 155,942 | 155,961 | GTGTCTAGACATGGCTACAC |
| 534004 | 4 | 155,941 | 155,960 | TGTCTAGACATGGCTACACT |
| 534005 | 5 | 155,940 | 155,959 | GTCTAGACATGGCTACACTT |
| 534006 | 6 | 155,937 | 155,956 | TAGACATGGCTACACTTTAT |
| 534007 | 7 | 155,935 | 155,954 | GACATGGCTACACTTTATAC |
| 534008 | 8 | 155,934 | 155,953 | ACATGGCTACACTTTATACT |
| 534009 | 9 | 155,933 | 155,952 | CATGGCTACACTTTATACTT |
| 534010 | 10 | 155,931 | 155,950 | TGGCTACACTTTATACTTTG |
| 534011 | 11 | 155,930 | 155,949 | GGCTACACTTTATACTTTGT |
| 534012 | 12 | 155,929 | 155,948 | GCTACACTTTATACTTTGTG |
| 534013 | 13 | 155,928 | 155,947 | CTACACTTTATACTTTGTGC |
| 534014 | 14 | 155,927 | 155,946 | TACACTTTATACTTTGTGCA |
| 534015 | 15 | 155,925 | 155,944 | CACTTTATACTTTGTGCATT |
| 534016 | 16 | 155,923 | 155,942 | CTTTATACTTTGTGCATTTA |
| 534017 | 17 | 155,921 | 155,940 | TTATACTTTGTGCATTTAGT |
| 534018 | 18 | 155,920 | 155,939 | TATACTTTGTGCATTTAGTT |
| 534019 | 19 | 155,919 | 155,938 | ATACTTTGTGCATTTAGTTG |
| 534020 | 20 | 155,905 | 155,924 | TAGTTGAGTATTTGTTCTGC |
| 534021 | 21 | 155,904 | 155,923 | AGTTGAGTATTTGTTCTGCT |
| 534022 | 22 | 155,876 | 155,895 | CCAATATGTACCAGACCTTC |
| 534023 | 23 | 155,875 | 155,894 | CAATATGTACCAGACCTTCC |
| 534024 | 24 | 155,874 | 155,893 | AATATGTACCAGACCTTCCC |
| 534025 | 25 | 155,873 | 155,892 | ATATGTACCAGACCTTCCCT |
| 534026 | 26 | 155,872 | 155,891 | TATGTACCAGACCTTCCCTG |
| 534027 | 27 | 155,871 | 155,890 | ATGTACCAGACCTTCCCTGT |
| 534028 | 28 | 155,867 | 155,886 | ACCAGACCTTCCCTGTGTTC |
| 534029 | 29 | 155,866 | 155,885 | CCAGACCTTCCCTGTGTTCA |
| 534030 | 30 | 155,865 | 155,884 | CAGACCTTCCCTGTGTTCAG |
| 534031 | 31 | 155,864 | 155,883 | AGACCTTCCCTGTGTTCAGC |
| 534032 | 32 | 155,863 | 155,882 | GACCTTCCCTGTGTTCAGCA |
| 534033 | 33 | 155,854 | 155,873 | TGTGTTCAGCACCTCCATGA |
| 534034 | 34 | 155,852 | 155,871 | TGTTCAGCACCTCCATGACA |
| 534035 | 35 | 155,851 | 155,870 | GTTCAGCACCTCCATGACAG |
| 534036 | 36 | 155,843 | 155,862 | CCTCCATGACAGTATTTCTG |
| 534037 | 37 | 155,842 | 155,861 | CTCCATGACAGTATTTCTGT |
| 534038 | 38 | 155,819 | 155,838 | CTGAGCCCTTGCTCTAAACA |
| 534039 | 39 | 155,818 | 155,837 | TGAGCCCTTGCTCTAAACAG |
| 534040 | 40 | 155,817 | 155,836 | GAGCCCTTGCTCTAAACAGA |
| 534041 | 41 | 155,816 | 155,835 | AGCCCTTGCTCTAAACAGAG |
| 534042 | 42 | 155,815 | 155,834 | GCCCTTGCTCTAAACAGAGT |
| 534043 | 43 | 155,814 | 155,833 | CCCTTGCTCTAAACAGAGTA |
| 534044 | 44 | 155,813 | 155,832 | CCTTGCTCTAAACAGAGTAT |
| 534045 | 45 | 155,812 | 155,831 | CTTGCTCTAAACAGAGTATT |
| 534046 | 46 | 155,768 | 155,787 | CCCTGCTGCAGAAGGCCAGT |
| 534047 | 47 | 155,715 | 155,734 | TGCATCTGCTCAGCCGAGGC |
| 534048 | 48 | 155,629 | 155,648 | CATCACTGGGTCTGATGGAA |
| 534049 | 49 | 155,628 | 155,647 | ATCACTGGGTCTGATGGAAG |
| 534050 | 50 | 155,625 | 155,644 | ACTGGGTCTGATGGAAGACA |
| 534051 | 51 | 155,588 | 155,607 | TTGGCCTATCTTGCAAACAT |
| 534052 | 52 | 155,584 | 155,603 | CCTATCTTGCAAACATTTCT |
| 534053 | 53 | 155,550 | 155,569 | ACCAGTGAGGTCTTGGGATA |
| 534054 | 54 | 155,491 | 155,510 | GTCTAGGATCCTAATCCATG |
| 534055 | 55 | 155,490 | 155,509 | TCTAGGATCCTAATCCATGT |
| 534056 | 56 | 155,427 | 155,446 | CCCATGTTGAGAGGAGCAAC |
| 534057 | 57 | 155,426 | 155,445 | CCATGTTGAGAGGAGCAACT |
| 534058 | 58 | 155,425 | 155,444 | CATGTTGAGAGGAGCAACTA |
| 534059 | 59 | 155,424 | 155,443 | ATGTTGAGAGGAGCAACTAG |
| 534060 | 60 | 155,418 | 155,437 | AGAGGAGCAACTAGGTCATT |
| 534061 | 61 | 155,417 | 155,436 | GAGGAGCAACTAGGTCATTC |
| 534062 | 62 | 155,416 | 155,435 | AGGAGCAACTAGGTCATTCT |
| 534063 | 63 | 155,415 | 155,434 | GGAGCAACTAGGTCATTCTT |
| 534064 | 64 | 155,414 | 155,433 | GAGCAACTAGGTCATTCTTC |
| 534065 | 65 | 155,413 | 155,432 | AGCAACTAGGTCATTCTTCC |
| 534066 | 66 | 155,412 | 155,431 | GCAACTAGGTCATTCTTCCA |
| 534067 | 67 | 155,411 | 155,430 | CAACTAGGTCATTCTTCCAT |
| 534068 | 68 | 155,409 | 155,428 | ACTAGGTCATTCTTCCATCA |
| 534069 | 69 | 155,408 | 155,427 | CTAGGTCATTCTTCCATCAG |
| 534070 | 70 | 155,407 | 155,426 | TAGGTCATTCTTCCATCAGC |
| 534071 | 71 | 155,405 | 155,424 | GGTCATTCTTCCATCAGCAA |
| 534072 | 72 | 155,404 | 155,423 | GTCATTCTTCCATCAGCAAG |
| 534073 | 73 | 155,403 | 155,422 | TCATTCTTCCATCAGCAAGT |
| 534074 | 74 | 155,402 | 155,421 | CATTCTTCCATCAGCAAGTA |
| 534075 | 75 | 155,401 | 155,420 | ATTCTTCCATCAGCAAGTAC |
| 534076 | 76 | 155,400 | 155,419 | TTCTTCCATCAGCAAGTACT |
| 534077 | 77 | 155,399 | 155,418 | TCTTCCATCAGCAAGTACTT |
| 534078 | 78 | 155,398 | 155,417 | CTTCCATCAGCAAGTACTTA |
| 534079 | 79 | 155,397 | 155,416 | TTCCATCAGCAAGTACTTAT |
| 534080 | 80 | 155,396 | 155,415 | TCCATCAGCAAGTACTTATG |
| 534081 | 81 | 155,395 | 155,414 | CCATCAGCAAGTACTTATGA |
| 534082 | 82 | 155,394 | 155,413 | CATCAGCAAGTACTTATGAT |
| 534083 | 83 | 155,393 | 155,412 | ATCAGCAAGTACTTATGATG |
| 534084 | 84 | 155,390 | 155,409 | AGCAAGTACTTATGATGAGT |
| 534085 | 85 | 155,389 | 155,408 | GCAAGTACTTATGATGAGTT |
| 534086 | 86 | 155,388 | 155,407 | CAAGTACTTATGATGAGTTC |
| 534087 | 87 | 155,387 | 155,406 | AAGTACTTATGATGAGTTCT |
| 534088 | 88 | 155,386 | 155,405 | AGTACTTATGATGAGTTCTC |
| 534089 | 89 | 155,385 | 155,404 | GTACTTATGATGAGTTCTCT |
| 534090 | 90 | 155,383 | 155,402 | ACTTATGATGAGTTCTCTTG |
| 534091 | 91 | 155,382 | 155,401 | CTTATGATGAGTTCTCTTGT |
| 534092 | 92 | 155,378 | 155,397 | TGATGAGTTCTCTTGTGAGT |
| 534093 | 93 | 155,375 | 155,394 | TGAGTTCTCTTGTGAGTTAA |
| 534094 | 94 | 155,373 | 155,392 | AGTTCTCTTGTGAGTTAAGT |
| 534095 | 95 | 155,372 | 155,391 | GTTCTCTTGTGAGTTAAGTC |
| 534096 | 96 | 155,368 | 155,387 | TCTTGTGAGTTAAGTCAAAA |
| 534097 | 97 | 155,367 | 155,386 | CTTGTGAGTTAAGTCAAAAC |
| 534098 | 98 | 155,366 | 155,385 | TTGTGAGTTAAGTCAAAACC |
| 534099 | 99 | 155,365 | 155,384 | TGTGAGTTAAGTCAAAACCC |
| 534100 | 100 | 155,363 | 155,382 | TGAGTTAAGTCAAAACCCGT |
| 534101 | 101 | 155,362 | 155,381 | GAGTTAAGTCAAAACCCGTA |
| 534102 | 102 | 155,361 | 155,380 | AGTTAAGTCAAAACCCGTAT |
| 534103 | 103 | 155,360 | 155,379 | GTTAAGTCAAAACCCGTATT |
| 534104 | 104 | 155,357 | 155,376 | AAGTCAAAACCCGTATTTCT |
| 534105 | 105 | 155,356 | 155,375 | AGTCAAAACCCGTATTTCTA |
| 534106 | 106 | 155,355 | 155,374 | GTCAAAACCCGTATTTCTAA |
| 534107 | 107 | 155,353 | 155,372 | CAAAACCCGTATTTCTAAAG |
| 534108 | 108 | 155,352 | 155,371 | AAAACCCGTATTTCTAAAGT |
| 534109 | 109 | 155,351 | 155,370 | AAACCCGTATTTCTAAAGTT |
| 534110 | 110 | 155,350 | 155,369 | AACCCGTATTTCTAAAGTTA |

**[Table 2]**

| Compound ID | SEQ ID NO | Start point | End point | Sequence (5' to 3') |
|---|---|---|---|---|
| 534111 | 111 | 155,349 | 155,368 | ACCCGTATTTCTAAAGTTAT |
| 534112 | 112 | 155,348 | 155,367 | CCCGTATTTCTAAAGTTATG |
| 534113 | 113 | 155,347 | 155,366 | CCGTATTTCTAAAGTTATGG |
| 534114 | 114 | 155,346 | 155,365 | CGTATTTCTAAAGTTATGGA |
| 534115 | 115 | 155,341 | 155,360 | TTCTAAAGTTATGGATCTTC |
| 534116 | 116 | 155,339 | 155,358 | CTAAAGTTATGGATCTTCTG |
| 534117 | 117 | 155,335 | 155,354 | AGTTATGGATCTTCTGTTCC |
| 534118 | 118 | 155,334 | 155,353 | GTTATGGATCTTCTGTTCCC |
| 534119 | 119 | 155,333 | 155,352 | TTATGGATCTTCTGTTCCCC |
| 534120 | 120 | 155,321 | 155,340 | TGTTCCCCAAAATGAATGGC |
| 534121 | 121 | 155,320 | 155,339 | GTTCCCCAAAATGAATGGCT |
| 534122 | 122 | 155,318 | 155,337 | TCCCCAAAATGAATGGCTTG |
| 534123 | 123 | 155,306 | 155,325 | ATGGCTTGATTTACGTGGTA |
| 534124 | 124 | 155,305 | 155,324 | TGGCTTGATTTACGTGGTAT |
| 534125 | 125 | 155,304 | 155,323 | GGCTTGATTTACGTGGTATT |
| 534126 | 126 | 155,303 | 155,322 | GCTTGATTTACGTGGTATTA |
| 534127 | 127 | 155,302 | 155,321 | CTTGATTTACGTGGTATTAC |
| 534128 | 128 | 155,301 | 155,320 | TTGATTTACGTGGTATTACT |
| 534129 | 129 | 154,352 | 154,371 | CAGCCAATATAGCACTGGCA |
| 534130 | 130 | 154,282 | 154,301 | GAACATCCAAAGTGTCATCA |
| 534131 | 131 | 154,277 | 154,296 | TCCAAAGTGTCATCATGAGA |
| 534132 | 132 | 153,238 | 153,257 | AGCTTATCTCGAACCCTGTT |
| 534133 | 133 | 153,237 | 153,256 | GCTTATCTCGAACCCTGTTA |
| 534134 | 134 | 153,186 | 153,205 | CTGGTTTCACCAAACCGTCT |
| 534135 | 135 | 153,185 | 153,204 | TGGTTTCACCAAACCGTCTC |
| 534136 | 136 | 152,824 | 152,843 | GTTCCACACCGTCCAAAATT |
| 534137 | 137 | 152,823 | 152,842 | TTCCACACCGTCCAAAATTT |
| 534138 | 138 | 148,215 | 148,234 | TGTCCATCAGCCTCCAGTTC |
| 534139 | 139 | 148,110 | 148,129 | TGATTCTGTAGTTGCCATCC |
| 534140 | 140 | 148,109 | 148,128 | GATTCTGTAGTTGCCATCCA |
| 534141 | 141 | 147,707 | 147,726 | GTCAACATTCTTGTTAGTCT |
| 534142 | 142 | 147,706 | 147,725 | TCAACATTCTTGTTAGTCTA |
| 534143 | 143 | 147,670 | 147,689 | GAAACTTCTCTCGGGTCATA |
| 534144 | 144 | 147,099 | 147,118 | CACTCAGACGGTCCAGCATC |
| 534145 | 145 | 147,098 | 147,117 | ACTCAGACGGTCCAGCATCA |
| 534146 | 146 | 145,529 | 145,548 | CTATCTCCCAGGCCTAAAAT |
| 534147 | 147 | 145,527 | 145,546 | ATCTCCCAGGCCTAAAATAT |
| 534148 | 148 | 141,227 | 141,246 | GACGGCATGCTCAAACACCT |
| 534149 | 149 | 141,226 | 141,245 | ACGGCATGCTCAAACACCTC |
| 534150 | 150 | 141,196 | 141,215 | ATCAGAGTCAAGTGGTCATA |
| 534151 | 151 | 137,978 | 137,997 | AAGAGCTGCATCACACGCTC |
| 534152 | 152 | 137,976 | 137,995 | GAGCTGCATCACACGCTCAT |
| 534153 | 153 | 135,788 | 135,807 | CCCGGCACATCAGAAGTTTT |
| 534154 | 154 | 135,765 | 135,784 | GAAACATATTGCAGCTCTAA |
| 534155 | 155 | 135,764 | 135,783 | AAACATATTGCAGCTCTAAG |
| 534156 | 156 | 135,509 | 135,528 | TTGAGATTCGTCGGAACACA |
| 534157 | 157 | 135,508 | 135,527 | TGAGATTCGTCGGAACACAT |
| 534158 | 158 | 135,423 | 135,442 | CACTCTTGGGCCTCCATTAA |
| 534159 | 159 | 135,422 | 135,441 | ACTCTTGGGCCTCCATTAAA |
| 534160 | 160 | 134,876 | 134,895 | GCCCCGTTCCATCATAGCAT |
| 534161 | 161 | 134,872 | 134,891 | CGTTCCATCATAGCATGCAA |
| 534162 | 162 | 134,841 | 134,860 | CCTCAAACATGCCTTTCACG |
| 534163 | 163 | 134,840 | 134,859 | CTCAAACATGCCTTTCACGT |
| 534164 | 164 | 134,814 | 134,833 | CCCCAAAGTACAAACGAGAT |
| 534165 | 165 | 134,811 | 134,830 | CAAAGTACAAACGAGATGCC |
| 534166 | 166 | 134,497 | 134,516 | GCTCACACATGTTCTTCAGA |
| 534167 | 167 | 134,043 | 134,062 | GTGAATGAGACGTCCCACCA |
| 534168 | 168 | 134,011 | 134,030 | TATCAATTCTTGCAATGAGC |
| 534169 | 169 | 134,010 | 134,029 | ATCAATTCTTGCAATGAGCT |
| 534170 | 170 | 133,610 | 133,629 | GACCATAATCAAACCATAAG |
| 534171 | 171 | 133,602 | 133,621 | TCAAACCATAAGGTGAGAAC |
| 534172 | 172 | 131,850 | 131,869 | GCATGACGCAGTTTCTTCTT |
| 534173 | 173 | 131,849 | 131,868 | CATGACGCAGTTTCTTCTTC |
| 534174 | 174 | 129,419 | 129,438 | CTGTAGTACTGCAGCACTTT |
| 534175 | 175 | 129,366 | 129,385 | CAGCTGCCACTCTCCAAGTT |
| 534176 | 176 | 129,365 | 129,384 | AGCTGCCACTCTCCAAGTTT |
| 534177 | 177 | 129,364 | 129,383 | GCTGCCACTCTCCAAGTTTC |
| 534178 | 178 | 129,363 | 129,382 | CTGCCACTCTCCAAGTTTCA |
| 534179 | 179 | 129,360 | 129,379 | CCACTCTCCAAGTTTCAGGA |
| 534180 | 180 | 129,359 | 129,378 | CACTCTCCAAGTTTCAGGAA |
| 534181 | 181 | 128,111 | 128,130 | CTGCATGGTCTGGACAAAAT |
| 534182 | 182 | 123,140 | 123,159 | GATCAACTCCCAGGAGCAAC |
| 534183 | 183 | 123,136 | 123,155 | AACTCCCAGGAGCAACACTA |
| 534184 | 184 | 123,135 | 123,154 | ACTCCCAGGAGCAACACTAA |
| 534185 | 185 | 122,969 | 122,988 | TACTTGAGCCAGGTTCTCAT |
| 534186 | 186 | 122,919 | 122,938 | CACCATAAGGATTTTCTGCC |
| 534187 | 187 | 122,910 | 122,929 | GATTTTCTGCCAGTCCTCTA |
| 534188 | 188 | 122,906 | 122,925 | TTCTGCCAGTCCTCTACGAT |
| 534189 | 189 | 122,905 | 122,924 | TCTGCCAGTCCTCTACGATA |
| 534190 | 190 | 122,904 | 122,923 | CTGCCAGTCCTCTACGATAC |
| 534191 | 191 | 122,901 | 122,920 | CCAGTCCTCTACGATACGCT |
| 534192 | 192 | 117,884 | 117,903 | TGCAGTCTCTCCCACCAGAT |
| 534193 | 193 | 117,836 | 117,855 | ACAAGTTTGTACTGGATAAC |
| 534194 | 194 | 117,835 | 117,854 | CAAGTTTGTACTGGATAACC |
| 534195 | 195 | 117,834 | 117,853 | AAGTTTGTACTGGATAACCT |
| 534196 | 196 | 117,833 | 117,852 | AGTTTGTACTGGATAACCTC |
| 534197 | 197 | 117,832 | 117,851 | GTTTGTACTGGATAACCTCC |
| 534198 | 198 | 117,831 | 117,850 | TTTGTACTGGATAACCTCCT |
| 534199 | 199 | 117,830 | 117,849 | TTGTACTGGATAACCTCCTC |
| 534200 | 200 | 117,800 | 117,819 | AGCATGTGGCAAGAAACCAT |
| 534201 | 201 | 117,562 | 117,581 | ATATGCCCGACTGTAACTCT |
| 534202 | 202 | 117,561 | 117,580 | TATGCCCGACTGTAACTCTC |
| 534203 | 203 | 117,560 | 117,579 | ATGCCCGACTGTAACTCTCT |
| 534204 | 204 | 117,559 | 117,578 | TGCCCGACTGTAACTCTCTC |
| 534205 | 205 | 117,558 | 117,577 | GCCCGACTGTAACTCTCTCC |
| 534206 | 206 | 117,557 | 117,576 | CCCGACTGTAACTCTCTCCT |
| 534207 | 207 | 117,530 | 117,549 | CAGTTAATTCAGCATCCAGC |
| 534208 | 208 | 117,529 | 117,548 | AGTTAATTCAGCATCCAGCA |
| 534209 | 209 | 115,827 | 115,846 | CAGTGCCAGCACAGCTCTAT |
| 534210 | 210 | 115,826 | 115,845 | AGTGCCAGCACAGCTCTATA |
| 534211 | 211 | 115,823 | 115,842 | GCCAGCACAGCTCTATAAAA |
| 534212 | 212 | 115,822 | 115,841 | CCAGCACAGCTCTATAAAAT |
| 534213 | 213 | 115,814 | 115,833 | GCTCTATAAAATGCCCCATC |
| 534214 | 214 | 115,811 | 115,830 | CTATAAAATGCCCCATCATG |
| 534215 | 215 | 115,779 | 115,798 | GATCATACAGGTGTATTCTT |
| 534216 | 216 | 115,778 | 115,797 | ATCATACAGGTGTATTCTTC |
| 534217 | 217 | 115,777 | 115,796 | TCATACAGGTGTATTCTTCC |
| 534218 | 218 | 115,776 | 115,795 | CATACAGGTGTATTCTTCCA |
| 534219 | 219 | 115,766 | 115,785 | TATTCTTCCATGCTGTCCCA |
| 534220 | 220 | 115,765 | 115,784 | ATTCTTCCATGCTGTCCCAC |

**[Table 3]**

| Compound ID | SEQ ID NO | Start point | End point | Sequence (5' to 3') |
|---|---|---|---|---|
| 534221 | 221 | 105,268 | 105,287 | TTTCTCATACCAGGTAGCCT |
| 534222 | 222 | 95,084 | 95,103 | CCAAAGTGTTTCATGGCATA |
| 534223 | 223 | 95,077 | 95,096 | GTTTCATGGCATATTCTAAC |
| 534224 | 224 | 95,072 | 95,091 | ATGGCATATTCTAACACTCC |
| 534225 | 225 | 95,071 | 95,090 | TGGCATATTCTAACACTCCG |
| 534226 | 226 | 95,069 | 95,088 | GCATATTCTAACACTCCGGC |
| 534227 | 227 | 95,068 | 95,087 | CATATTCTAACACTCCGGCC |
| 534228 | 228 | 95,067 | 95,086 | ATATTCTAACACTCCGGCCG |
| 534229 | 229 | 95,065 | 95,084 | ATTCTAACACTCCGGCCGCT |
| 534230 | 230 | 95,039 | 95,058 | GGCTGCTGTAGCTTATTATT |
| 534231 | 231 | 94,177 | 94,196 | TAAGTCTTAACTTCTCATCA |
| 534232 | 232 | 63,171 | 63,190 | CAGCAGAACAATGCCATTGT |
| 534233 | 233 | 63,170 | 63,189 | AGCAGAACAATGCCATTGTC |
| 534234 | 234 | 63,166 | 63,185 | GAACAATGCCATTGTCATCT |
| 534235 | 235 | 62,983 | 63,002 | GTGTTCCATGAATTCAGCCA |
| 534236 | 236 | 62,977 | 62,996 | CATGAATTCAGCCAAGTTTA |
| 534237 | 237 | 62,907 | 62,926 | ATGCTTCTGATGAGCTCATC |
| 534238 | 238 | 62,906 | 62,925 | TGCTTCTGATGAGCTCATCC |
| 534239 | 239 | 62,895 | 62,914 | AGCTCATCCTGTTGATCTTC |
| 534240 | 240 | 62,856 | 62,875 | GACACAAATGCAGCATTGAA |
| 534241 | 241 | 62,855 | 62,874 | ACACAAATGCAGCATTGAAG |
| 534242 | 242 | 62,854 | 62,873 | CACAAATGCAGCATTGAAGA |
| 534243 | 243 | 62,850 | 62,869 | AATGCAGCATTGAAGAGATC |
| 534244 | 244 | 57,891 | 57,910 | CTCAGCCGTCTCAGCCATTC |
| 534245 | 245 | 57,890 | 57,909 | TCAGCCGTCTCAGCCATTCC |
| 534246 | 246 | 57,879 | 57,898 | AGCCATTCCAGCCAGTCATC |
| 534247 | 247 | 57,878 | 57,897 | GCCATTCCAGCCAGTCATCT |
| 534248 | 248 | 57,877 | 57,896 | CCATTCCAGCCAGTCATCTT |
| 534249 | 249 | 57,876 | 57,895 | CATTCCAGCCAGTCATCTTT |
| 534250 | 250 | 53,118 | 53,137 | TGGTAAATCAAAGGATCCTC |
| 534251 | 251 | 53,117 | 53,136 | GGTAAATCAAAGGATCCTCC |
| 534252 | 252 | 53,116 | 53,135 | GTAAATCAAAGGATCCTCCT |
| 534253 | 253 | 53,115 | 53,134 | TAAATCAAAGGATCCTCCTC |
| 534254 | 254 | 53,113 | 53,132 | AATCAAAGGATCCTCCTCTT |
| 534255 | 255 | 53,112 | 53,131 | ATCAAAGGATCCTCCTCTTC |
| 534256 | 256 | 53,108 | 53,127 | AAGGATCCTCCTCTTCATCA |
| 534257 | 257 | 53,100 | 53,119 | TCCTCTTCATCAGCAAGTGT |
| 534258 | 258 | 51,698 | 51,717 | CACATCATAGCGCTGATGAT |
| 534259 | 259 | 51,697 | 51,716 | ACATCATAGCGCTGATGATT |
| 534260 | 260 | 51,672 | 51,691 | GGTGTCGCACCAGAACTTTA |
| 534261 | 261 | 51,671 | 51,690 | GTGTCGCACCAGAACTTTAT |
| 534262 | 262 | 51,670 | 51,689 | TGTCGCACCAGAACTTTATT |
| 534263 | 263 | 51,669 | 51,688 | GTCGCACCAGAACTTTATTC |
| 534264 | 264 | 51,668 | 51,687 | TCGCACCAGAACTTTATTCA |
| 534265 | 265 | 51,667 | 51,686 | CGCACCAGAACTTTATTCAC |
| 534266 | 266 | 51,661 | 51,680 | AGAACTTTATTCACCATTGG |
| 534267 | 267 | 50,153 | 50,172 | GGTCCAGTGTTCGAACAATA |
| 534268 | 268 | 50,116 | 50,135 | GGCATAGTCAGTGAAATCCA |
| 534269 | 269 | 49,718 | 49,737 | CTTCAGGGGCATCAAACAAC |
| 534270 | 270 | 49,717 | 49,736 | TTCAGGGGCATCAAACAACT |
| 534271 | 271 | 49,716 | 49,735 | TCAGGGGCATCAAACAACTT |
| 534272 | 272 | 49,663 | 49,682 | ATCCAGGTTGGCGCCAAACA |
| 534273 | 273 | 49,055 | 49,074 | AGCTTAAATTCACCCCCAAG |
| 534274 | 274 | 49,054 | 49,073 | GCTTAAATTCACCCCCAAGA |
| 534275 | 275 | 49,053 | 49,072 | CTTAAATTCACCCCCAAGAG |
| 534276 | 276 | 49,051 | 49,070 | TAAATTCACCCCCAAGAGCT |
| 534277 | 277 | 49,050 | 49,069 | AAATTCACCCCCAAGAGCTA |
| 534278 | 278 | 49,021 | 49,040 | GCTCAATGAGAAGAATGATC |
| 534279 | 279 | 46,371 | 46,390 | CTATTTCATCCATATAAGGT |
| 534280 | 280 | 46,370 | 46,389 | TATTTCATCCATATAAGGTC |
| 534281 | 281 | 46,369 | 46,388 | ATTTCATCCATATAAGGTCT |
| 534282 | 282 | 46,367 | 46,386 | TTCATCCATATAAGGTCTGA |
| 534283 | 283 | 46,366 | 46,385 | TCATCCATATAAGGTCTGAT |
| 534284 | 284 | 46,333 | 46,352 | AAGGACACCAACATTCCCAG |
| 534285 | 285 | 33,807 | 33,826 | GGCAGGAACTGCACACATTT |
| 534286 | 286 | 33,801 | 33,820 | AACTGCACACATTTGAGTCC |
| 534287 | 287 | 33,708 | 33,727 | ATCCGCATCAGGGCCACCAT |
| 534288 | 288 | 33,686 | 33,705 | ACACAGCTGGGTAGAACTCA |
| 534289 | 289 | 33,685 | 33,704 | CACAGCTGGGTAGAACTCAT |
| 534290 | 290 | 33,684 | 33,703 | ACAGCTGGGTAGAACTCATC |
| 534291 | 291 | 33,683 | 33,702 | CAGCTGGGTAGAACTCATCC |
| 534292 | 292 | 33,682 | 33,701 | AGCTGGGTAGAACTCATCCA |
| 534293 | 293 | 33,637 | 33,656 | CATTTCACTAGTGCTATAGT |
| 534294 | 294 | 33,635 | 33,654 | TTTCACTAGTGCTATAGTCA |
| 534295 | 295 | 31,555 | 31,574 | GACTGGTCTATCATGCCAAT |
| 534296 | 296 | 31,550 | 31,569 | GTCTATCATGCCAATGTTCA |
| 534297 | 297 | 31,509 | 31,528 | AAGCCCCTAAAAGCCCTAAC |
| 534298 | 298 | 31,504 | 31,523 | CCTAAAAGCCCTAACACACG |
| 534299 | 299 | 31,503 | 31,522 | CTAAAAGCCCTAACACACGG |
| 534300 | 300 | 31,502 | 31,521 | TAAAAGCCCTAACACACGGA |
| 534301 | 301 | 31,501 | 31,520 | AAAAGCCCTAACACACGGAT |
| 534302 | 302 | 31,500 | 31,519 | AAAGCCCTAACACACGGATG |
| 534303 | 303 | 31,201 | 31,220 | TCAGAAAATTCAGTAGCACC |
| 534304 | 304 | 31,166 | 31,185 | TACTTCCTGTAGGGCTCTAC |
| 534305 | 305 | 31,165 | 31,184 | ACTTCCTGTAGGGCTCTACT |
| 534306 | 306 | 31,163 | 31,182 | TTCCTGTAGGGCTCTACTAC |
| 534307 | 307 | 31,162 | 31,181 | TCCTGTAGGGCTCTACTACA |
| 534308 | 308 | 31,160 | 31,179 | CTGTAGGGCTCTACTACATA |
| 534309 | 309 | 31,127 | 31,146 | ACCAACTGTCCCAGGGTCCA |
| 534310 | 310 | 31,125 | 31,144 | CAACTGTCCCAGGGTCCACA |
| 534311 | 311 | 30,971 | 30,990 | ACACTAACCATGAGCAGAAA |
| 534312 | 312 | 30,024 | 30,043 | CTCATTTCCAGGCCACTAAC |
| 534313 | 313 | 29,122 | 29,141 | CCTATTGTTGCCAGGACATT |
| 534314 | 314 | 29,121 | 29,140 | CTATTGTTGCCAGGACATTA |
| 534315 | 315 | 29,120 | 29,139 | TATTGTTGCCAGGACATTAT |
| 534316 | 316 | 29,119 | 29,138 | ATTGTTGCCAGGACATTATT |
| 534317 | 317 | 29,115 | 29,134 | TTGCCAGGACATTATTGATC |
| 534318 | 318 | 29,108 | 29,127 | GACATTATTGATCACACCTG |
| 534319 | 319 | 29,081 | 29,100 | ATCAGGGTCTGGATCTTTCA |
| 534320 | 320 | 28,389 | 28,408 | TTCAGAATAGGCTCCATGTA |
| 534321 | 321 | 28,320 | 28,339 | GCACTCTGCTCTTTGATTCT |
| 534322 | 322 | 28,311 | 28,330 | TCTTTGATTCTTCCAATCCC |
| 534323 | 323 | 28,309 | 28,328 | TTTGATTCTTCCAATCCCAC |
| 534324 | 324 | 24,614 | 24,633 | GCCAGCTCCCGGATCTCAAA |
| 534325 | 325 | 24,613 | 24,632 | CCAGCTCCCGGATCTCAAAC |
| 534326 | 326 | 24,612 | 24,631 | CAGCTCCCGGATCTCAAACA |
| 534327 | 327 | 24,611 | 24,630 | AGCTCCCGGATCTCAAACAC |
| 534328 | 328 | 24,610 | 24,629 | GCTCCCGGATCTCAAACACC |
| 534329 | 329 | 24,609 | 24,628 | CTCCCGGATCTCAAACACCT |
| 534330 | 330 | 24,608 | 24,627 | TCCCGGATCTCAAACACCTG |

**[Table 4]**

| Compound ID | SEQ ID NO | Start point | End point | Sequence (5' to 3') |
|---|---|---|---|---|
| 534331 | 331 | 24,604 | 24,623 | GGATCTCAAACACCTGGTCA |
| 534332 | 332 | 23,953 | 23,972 | ATCCGCACAGTGGCGAACAA |
| 534333 | 333 | 23,952 | 23,971 | TCCGCACAGTGGCGAACAAA |
| 534334 | 334 | 23,951 | 23,970 | CCGCACAGTGGCGAACAAAT |
| 534335 | 335 | 23,950 | 23,969 | CGCACAGTGGCGAACAAATT |
| 534336 | 336 | 22,227 | 22,246 | CAAATTCAAAGCTGCCAAGC |
| 534337 | 337 | 22,226 | 22,245 | AAATTCAAAGCTGCCAAGCG |
| 534338 | 338 | 22,225 | 22,244 | AATTCAAAGCTGCCAAGCGT |
| 534339 | 339 | 22,224 | 22,243 | ATTCAAAGCTGCCAAGCGTT |
| 534340 | 340 | 20,956 | 20,975 | TCCAGCAGCTCCTTGATATC |
| 534341 | 341 | 19,377 | 19,396 | TCCAGCACGCGAGGCAAATA |
| 534342 | 342 | 19,375 | 19,394 | CAGCACGCGAGGCAAATAGA |
| 534343 | 343 | 19,374 | 19,393 | AGCACGCGAGGCAAATAGAC |
| 534344 | 344 | 19,373 | 19,392 | GCACGCGAGGCAAATAGACC |
| 534345 | 345 | 19,372 | 19,391 | CACGCGAGGCAAATAGACCT |
| 534346 | 346 | 19,371 | 19,390 | ACGCGAGGCAAATAGACCTT |
| 534347 | 347 | 19,370 | 19,389 | CGCGAGGCAAATAGACCTTA |
| 534348 | 348 | 19,369 | 19,388 | GCGAGGCAAATAGACCTTAA |
| 534349 | 349 | 19,368 | 19,387 | CGAGGCAAATAGACCTTAAA |
| 534350 | 350 | 19,367 | 19,386 | GAGGCAAATAGACCTTAAAC |
| 534351 | 351 | 19,366 | 19,385 | AGGCAAATAGACCTTAAACT |
| 534352 | 352 | 19,365 | 19,384 | GGCAAATAGACCTTAAACTC |
| 534353 | 353 | 19,364 | 19,383 | GCAAATAGACCTTAAACTCA |
| 534354 | 354 | 19,363 | 19,382 | CAAATAGACCTTAAACTCAG |
| 534355 | 355 | 19,362 | 19,381 | AAATAGACCTTAAACTCAGA |
| 534356 | 356 | 19,361 | 19,380 | AATAGACCTTAAACTCAGAC |
| 534357 | 357 | 19,360 | 19,379 | ATAGACCTTAAACTCAGACC |
| 534358 | 358 | 19,359 | 19,378 | TAGACCTTAAACTCAGACCT |
| 534359 | 359 | 19,356 | 19,375 | ACCTTAAACTCAGACCTCAC |
| 534360 | 360 | 19,355 | 19,374 | CCTTAAACTCAGACCTCACA |
| 534361 | 361 | 19,348 | 19,367 | CTCAGACCTCACAGCCACAG |
| 534362 | 362 | 19,347 | 19,366 | TCAGACCTCACAGCCACAGA |
| 534363 | 363 | 19,346 | 19,365 | CAGACCTCACAGCCACAGAA |
| 534364 | 364 | 19,345 | 19,364 | AGACCTCACAGCCACAGAAA |
| 534365 | 365 | 19,344 | 19,363 | GACCTCACAGCCACAGAAAG |
| 534366 | 366 | 19,343 | 19,362 | ACCTCACAGCCACAGAAAGT |
| 534367 | 367 | 19,342 | 19,361 | CCTCACAGCCACAGAAAGTA |
| 534368 | 368 | 19,340 | 19,359 | TCACAGCCACAGAAAGTAGC |
| 534369 | 369 | 19,339 | 19,358 | CACAGCCACAGAAAGTAGCC |
| 534370 | 370 | 19,337 | 19,356 | CAGCCACAGAAAGTAGCCCC |
| 534371 | 371 | 19,336 | 19,355 | AGCCACAGAAAGTAGCCCCA |
| 534372 | 372 | 19,335 | 19,354 | GCCACAGAAAGTAGCCCCAG |
| 534373 | 373 | 19,334 | 19,353 | CCACAGAAAGTAGCCCCAGG |
| 534374 | 374 | 19,330 | 19,349 | AGAAAGTAGCCCCAGGGCTT |
| 534375 | 375 | 19,308 | 19,327 | AAGGCCGCTGTACGTTCCTT |
| 534376 | 376 | 19,307 | 19,326 | AGGCCGCTGTACGTTCCTTC |
| 534377 | 377 | 19,306 | 19,325 | GGCCGCTGTACGTTCCTTCT |
| 534378 | 378 | 19,305 | 19,324 | GCCGCTGTACGTTCCTTCTC |
| 534379 | 379 | 19,304 | 19,323 | CCGCTGTACGTTCCTTCTCC |
| 534380 | 380 | 19,303 | 19,322 | CGCTGTACGTTCCTTCTCCT |
| 534381 | 381 | 19,302 | 19,321 | GCTGTACGTTCCTTCTCCTT |
| 534382 | 382 | 19,301 | 19,320 | CTGTACGTTCCTTCTCCTTC |
| 534383 | 383 | 19,300 | 19,319 | TGTACGTTCCTTCTCCTTCT |
| 534384 | 384 | 19,283 | 19,302 | TCTTGACACAGCTTAGGACA |
| 534385 | 385 | 19,282 | 19,301 | CTTGACACAGCTTAGGACAT |
| 534386 | 386 | 17,231 | 17,250 | AGGCACCAGTAGTACACATA |
| 534387 | 387 | 17,230 | 17,249 | GGCACCAGTAGTACACATAA |
| 534388 | 388 | 15,965 | 15,984 | AACACCTGACCAGCTACATT |
| 534389 | 389 | 15,964 | 15,983 | ACACCTGACCAGCTACATTT |
| 534390 | 390 | 15,963 | 15,982 | CACCTGACCAGCTACATTTC |
| 534391 | 391 | 14,883 | 14,902 | AGCCAAGCGGGGCAACAAAT |
| 534392 | 392 | 14,882 | 14,901 | GCCAAGCGGGGCAACAAATT |
| 534393 | 393 | 14,881 | 14,900 | CCAAGCGGGGCAACAAATTA |
| 534394 | 394 | 14,880 | 14,899 | CAAGCGGGGCAACAAATTAA |
| 534395 | 395 | 14,872 | 14,891 | GCAACAAATTAAGGATTGTC |
| 534396 | 396 | 14,846 | 14,865 | ATCAGCGAGTTCTTGCTATT |
| 534397 | 397 | 14,845 | 14,864 | TCAGCGAGTTCTTGCTATTC |
| 534398 | 398 | 14,844 | 14,863 | CAGCGAGTTCTTGCTATTCC |
| 534399 | 399 | 14,843 | 14,862 | AGCGAGTTCTTGCTATTCCT |
| 534400 | 400 | 14,841 | 14,860 | CGAGTTCTTGCTATTCCTGC |
| 534401 | 401 | 14,840 | 14,859 | GAGTTCTTGCTATTCCTGCA |
| 534402 | 402 | 14,838 | 14,857 | GTTCTTGCTATTCCTGCATT |
| 534403 | 403 | 14,835 | 14,854 | CTTGCTATTCCTGCATTTCA |
| 534404 | 404 | 14,833 | 14,852 | TGCTATTCCTGCATTTCAGC |
| 534405 | 405 | 14,832 | 14,851 | GCTATTCCTGCATTTCAGCA |
| 534406 | 406 | 14,830 | 14,849 | TATTCCTGCATTTCAGCACC |
| 534407 | 407 | 14,829 | 14,848 | ATTCCTGCATTTCAGCACCC |
| 534408 | 408 | 14,826 | 14,845 | CCTGCATTTCAGCACCCACT |
| 534409 | 409 | 14,458 | 14,477 | TCCATTTCTTCTCTCAGACG |
| 534410 | 410 | 11,693 | 11,712 | CCTCCTAATACTCAGGCCAA |
| 534411 | 411 | 11,692 | 11,711 | CTCCTAATACTCAGGCCAAA |
| 534412 | 412 | 11,691 | 11,710 | TCCTAATACTCAGGCCAAAC |
| 534413 | 413 | 8,637 | 8,656 | GATCCGATCATCCCGATTCA |
| 534414 | 414 | 8,636 | 8,655 | ATCCGATCATCCCGATTCAT |
| 534415 | 415 | 8,630 | 8,649 | TCATCCCGATTCATGCCCTT |
| 534416 | 416 | 8,628 | 8,647 | ATCCCGATTCATGCCCTTCT |
| 534417 | 417 | 8,624 | 8,643 | CGATTCATGCCCTTCTCTTT |
| 534418 | 418 | 8,606 | 8,625 | TTGGCCAAGGTCTCATCAAA |
| 534419 | 419 | 6,524 | 6,543 | TCTGCATCTCCTTCGGCTCA |
| 534420 | 420 | 6,523 | 6,542 | CTGCATCTCCTTCGGCTCAC |
| 534421 | 421 | 5,554 | 5,573 | AGGGCTCGCTTCACCTCAAA |
| 534422 | 422 | 5,553 | 5,572 | GGGCTCGCTTCACCTCAAAT |
| 534423 | 423 | 5,552 | 5,571 | GGCTCGCTTCACCTCAAATT |
| 534424 | 424 | 5,551 | 5,570 | GCTCGCTTCACCTCAAATTC |
| 534425 | 425 | 5,549 | 5,568 | TCGCTTCACCTCAAATTCCA |
| 534426 | 426 | 5,548 | 5,567 | CGCTTCACCTCAAATTCCAC |
| 534427 | 427 | 4,038 | 4,057 | GATGCCACCTTTCCTCTCAT |
| 534428 | 428 | 4,031 | 4,050 | CCTTTCCTCTCATTGGCATC |
| 534429 | 429 | 4,015 | 4,034 | CATCTGAGCTGGAAACCAAT |
| 534430 | 430 | 4,014 | 4,033 | ATCTGAGCTGGAAACCAATT |
| 534431 | 431 | 4,013 | 4,032 | TCTGAGCTGGAAACCAATTC |
| 534432 | 432 | 4,012 | 4,031 | CTGAGCTGGAAACCAATTCA |
| 534433 | 433 | 68 | 87 | CTCAGCTGCCGCCGCCAGCA |
| 534434 | 434 | 67 | 86 | TCAGCTGCCGCCGCCAGCAC |
| 534435 | 435 | 19 | 38 | CACCGCCCGCCTTCCCCGCT |
| 534436 | 436 | 18 | 37 | ACCGCCCGCCTTCCCCGCTG |
| 534437 | 437 | 17 | 36 | CCGCCCGCCTTCCCCGCTGT |
| 534438 | 438 | 16 | 35 | CGCCCGCCTTCCCCGCTGTC |
| 534439 | 439 | 12 | 31 | CGCCTTCCCCGCTGTCCTCT |

In a specific example, the oligomer compound comprises a modified oligonucleotide composed of 8 to 80 linked nucleosides, wherein the nucleic acid sequence of modified oligonucleotide may comprise 12, 13, 14, 15, 16, 17, 18, 19, or 20 contiguous nucleobases within a region selected from the group consisting of SEQ ID NOs 1 to 439. The modified oligonucleotide comprising at least one modification selected from the group consisting of a modified sugar moiety, a modified internucleoside linkage and a modified nucleobase.

The specific oligonucleotide may bind complementarily to TR1 region, more specifically the TR6 region; TR2 region; TR3 region, more specifically the TR7 region; TR4 region; or TR5 region in the nucleic acid sequence of human MTOR, for example, the MTOR nucleic acid sequence of Ensembl Transcript ID: ENST00000361445.9. Specific information regarding each of the regions is provided in Tables 5 and 6 below. The target region may specifically be the nucleic acid sequence within the human MTOR transcript, for example, the MTOR nucleic acid sequence of Ensembl Transcript ID: ENST00000361445.9. Accordingly, the antisense oligonucleotide may be an oligomeric compound comprising an oligonucleotide consisting of 8 to 80, for example, 12 to 30, or 12 to 22, specifically, 12, 13, 14, 15, 16, 17, 18, 19, or 20 contiguous linked nucleosides, complementary to a region of MTOR gene selected from the group consisting of SEQ ID NOs: 440 to 446, and may have an MTOR knockdown (KD) efficiency of 35% or more, or 40% or more, based on the control group before treatment and the non-treatment of oligonucleotide, which is set on 0%.

The region may be identified as a target site providing highly effective inhibition of MTOR expression in antisense oligonucleotide screening, and such a region on MTOR mRNA can be considered a favorable target region for an antisense oligonucleotide or an RNAi agent.

**[Table 5]**

| Region ID | Start point of SEQ ID NO: 2 | End point of SEQ ID NO: 2 | Nucleotide sequence for TR region of SEQ ID NO:2 (5' to 3') | SEQ ID NO |
|---|---|---|---|---|
| TR1 | 155,919 | 155,963 | CAACTAAATGCACAAAGTATAAAGTG TAGCCATGTCTAGACACCA | 440 |
| TR2 | 155,843 | 155,890 | CAGAAATACTGTCATGGAGGTGCTGA ACACAGGGAAGGTCTGGTACAT | 441 |
| TR3 | 155,395 | 155,428 | TCATAAGTACTTGCTGATGGAAGAAT GACCTAGT | 442 |
| TR4 | 14,835 | 14,865 | TGAAATGCAGGAATAGCAAGAACTCG CTGAT | 443 |
| TR5 | 8,624 | 8,655 | AAAGAGAAGGGCATGAATCGGGATG ATCGGAT | 444 |
| TR6 | 155,925 | 155,950 | AATGCACAAAGTATAAAGTGTAGCCA | 445 |
| TR7 | 155,402 | 155,426 | TACTTGCTGATGGAAGAATGACCTA | 446 |

**[Table 6]**

| Target Region ID | Reverse complementary Nucleotide sequence for TR region of SEQ ID NO:2 | SEQ ID NO |
|---|---|---|
| | Nucleotide sequence (5' to 3') | |
| TR1 | TGGTGTCTAGACATGGCTACACTTTATACTTTGTGC ATTTAGTTG | 447 |
| TR2 | ATGTACCAGACCTTCCCTGTGTTCAGCACCTCCAT GACAGTATTTCTG | 448 |
| TR3 | ACTAGGTCATTCTTCCATCAGCAAGTACTTATGA | 449 |
| TR4 | ATCAGCGAGTT***CTTGCTATT***CCTGCATTTCA | 450 |
| TR5 | ATCCGATCATCCCGATTCATGCCCTTCTCTTT | 451 |
| TR6 | TGGCTACACTTTATACTTTGTGCATT | 452 |
| TR7 | TAGGTCATTCTTCCATCAGCAAGTA | 453 |
| TR1-part | CACTTTATACTTTG | 454 |
| TR4-part | CTTGCTATT | 455 |

For example, the oligonucleotide may be an oligomer compound comprising an oligonucleotide consisting of 8 to 80, for example, 12 to 30, or 12 to 22, specifically, 12, 13, 14, 15, 16, 17, 18, 19, 20, 21 or 22 contiguous linked nucleosides, which are reverse complementary to the MTOR gene region (sense strand) selected from the group consisting of SEQ ID NOs 440 to 446.

More specifically, the nucleic acid sequence of an oligonucleotide reversely complementary to an MTOR gene region (sense strand) selected from the group consisting of SEQ ID NOs 440 to 446 is, for example, an oligonucleotide comprising 12, 13, 14, 15, 16, 17, 18, 19, or 20 contiguous linked nucleosides in the nucleic acid sequences of SEQ ID NOs 1 to 19 as an oligonucleotide reversely complementary to a portion of the nucleic acid sequence of TR1, more specifically, the nucleic acid sequences of SEQ ID NOs 10 to 15 as an oligonucleotide reversely complementary to a portion of the nucleic acid sequence of TR6; for example, the nucleic acid sequences of SEQ ID NOs 27 to 36 as an oligonucleotide reversely complementary to a portion of the nucleic acid sequence of TR2; the nucleic acid sequences of SEQ ID NOs 68 to 81 as an oligonucleotide reversely complementary to a portion of the nucleic acid sequence of TR3, more specifically, for example, the nucleic acid sequences of SEQ ID NOs 70 to 74 as an oligonucleotide reversely complementary to a portion of the nucleic acid sequence of TR7; for example, the nucleic acid sequences of SEQ ID NOs 396 to 403 as an oligonucleotide reversely complementary to a portion of the nucleic acid sequence of TR4; or for example, the nucleic acid sequences of SEQ ID NOs 414 to 417 as an oligonucleotide reversely complementary to a portion of the nucleic acid sequence of TR5. In addition, the oligonucleotide may be an oligomer compound comprising the modified oligonucleotide comprises at least one modification selected from the group consisting of a modified sugar moiety, a modified internucleoside linkage and a modified nucleobase.

Specifically, the nucleic acid sequence of the oligonucleotide may be an oligomer compound comprising 12, 13, 14, 15, 16, 17, 18, 19, or 20 contiguous linked nucleosides in the nucleic acid sequence selected from the group consisting of SEQ ID Nos: 10, 11, 12, 13, 14, 28, 32, 35, 71, 396, 397, 398, 399, 400, 401, 402, 403, 414, and 416, and the oligonucleotide may also be an oligomer compound comprising the modified oligonucleotide comprises at least one modification selected from the group consisting of a modified sugar moiety, a modified internucleoside linkage and a modified nucleobase.

The oligonucleotide may be an oligomer compound comprising 8 to 80, for example, 12 to 30, or 12 to 22, specifically 12, 13, 14, 15, 16, 17, 18, 19, or 20 contiguous linked nucleosides within the nucleic acid sequence selected from the group consisting of SEQ ID Nos: 447 to 453. In a specific example, the oligonucleotide complementarily binding to TR1 region may be an oligomer compound that comprises 14 to 80, for example, 14 to 30, or 14 to 22, specifically 14, 15, 16, 17, 18, 19, or 20 contiguous linked nucleosides and the nucleic acid sequence of SEQ ID NO. 454, and may be, for example, an oligomer compound having the nucleic acid sequences of SEQ ID Nos: 10 to 15. The oligonucleotide complementarily binding to TR4 region may be an oligomer compound that comprises 9 to 80, for example, 12 to 30, or 12 to 22, specifically 12, 13, 14, 15, 16, 17, 18, 19, or 20 contiguous linked nucleosides and the nucleic acid sequence of SEQ ID NO. 455, and may be, for example, an oligomer compound having the nucleic acid sequences of SEQ ID NO. 396 to 403.

In the present disclosure, "modified oligonucleotide" refers to an oligonucleotide in which at least one of a sugar moiety, a nucleobase, and an internucleoside linkage is modified. The modified oligonucleotide may comprise at least one modification selected from the group consisting of a modified sugar moiety, a modified internucleoside linkage and a modified nucleobase. The term "unmodified oligonucleotide" means an oligonucleotide that does not contain any modification of a sugar moiety, a nucleobase, and an internucleoside linkage,

"Nucleic acid" refers to a molecule composed of nucleotide monomers. The nucleic acid includes, but is not limited to, ribonucleic acid (RNA), deoxyribonucleic acid (DNA), single-stranded nucleic acid, and double-stranded nucleic acid.

As used in the present disclosure, "modified nucleoside" refers to a nucleoside including a modified nucleobase and/or a modified sugar moiety.

As used herein, "nucleobase" means an unmodified nucleobase or a modified nucleobase. A nucleobase is a heterocyclic moiety. As used herein, an "unmodified nucleobase" is adenine (A), thymine (T), cytosine (C), uracil (U), or guanine (G). As used herein, a "modified nucleobase" is any nucleobase ( or moiety) other than unmodified A, T, C, U, or G that is capable of pairing with at least one other nucleobase. "5-methylcytosine" is a modified nucleobase.

Within the nucleobase sequences of the oligonucleotide, there may be 1, 2, 3, 4, 5, 6, 7, 8, 9, 10, 11, 12, 13, 14, 15, 16, 17, 18, 19, or 20 modified nucleobases at any position.

The modified oligonucleotide has a sugar motif, wherein the sugar motif may comprise: a 5'-wing region consisting of 1 to 10, 1 to 9, 1 to 8, 1 to 7, or 1 to 6 linked modified nucleosides; a central gap region consisting of 6 to 23, 6 to 22, 6 to 21, 6 to 20, 6 to 19, 6 to 18, 6 to 17, 6 to 16, 6 to 15, 6 to 14, 6 to 13, 6 to 12, 6 to 11, or 6 to 10 linked central region nucleosides; and a 3'-wing region consisting of 1 to 10, 1 to 9, 1 to 8, 1 to 7, or 1 to 6 linked modified nucleosides. More specifically, the sugar motif may include: a 5'-wing region consisting of 1 to 6 linked 5'-region nucleosides; a central gap region consisting of 6 to 10 linked central region nucleosides; and a 3'-wing region consisting of 1 to 6 linked 3'-region nucleosides.

In example, the modified sugar moiety may include one or more selected from the group consisting of a modified non-bicyclic sugar moiety and a modified bicyclic or tricyclic sugar moiety. In specific examples, the modified sugar moiety is a sugar surrogate. Such sugar surrogate may include one or more substitutions corresponding to those of other types of modified sugar moieties.

In a specific example, the modified sugar moiety is a modified non-bicyclic sugar moiety containing a furanose ring having one or more substituents, none of which bridges two atoms of the furanose ring to form a cyclic structure. Such non-bridging substituents may be at any position on the furanose ring, including, but not limited to, substituents at the 2', 3', 4' and/or 5' positions (not limited thereto). Examples of 2'-substituents suitable for the modified non-bicyclic sugar moiety include, but are not limited to, the following: 2'-F, 2'-OCH₃ ("OMe" or "O-methyl"), and 2'-O(CH₂) ₂OCH₃ ("MOE"). In specific examples, the 2'-substituent is a halo, allyl, amino, azido, SH, CN, OCN, CF₃, OCF₃, O-C₁-C₁₀ alkoxy, O-C₁-C₁₀ substituted alkoxy, O- C₁-C₁₀ alkyl, O- C₁-C₁₀ substituted alkyl, S-alkyl, N(Rm)-alkyl, O-alkenyl, S-alkenyl, N(Rm)-alkenyl, O-alkynyl, S-alkynyl, N(Rm)-alkynyl, O-alkylenyl-O-alkyl, alkynyl, alkaryl, aralkyl, O-alkaryl, O-aralkyl, O(CH₂)₂SCH₃, O(CH₂)₂ON(Rm)(Rn) or OCH₂C(=O)-N(Rm)(Rn) (wherein each Rm and Rn is independently H, an amino protecting group, or substituted or unsubstituted C₁-C₁₀ alkyl), -O(CH₂) ₂ON(CH₃) ₂ ("DMAOE"), 2'-OCH₂OCH₂N(CH₂) ₂ ("DMAEOE"), and the like.

In specific examples, the modified non-bicyclic sugar moiety includes a substituent at the 3'-position. Examples of substituents suitable for the 3'-position of the modified sugar moiety include, but are not limited to, alkoxy (e.g., methoxy) and alkyl (e.g., methyl, ethyl). In specific examples, the modified non-bicyclic sugar moiety comprises a substituent at the 4'-position. Examples of 4'-substituents suitable for the modified non-bicyclic sugar moiety include, but are not limited to, alkoxy (e.g., methoxy), alkyl, and those described in WO 2015/106128 (Manoharan et al.).

Examples of 5'-substituents suitable for the modified non-bicyclic sugar moiety include, but are not limited to, 5'-methyl (R or S), 5'-vinyl, ethyl, 5'-methoxy, 5'-aminopropyl, or 5'-CP. One embodiment of a 5'-substituent suitable for the modified non-bicyclic sugar moiety is 5'-CP, and specifically "5'-CP" is a modified sugar moiety compound in which two methyl groups are substituted at the 5-position of the included 2-deoxyribose, and these methyl groups are connected to each other to form cyclopropane, and is named as "5'-CP nucleoside", and a detailed description thereof is provided in WO2022/211095A1. In addition, in specific examples, the modified non-bicyclic sugar moiety includes, but is not limited to, one or more non-bridging sugar substituents, e.g., 2'-F-5'-methyl sugar moiety.

In a specific example, the 2'-substituted modified non-bicyclic nucleoside includes a sugar moiety containing a non-bridging 2'-substituent selected from F, OCH₃, and OCH₂CH₂OCH₃.

Specific modified sugar moieties include substituents that bridge two atoms of the furanose ring to form a second ring, thereby generating a bicyclic sugar moiety. Nucleosides including such bicyclic sugar moieties have been referred to as bicyclic nucleosides (BNAs), locked nucleosides (LNAs), or conformationally restricted nucleotides (CRNs). Such compounds are described in U.S. Patent Publication No. 2013/0190383; and PCT Publication WO2013/036868. In these specific embodiments, the bicyclic sugar moiety includes a bridge between the 4' and 2' furanose ring atoms. In certain examples, the furanose is a ribose ring. Examples of 4' to 2' bridging substituents include, but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂) ₂-O-2' ("ENA"), 4'-CH(CH₃)-O-2' (referred to as "constrained ethyl" or "cEt" when present in S configuration), 4'-CH₂-O-CH₂-2', 4'-CH₂-N(R)-2', 4'-CH(CH₂OCH₃)-O-2' ("constrained MOE" or "cMOE") and analogs thereof, 4'-C(CH₃)(CH₃)-O-2' and analogs thereof, 4'-CH₂-N(OCH₃)-2' and analogs thereof, 4'-CH₂-O-N(CH₃)-2', 4'-CH₂-C(H)(CH₃)-2', 4'-CH₂-C(=CH₂)-2' and analogs thereof, 4'-C(RaRb)-N(R)-O-2', 4'-C(RaRb)-O-N(R)-2', 4'-CH₂-O-N(R)-2' and 4'-CH₂-N(R)-O-2' (where each of R, Ra, and Rb is independently H, a protecting group, or a C1-C12 alkyl).

The modified sugar moiety described above includes a substituent that bridges two atoms of the furanose ring to form a second ring, thereby generating a bicyclic sugar moiety. Nucleosides having such a bicyclic sugar moiety have been referred to as bicyclic nucleosides (BNAs) or constrained nucleosides. The bicyclic sugar moiety includes a bridge between 4' and 2' furanose ring atoms. In certain of these embodiments, the furanose is a ribose ring. Examples of 4' to 2' bridging substituents include, but are not limited to: 4'-CH₂-2', 4'-(CH₂) ₂-2', 4'-(CH₂)₃-2', 4'-CH₂-O-2' ("LNA"), 4'-CH₂-S-2', 4'-(CH₂) ₂-O-2' ("ENA"), 4'-CH(CH₃)-O-2' (referred to as " constrained ethyl" or "cEt" when present in S configuration), 4'-CH₂-O-CH₂-2', 4'-CH₂-N(R)-2', 4'-CH(CH₂OCH₃)-O-2' ("constrained MOE" or "cMOE") and analogs thereof, 4'-C(CH₃)(CH₃)-O-2' and analogs thereof, 4'-CH₂-N(OCH₃)-2' and analogs thereof, 4'-CH₂-O-N(CH₃)-2', 4'-CH₂-C(H)(CH₃)-2', 4'-CH₂-C(=CH₂)-2' and analogs thereof, 4'-C(RaRb)-N(R)-O-2', 4'-C(RaRb)-O-N(R)-2', 4'-CH₂-O-N(R)-2' and 4'-CH₂-N(R)-O-2' (where R, Ra and Rb are each independently H, a protecting group or a C₁-C₁₂ alkyl) (e.g., U.S. 7,427,672).

In the present disclosure, the general description of a bicyclic nucleoside includes both isomer configurations. When specific bicyclic nucleosides (e.g., LNA or cEt) are identified in the examples herein, they exist in β-D configuration, unless otherwise specified.

In specific examples, the modified sugar moiety is a sugar surrogate. In these specific embodiments, the ring oxygen atom of the sugar moiety is replaced by, for example, sulfur, carbon, or nitrogen atom.

Many other bicyclic and tricyclic sugars and sugar surrogates that can be used in modified nucleosides are known in the art.

In a specific example, the oligonucleotide compound may comprise an unmodified internucleoside linkage (phosphodiester internucleoside linkage) and/or one or more modified internucleoside linkages. In a specific embodiment, each internucleoside linkage may be one or more internucleoside linkages selected from the group consisting of phosphodiester internucleoside linkages (PO), phosphorothioate internucleoside linkages (PS), and methanesulfonyl phosphoramidate internucleoside linkages (MsPA). The methanesulfonyl phosphoramidate internucleoside linkage (MsPA) is used interchangeably with the mesyl-phosphoramidate internucleoside linkage.

In a specific example, each internucleoside linkage of the modified oligonucleotide may be independently selected from the group consisting of phosphorothioate internucleoside linkage and phosphodiester internucleoside linkage. In a specific example, each phosphorothioate internucleoside linkage may be a stereorandom phosphorothioate internucleoside linkage.

In a specific example, the modified oligonucleotide of the present invention may include one or more internucleoside linkages selected from the group consisting of phosphodiester internucleoside linkage (PO), phosphorothioate internucleoside linkage (PS), and methanesulfonyl phosphoramidate internucleoside linkage (MsPA), and for example, may include phosphodiester (PO) and/or phosphorothioate (PS) to have an internucleoside linkage of PS, PO, or a mixture of PS and PO. In this case, the modified oligonucleotide may have an unmodified or modified sugar moiety and an unmodified or modified nucleobase, and the sugar moiety and nucleobase are described above. For example, when the internucleoside linkages of the modified oligonucleotide include both PS and PO, the PO may be located in both the wing and gap regions of the gapmer structure, and preferably may exist in each wing. Specifically, when the oligonucleotide compound has a gapmer structure of a 5'-wing region, a central gap region, and a 3'-wing region, and 1 to 6 nucleobase in each wing region, the examples of modified oligonucleotides containing mixed internucleoside linkages of PS and PO may be selected from the group consisting of, for example, SOSSS, SOOSS, SOSOS, SOOOSS, SOOOOS, SSSOSS, SSOOSSS, SSOSOS, SSOOSS, SSOOOS, and SSOOSS. In this case, 'O' means a phosphodiester internucleoside linkage (PO), and 'S' means a phosphorothioate internucleoside linkage (PS). More specifically, it may be a combination of a 5'-wing region including at least one selected from the group consisting of SOSSS, SOOSS, SOSOS, SOOOSS, and SOOOOS, and a 3'-wing region including at least one selected from the group consisting of SSSOSS, SSOOSSS, SSOSOS, SSOOSS, SSOOOS, and SSOOSS, specifically SOSSSSSSSSSSSSSSSSOSS, SOSSSSSSSSSSSSSSSSSOOSS, SOOSSSSSSSSSSSSSSSSOOSS, SOSOSSSSSSSSSSSSSSSOSOS, SOOOSSSSSSSSSSSSSSS, SOOOSSSSSSSSSSSSSOOOS, or SOOOOSSSSSSSSSSSSOOSS, and more specifically SOSSSSSSSSSSSSSSSSSOOSS, SOSOSSSSSSSSSSSSSSSOSOS, or SOOOOSSSSSSSSSSSSOOSS. Examples of the oligomer compounds may be a sequence comprising 12, 13, 14, 15, 16, 17, 18, 19, 20, 21, or 22 contiguous nucleobases in the nucleic acid sequences of SEQ ID NOs: 1 to 439, more specifically SEQ ID Nos: 1 to 19, SEQ NOs: 27 to 36, SEQ ID NOs: 68 to 81, SEQ ID NOs: 396 to 403, or SEQ ID NOs: 414 to 417, and even more specifically, a sequence comprising a mixed internucleoside linkage of PO and PS in the nucleic acid sequences comprising 12, 13, 14, 15, 16, 17, 18, 19, or 20 contiguous nucleobases among the nucleic acid sequences of SEQ ID Nos: 11, 12, 13, 14, 28, 95, 102, 105, 106, 319, 396, 398, 399, 403, 409, 414, or 416. When the oligomer compound has a gapmer structure comprised of a 5'-wing region, a gap region, and a 3'-wing region, an example of a modified oligonucleotide comprising a mixed nucleoside linker of PO and PS is described in Table 12 below.

In a specific example, the modified oligonucleotide comprises or consists of a region having a completely modified sugar motif, and each nucleoside within the completely modified region may comprise the same single modified sugar moiety referred to in the present disclosure as a uniformly modified sugar motif, or may comprise two or more different types of modified sugar moieties, and specifically, the modified oligonucleotide may contain a modified bicyclic sugar moiety, e.g., a 4' to 2' bridging sugar substituent, a modified non-bicyclic sugar moiety such as 2'-MOE sugar moiety, or may include at least a modified bicyclic sugar moieties and at least a modified non-bicyclic sugar moieties.

In a specific example, the modified oligonucleotide may include a region having a gapmer motif defined by two outer regions or "wings" and a central or inner region or "gap," wherein the central gap region is a region capable of recruiting RNase H by binding complementarily to the target nucleic acid. The three regions of the gapmer motif (5'-wing, gap, and 3'-wing) form a contiguous sequence of nucleosides, wherein at least a portion of the sugar moieties of the nucleosides in each wing may differ from at least a portion of the sugar moieties of the nucleosides in the gap.

The term "gapmer" refers to an oligonucleotide including an inner region having a plurality of nucleosides that support RNase H cleavage, and being positioned between outer regions having one or more nucleosides, wherein the nucleosides included in the inner region are chemically distinct from the nucleoside or nucleosides included in the outer regions. The inner region may be referred to as a "gap," and the outer regions may be referred to as "wings." Here, the lengths (the number of nucleosides) of the three regions in the gapmer may be provided using a notation of [number of nucleosides in the 5'-wing] - [number of nucleosides in the gap] - [number of nucleosides in the 3'-wing]. For example, a 5-10-5 gapmer contains 5 linked nucleosides in each wing and 10 linked nucleosides in the gap. In the case of oligonucleotides containing chemical modifications in the nomenclature, for example, 5-10-5 MOE gapmer consists of 5 modified nucleosides in the 5'-wing, 10 linked deoxynucleosides in the gap, and 5 linked MOE nucleosides in the 3'-wing. In a specific embodiment, the modified oligonucleotide may include 5-10-5 MOE gapmer, 3-10-3 BNA gapmer, 3-10-3 cEt gapmer, or 3-10-3 LNA gapmer, etc. In a specific embodiment, the modified oligonucleotide may include 5-10-5 MOE gapmer, 3-10-3 BNA gapmer, 3-10-3 cEt gapmer, or 3-10-3 LNA gapmer, etc.

The modified oligonucleotide may include a 5'-wing region having 1 to 10, 1 to 9, 1 to 8, 1 to 7, or 1 to 6 linked modified nucleosides; a central gap region having 6 to 23, 6 to 22, 6 to 21, 6 to 20, 6 to 19, 6 to 18, 6 to 17, 6 to 16, 6 to 15, 6 to 14, 6 to 13, 6 to 12, 6 to 11, or 6 to 10 linked central region nucleosides; and a 3'-wing region having 1 to 10, 1 to 9, 1 to 8, 1 to 7, or 1 to 6 linked modified nucleosides.

In a specific example, the modified oligonucleotide may include at least one modification selected from the group consisting of a modified sugar moiety, a modified internucleoside linkage and a modified nucleobase in the three regions (5'-wing, gap, and 3'-wing) of the gapmer motif. Specifically, one or more modified nucleobases, e.g., 5-mC (5'-methylcytosine), may be included in the modified oligonucleotide, and is mainly included in the gap region, but may also be included in the wing region. The internucleoside linkages, for example, PS and PO linkages, may each be included one or more times in the all internucleoside linkages of the modified oligonucleotide, and for example, the total linkages may be included as PS linkages, or may be included as a mixture of PS and PO linkages. PO linkages may be included in one or more numbers in the total linkages of the modified oligonucleotide, and may be mainly included in the wing region, but may be included in the gap region. The modified sugar moiety may be mainly included in one or more numbers in the total nucleotides included in the oligonucleotide, and specifically, non-bicyclic sugar moiety may be included in the wing region, and bicyclic sugar moiety may be mainly included in the wing region but may be included in the gap region. As a specific example, (i) the modified oligonucleotide may have a structure in which 5-mC is contained in the gap region and all linkages are PS linkages, and a non-bicyclic sugar moiety is contained in the wing region; (ii) the modified oligonucleotide may have a structure in which 5-mC may be included in the gap region and each of PS linkage and PO linkage is included in one or more numbers, and a non-bicyclic sugar moiety is contained in the wing region, for example, 5-mC may be contained in the gap region, all linkages in the gap region are PS linkages, and each of PO linkage and PS linkage are contained in one or more numbers in the wing region, and a non-bicyclic sugar moiety may be contained in the wing region; or (iii) the modified oligonucleotide may have a structure in which 5-mC is mainly contained in the gap region but also in the wing region, PS linkages are included in the entire gap region, PS linkages, PO linkages, or a mixture of PS and PO linkages are included in the wing region, and a bicyclic sugar moiety is mainly included in the wing region but also may be included in the gap region. However, a modified portion selected from the group consisting of modified sugar moiety, unmodified or modified internucleoside linkages, and modified nucleobases included in such modified oligonucleotides are provided merely as illustrative examples, and the present invention is not limited thereto.

For example, the modified sugar moiety may include one or more types selected from the group consisting of a modified non-bicyclic sugar moiety including a 2'-MOE sugar moiety and a 2'-OMe sugar moiety; and a modified bicyclic sugar moiety including a 2',4'-bridge selected from O-CH₂- and -O-CH(CH₃)-. The modified oligomer compound may include a modified non-bicyclic sugar moiety and a modified bicyclic sugar moiety, and specifically may contain at least one modified nucleoside including a modified bicyclic sugar moiety having a 2'-4' bridge and at least one modified nucleoside including a modified non-bicyclic sugar moiety. In a specific example, a 5'-wing and/or a 3'-wing may include at least a modified bicyclic sugar moiety and at least a modified non-bicyclic sugar moiety.

More specifically, when including a 5'-wing region composed of 1 to 6 linked 5'-region nucleosides; a central gap region composed of 6 to 10 linked central region nucleosides; and a 3'-wing region composed of 1 to 6 linked 3'-region nucleosides, the 5'-wing region and/or the 3'-wing region may include at least one modified bicyclic sugar moiety. For example, at least one modified bicyclic sugar moiety may be included in 5'-wing region and/or 3'-wing region, and the remaining nucleosides may include modified non-bicyclic sugar moieties, of which the detailed description is provided in patent documents such as WO2016/127002A1, WO2016/126995A1 and US2018/0023081A1.

In a specific example, when including a 5'-wing region consisting of 1 to 7 linked 5'-region nucleosides; a central gap region consisting of 6 to 10 linked central region nucleosides; and a 3'-wing region consisting of 1 to 7 linked 3'-region nucleosides, each of 5'-wing region and 3'-wing region may independently include one or more modified bicyclic sugar moieties, and specifically, the 5'-wing region may be selected from the group consisting of L, LL, LDL, LLL, LLLL, LLDL, LDLL, LDDL, LLDD, LLLLL, LLLDL, LLLLL, LDLLL, LLDDL, LDDLL, LLDLD, LLDLL, LDLLD, LDLDL, LLDLLL, LLDDLL, LDLLLL, LDLLDL, LDLDLL, LDLDDL, LDDLLL, LDDLDL, LDDDLL, LLLLLDL, LLLDLL, LLDLLLL, LLLLLLL, LLLDDDL, LLDDLDL, LLDDLL, LDDLLLL, LDLLLDL, LDDLL, LDDLL, LDDLL, LDDLL, LDLLLDL, LDDLL, and LDDDLLL; the 3'-wing region may be selected from the group consisting of L, LL, LDL, LLL, LLLL, LLDL, LDLL, LDDL, LLDD, LLLL, LLLLL, LLLDL, LLDLL, LDLLL, LLDDL, LDDLL, LLDLD, LDLLD, LDLDL, LDD LLLLLL, LLLLLDL, LLLDLL, LLLLLLL, LLLLLLL, LLLDDL, LDLDLL, LDDDLL, LLLDLL, LDLLLL, LLLLLL, LLDDLDL, LLDDLL, LDDLLLL, LDLLLDL, LDDLLL, LDDDLLL, LLDDDDL and LLDDL; wherein 'D' represents a 2'-deoxyribosyl sugar moiety and 'L' represents an LNA sugar moiety in the sugar moiety sequence.

Specifically, when the oligonucleotide compound of the present invention may include a specific bicyclic nucleoside (e.g., LNA or cEt) as a modified sugar moiety, it may be an LNA modified oligonucleotide or a cEt modified oligonucleotide, and may include one or more nucleoside linkages selected from the group consisting of phosphorothioate internucleoside linkages, mesyl phosphoramidate internucleoside linkages, and phosphodiester internucleoside linkages, and specifically phosphorothioate internucleoside linkage (PS linkage) and/or phosphodiester internucleoside linkage ((PO linkage).

In addition, the LNA modified oligonucleotide or cEt modified oligonucleotide may include a modified nucleobase and/or a non-modified nucleobase. Examples of an oligomer compounds including a specific bicyclic nucleoside (e.g., LNA or cEt) as a modified sugar moiety may be the modified sugar moiety may be the sequences comprising 12, 13, 14, 15, 16, 17, 18, 19, 20, 21, or 22 contiguous nucleobases within the nucleic acid sequences of SEQ ID Nos: 1 to 439, particularity SEQ Nos: 1 to 19, SEQ Nos: 27 to 36, SEQ ID Nos: 68 to 81, SEQ ID Nos: 396 to 403, or SEQ ID Nos: 414 to 417; or more specifically the sequences comprising 12, 13, 14, 15, 16, 17, 18, 19, 20, 21, or 22 contiguous nucleobases within the nucleic acid sequences of SEQ ID NOs 13, 14, 106, 409, or 416; in which the modified sugar moiety of the , in the nucleic acid sequences may be a bicyclic sugar moiety, and the bicyclic sugar moiety may exist in the 5'-wing region and the 3'-wing region.

The bicyclic sugar moiety present in the 5'-wing region may be independently selected from the group consisting of LLLLLL, LLLLL, LLLDLL, LLLDL, LLDLLL, LLDLL, LLDDLL, LLDDL, LDLLLL, LDLLL, LDLLDL, LDLDLL, LDLDLD, LDLDDL, LDDLLL, LDDLLD, LDDLDL, LDDL, LDDDLL and LDDDL, and the bicyclic sugar moiety in the 3'-wing region may be independently selected from the group consisting of LLLLLL, LLLDLL, LLDDLL, LDLLLL, LDLDLL, LDDLLL, LDDDLL, LLLLL, LLDLL, LDLLL, LDDLL, LLLL, LDLL, LLL, and LL. As a specific example, examples of the LNA-modified oligonucleotides are shown in Table 17, but are not limited thereto. The sequences in Table 17 are shown in the 5' to 3' direction, and 'D' represents a 2'-deoxyribosyl sugar moiety and 'L' represents an LNA sugar moiety among the sugar moiety formulas.

In a specific embodiment, the modified oligonucleotide contains one or more conjugate moieties or conjugate groups. In a specific example, the conjugate group modifies one or more properties of the oligonucleotide molecule, including but not limited to pharmacodynamics, pharmacokinetics, stability, binding, absorption, tissue distribution, cellular distribution, cellular uptake, charge, and clearance. In a certain example, the conjugate moiety provides a fluorescent group or reporter group that enables new properties of the molecule, for example, detection of the molecule.

The conjugate moieties include, but are not limited to, intercalators, reporter molecules, polyamines, polyamides, peptides, carbohydrates (e.g., GalNAc), vitamin moieties, polyethylene glycol, thioethers, polyethers, cholesterol, thiocholesterol, cholic acid moieties, folic acid, lipids, phospholipids, biotin, phenazine, phenanthridine, anthraquinone, adamantane, acridine, fluorescein, rhodamine, coumarin, fluorophores, and dyes.

In certain embodiments, The conjugate group includes a conjugate linker that attaches the conjugate moiety to the remaining portion of the modified oligonucleotide. In certain examples, the conjugate linker is a single chemical bond (i.e., the conjugate moiety is attached to the remaining portion of the modified oligonucleotide via a single bond through the conjugate linker). In a certain example, the conjugate linker includes a chain structure such as a hydrocarbyl chain, or an oligomer of repeating units such as ethylene glycol, nucleoside, or amino acid units. In a certain example, the conjugate linker includes one or more groups selected from alkyl, amino, oxo, amide, disulfide, polyethylene glycol, ether, thioether, and hydroxylamino. In such certain examples, the conjugate linker includes a group selected from alkyl, amino, oxo, amide, and ether groups. In certain examples, the conjugate linkers including the conjugate linker described above are a bifunctional linking moiety, for example, those known in the art to be useful for attaching a conjugate group to an oligomeric compound, such as the oligonucleotides provided herein.

In certain embodiments, the conjugate group or conjugate moiety may be preferably cleaved from the remaining portion of the modified oligonucleotide. For example, under certain circumstances, an oligomeric compound (including an antisense agent or an oligomeric compound that is a part thereof) or a modified oligonucleotide including a specific conjugate moiety is better uptake by a specific cell type. Once the compound is uptake by the cell, it is preferred that the conjugate group is cleaved to release the unconjugated oligonucleotide. Accordingly, certain conjugate moieties may typically contain one or more cleavable moieties within the conjugate linker. In certain examples, the cleavable moiety may be an atomic group having at least one cleavable bond. In certain examples, the cleavable moiety includes an atomic group having 1, 2, 3, 4, or more than 4 cleavable bonds. In certain examples, the cleavable moiety is selectively cleaved within a cell or an intracellular compartment, such as a lysosome. In certain examples, the cleavable moiety is selectively cleaved by an endogenous enzyme, such as a nuclease.

In specific example, the conjugate group includes a cell-targeting conjugate moiety. In certain examples, each ligand of the cell-targeting moiety has an affinity for at least one type of receptor on a target cell. In certain examples, each ligand has an affinity for at least one type of receptor on the surface of a mammalian lung cell. Each ligand of the cell-targeting moiety is a carbohydrate, a carbohydrate derivative, a modified carbohydrate, a polysaccharide, a modified polysaccharide, or a polysaccharide derivative.

An embodiment of the present invention relates to a pharmaceutical composition for preventing, ameliorating, or treating a disease associated with increased activity and/or expression of MTOR, comprising an agent capable of mediating RNA interference (RNAi) against MTOR, or a method for preventing, ameliorating, or treating a disease associated with increased activity and/or expression of MTOR, comprising administering an agent capable of mediating RNA interference (RNAi) against MTOR to a subject or individual in need thereof.

Another embodiment of the present invention provides a composition for reducing human MTOR protein synthesis or human MTOR mRNA levels in a human subject having an increased expression level of the human MTOR gene. Alternatively, the present invention provides a method for reducing MTOR protein synthesis or human MTOR mRNA levels in a human subject having an increased expression level of the MTOR gene.

In specific examples, the method according to the present invention comprises administering the RNAi agent to a subject and detecting or quantifying the amount of MTOR RNA or MTOR protein in a cell or biological fluid of said subject. In certain examples, the method comprises detecting or quantifying a first amount of MTOR RNA or MTOR protein in a first biological sample obtained prior to administration; detecting or quantifying a second amount of MTOR RNA or MTOR protein in a second biological sample obtained after administration; and comparing the first amount with the second amount to detect or quantify a reduction in the MTOR RNA or MTOR protein.

Inhibition of the level or expression of an MTOR nucleic acid can be assayed in various manners known in the art. For example, target nucleic acid levels can be quantified, for instance, by Northern blot analysis, competitive polymerase chain reaction (PCR), or quantitative real-time PCR. Antisense inhibition of an MTOR nucleic acid can be evaluated by measuring MTOR protein levels. Protein levels of MTOR can be evaluated or quantified in various manners well known in the art, such as immunoprecipitation, Western blot analysis (immunoblotting), enzyme-linked immunosorbent assay (ELISA), quantitative protein assays, protein activity assays (e.g., caspase activity assays), immunohistochemistry, immunocytochemistry, or fluorescence-activated cell sorting (FACS).

In specific examples, the subject or patient to be administered may have received one or more therapies selected from the group consisting of drug therapy, radiation therapy, and surgical therapy prior to the treatment.

In specific examples, described herein is a pharmaceutical composition comprising at least an oligomeric compound. In certain examples, each of the one or more oligomeric compounds may be a modified oligonucleotide. In certain examples, the pharmaceutical composition comprises a pharmaceutically acceptable diluent or carrier. In certain examples, the pharmaceutical composition comprises a modified oligonucleotide and artificial cerebrospinal fluid. In certain embodiments, the pharmaceutical composition consists of a modified oligonucleotide and artificial cerebrospinal fluid. In certain examples, the pharmaceutical composition comprises one or more oligomeric compounds and one or more excipients. In certain examples, the excipient may be selected from water, salt solutions, alcohols, polyethylene glycols, gelatin, lactose, amylase, magnesium stearate, talc, silicic acid, viscous paraffin, hydroxymethylcellulose, and polyvinylpyrrolidone.

In specific examples, a pharmaceutical composition comprising an oligomeric compound comprises any pharmaceutically acceptable salt of the oligomeric compound, an ester of the oligomeric compound, or a salt of such an ester.

In specific examples, the pharmaceutical composition comprises a delivery system. Examples of delivery systems include, but are not limited to, liposomes and emulsions.

In certain examples, the pharmaceutical composition may be formulated for oral administration. In certain examples, the pharmaceutical composition is formulated for buccal administration. In certain examples, the pharmaceutical composition is formulated for administration by injection (e.g., intravenous, subcutaneous, intramuscular, intrathecal (IT), intracerebroventricular (ICV), etc.). In such certain examples,, the pharmaceutical composition comprises a carrier and is formulated in an aqueous solution, such as water or a physiologically compatible buffer, such as phosphate-buffered saline (PBS), artificial CSF (aCSF), Hank's solution, Ringer's solution, or a physiological saline buffer. In certain examples, other ingredients (e.g., ingredients that aid solubility or act as preservatives) are included. In certain examples, injectable suspensions are prepared using appropriate liquid carriers, suspending agents, and the like. Certain injectable pharmaceutical compositions are present in unit dosage forms, for example, in ampoules or multi-dose containers. Certain injectable pharmaceutical compositions are suspensions, solutions, or emulsions in oily or aqueous vehicles, and may contain formulation agents such as suspending, stabilizing, and/or dispersing agents. Certain solvents suitable for use in injectable pharmaceutical compositions include, but are not limited to, lipophilic solvents and fatty oils such as sesame oil, synthetic fatty acid esters such as ethyl oleate or triglycerides, and liposomes.

The formulation composition and method of the pharmaceutical composition depend on several criteria including, but not limited to, the route of administration, the severity of the disease, or the dose to be administered.

In certain examples, the oligomeric compound may be mixed with pharmaceutically acceptable active and/or inactive substances for the preparation of a pharmaceutical composition or formulation. Compositions and methods for the formulation of a pharmaceutical composition depend on a number of criteria including, but not limited to, the route of administration, the severity of the disease, or the dose to be administered.

The RNAi formulation according to the present invention may be administered orally or parenterally, and parenteral administration refers to administration via injection (e.g., bolus injection) or infusion. Parenteral administration includes subcutaneous administration, intravenous administration, intramuscular administration, intraarterial administration, intraperitoneal administration, or intracranial administration, for example, intrathecal or intracerebroventricular administration.

### [Advantageous Effects]

The present invention relates to a compound for modulating the expression level and/or activity of an mTOR gene, and a composition comprising the same, which is useful for preventing, alleviating or treating diseases, disorders, and conditions related to MTOR.

### [Brief Description of Drawings]

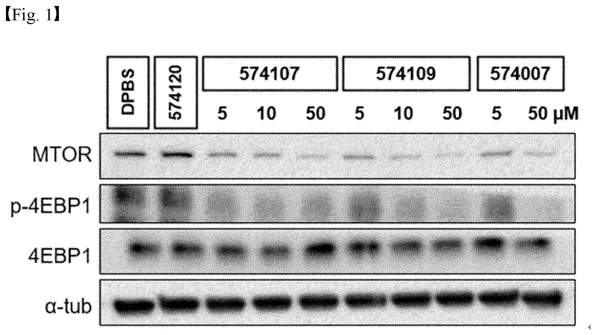

FIG. 1 shows the results of Western blot analysis for MTOR protein, p-4EBP1, 4EBP1, and α-tubulin in neurons harboring the MTOR p.Leu2427Pro mutation, following treatment with modified oligonucleotides complementary to human MTOR (574107, 574109, and 574007) at the indicated concentrations. Cells treated with PBS and cells treated with 50 µM of a non-targeting oligonucleotide were used as negative control groups.

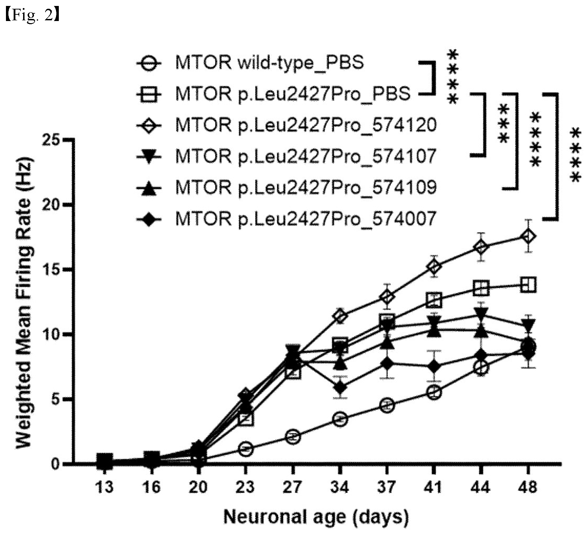

FIG. 2 shows the weighted mean firing rate, one of the analytical parameters obtained from the multi-electrode array (MEA) system, in neurons harboring the MTOR p.Leu2427Pro mutation, following treatment with modified oligonucleotides complementary to human MTOR (574107, 574109, and 574007). (***, p < 0.001; ****, p < 0.0001; two-way ANOVA with Tukey's post hoc test)

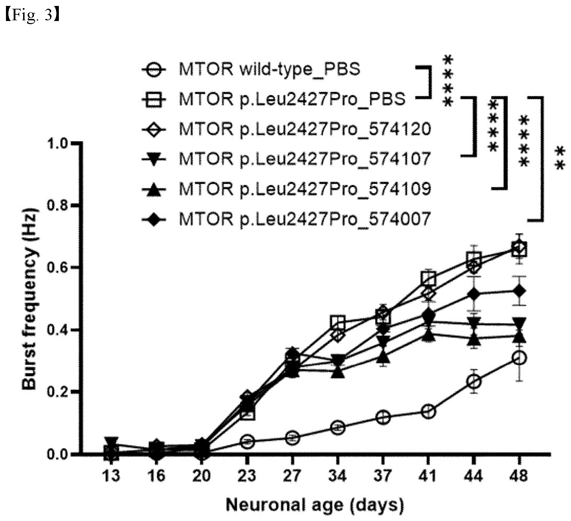

FIG. 3 shows the Burst frequency, one of the analytical parameters obtained from the multi-electrode array (MEA) system, in neurons harboring the MTOR p.Leu2427Pro mutation, following treatment with modified oligonucleotides complementary to human MTOR (574107, 574109, and 574007). (**, p < 0.01; ****, p < 0.0001; two-way ANOVA with Tukey's post hoc test)

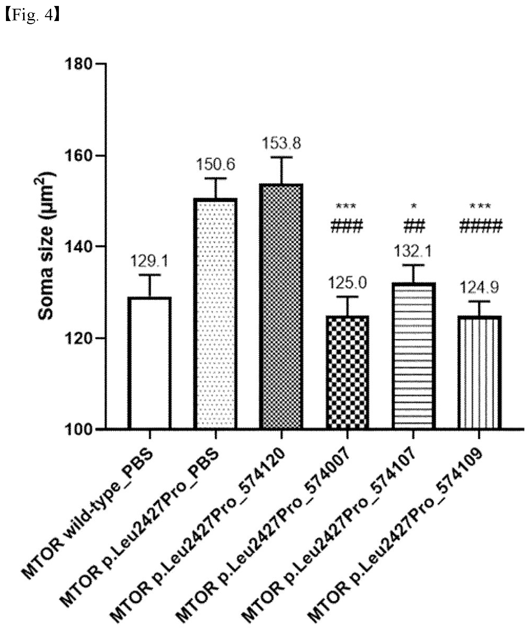

FIG. 4 shows the results of cell soma size analysis in neurons harboring the MTOR p.Leu2427Pro mutation, following treatment with modified oligonucleotides complementary to the nucleic acid sequence of human MTOR (574107, 574109, and 574007) and subsequent immunostaining for the neuronal marker MAP2. (, p < 0.05; ##, p < 0.01; *** or ###, p < 0.001; ####, p < 0.0001; asterisks (*) indicate comparisons with the PBS-treated group, and number signs (#) indicate comparisons with the group treated with non-targeting-oligonucleotide-; one-way ANOVA with Tukey's post hoc test)

### [Mode for Invention]

The present invention will be explained in more detail through the following Examples, but the scope of the present invention is not intended to be limited to the following Examples.

### Example 1: Inhibition of MTOR in SNB-19 Cells by 5-10-5 2'-MOE gapmers (single dose)

5-10-5 2'-MOE gapmer modified oligonucleotides targeting human MTOR RNA were designed and tested for their in vitro effects on MTOR mRNA. The modified oligonucleotides in the table below were 5-10-5 2'-MOE gapmers having phosphorothioate (PS) internucleoside linkages (PS linkages) and contained no modified bases. Each gapmer was 20 nucleosides in length, wherein the central gap segment consists of ten(10) 2'-deoxynucleosides, and the 3' and 5' wings each consist of five (5) 2'-MOE nucleosides. The sugar motif for the gapmer (from 5' to 3') is eeeeeddddddddddeeeee; wherein 'd' represents 2'-deoxyribosyl sugar moiety and 'e' represents the 2'-MOE sugar moiety.

SNB-19 cells, cultured at a density of 7,000 cells/well, were treated with 5 µM of modified oligonucleotides by free uptake. Total RNA was extracted from the cells after 24 hours of treatment. The mRNA expression level of the MTOR gene was measured relative to RPL13, one of the housekeeping genes, by quantitative real-time PCR. The mRNA expression level was measured using Human RPL 13 Primer-Probe Set (Forward primer sequence: ATTTCTGTGGATCCGAGGAGG (SEQ ID NO. 467), Reverse primer sequence: GCCAGTTTCAGTTCTTCAGCAGA (SEQ ID NO: 468), Probe sequence: AGTACCGCTCCAAACTCATCCTCTTCC (SEQ ID NO: 469) and Human MTOR Primer-Probe Set 1 (Forward primer sequence: GCCTTCACAGATACCCAGTAT (SEQ ID NO: 470), Reverse primer sequence: CACGCGAGGCAAATAGACCT (SEQ ID NO: 471), Probe sequence: TGTCAAGAAGGAGAAGGAACGTACAGCG (SEQ ID NO: 472)) or Human MTOR Primer-Probe Set 3 (Forward primer sequence: GAGATCCAGGCTACCTGGT (SEQ ID NO: 473), Reverse primer sequence: GGTGGAGTTGACCCCATTC (SEQ ID NO: 474), Probe sequence: ATGGACACCAACAAGGACGACCCAGA (SEQ ID NO: 475).

The analysis results represent the relative expression level of MTOR mRNA as compared to untreated control cells. Each gapmer listed in the table below targets the human MTOR transcript sequence disclosed herein (Ensembl Transcript ID: ENST00000361445.9). Tables 7 through10 below show the expression levels of human MTOR mRNA following treatment with 5-10-5 2'-MOE gapmer-modified oligonucleotides.

**[Table 7]**

| Compound ID | SEQ ID NO | Sequence (5'to 3') | Relative expression level |
|---|---|---|---|
| 534001 | 1 | TGGTGTCTAGACATGGCTAC | 0.48 |
| 534002 | 2 | GGTGTCTAGACATGGCTACA | 0.39 |
| 534003 | 3 | GTGTCTAGACATGGCTACAC | 0.64 |
| 534004 | 4 | TGTCTAGACATGGCTACACT | 0.53 |
| 534005 | 5 | GTCTAGACATGGCTACACTT | 0.66 |
| 534006 | 6 | TAGACATGGCTACACTTTAT | 0.59 |
| 534007 | 7 | GACATGGCTACACTTTATAC | 0.61 |
| 534008 | 8 | ACATGGCTACACTTTATACT | 0.67 |
| 534009 | 9 | CATGGCTACACTTTATACTT | 0.64 |
| 534010 | 10 | TGGCTACACTTTATACTTTG | 0.5 |
| 534011 | 11 | GGCTACACTTTATACTTTGT | 0.36 |
| 534012 | 12 | GCTACACTTTATACTTTGTG | 0.44 |
| 534013 | 13 | CTACACTTTATACTTTGTGC | 0.24 |
| 534014 | 14 | TACACTTTATACTTTGTGCA | 0.43 |
| 534015 | 15 | CACTTTATACTTTGTGCATT | 0.51 |
| 534016 | 16 | CTTTATACTTTGTGCATTTA | 0.74 |
| 534017 | 17 | TTATACTTTGTGCATTTAGT | 0.46 |
| 534018 | 18 | TATACTTTGTGCATTTAGTT | 0.54 |
| 534019 | 19 | ATACTTTGTGCATTTAGTTG | 0.61 |
| 534020 | 20 | TAGTTGAGTATTTGTTCTGC | 0.62 |
| 534021 | 21 | AGTTGAGTATTTGTTCTGCT | 0.67 |
| 534022 | 22 | CCAATATGTACCAGACCTTC | 0.68 |
| 534023 | 23 | CAATATGTACCAGACCTTCC | 0.92 |
| 534024 | 24 | AATATGTACCAGACCTTCCC | 0.76 |
| 534025 | 25 | ATATGTACCAGACCTTCCCT | 0.66 |
| 534026 | 26 | TATGTACCAGACCTTCCCTG | 0.62 |
| 534027 | 27 | ATGTACCAGACCTTCCCTGT | 0.53 |
| 534028 | 28 | ACCAGACCTTCCCTGTGTTC | 0.44 |
| 534029 | 29 | CCAGACCTTCCCTGTGTTCA | 0.56 |
| 534030 | 30 | CAGACCTTCCCTGTGTTCAG | 0.7 |
| 534031 | 31 | AGACCTTCCCTGTGTTCAGC | 0.65 |
| 534032 | 32 | GACCTTCCCTGTGTTCAGCA | 0.44 |
| 534033 | 33 | TGTGTTCAGCACCTCCATGA | 0.71 |
| 534034 | 34 | TGTTCAGCACCTCCATGACA | 0.62 |
| 534035 | 35 | GTTCAGCACCTCCATGACAG | 0.46 |
| 534036 | 36 | CCTCCATGACAGTATTTCTG | 0.57 |
| 534037 | 37 | CTCCATGACAGTATTTCTGT | 0.67 |
| 534038 | 38 | CTGAGCCCTTGCTCTAAACA | 0.84 |
| 534039 | 39 | TGAGCCCTTGCTCTAAACAG | 0.73 |
| 534040 | 40 | GAGCCCTTGCTCTAAACAGA | 0.94 |
| 534041 | 41 | AGCCCTTGCTCTAAACAGAG | 0.9 |
| 534042 | 42 | GCCCTTGCTCTAAACAGAGT | 1.02 |
| 534043 | 43 | CCCTTGCTCTAAACAGAGTA | 0.94 |
| 534044 | 44 | CCTTGCTCTAAACAGAGTAT | 1.05 |
| 534045 | 45 | CTTGCTCTAAACAGAGTATT | 1.01 |
| 534046 | 46 | CCCTGCTGCAGAAGGCCAGT | 0.97 |
| 534047 | 47 | TGCATCTGCTCAGCCGAGGC | 0.76 |
| 534048 | 48 | CATCACTGGGTCTGATGGAA | 0.95 |
| 534049 | 49 | ATCACTGGGTCTGATGGAAG | 1.06 |
| 534050 | 50 | ACTGGGTCTGATGGAAGACA | 0.91 |
| 534051 | 51 | TTGGCCTATCTTGCAAACAT | 0.98 |
| 534052 | 52 | CCTATCTTGCAAACATTTCT | 0.96 |
| 534053 | 53 | ACCAGTGAGGTCTTGGGATA | 1.04 |
| 534054 | 54 | GTCTAGGATCCTAATCCATG | 0.74 |
| 534055 | 55 | TCTAGGATCCTAATCCATGT | 0.74 |
| 534056 | 56 | CCCATGTTGAGAGGAGCAAC | 0.94 |
| 534057 | 57 | CCATGTTGAGAGGAGCAACT | 0.8 |
| 534058 | 58 | CATGTTGAGAGGAGCAACTA | 1 |
| 534059 | 59 | ATGTTGAGAGGAGCAACTAG | 1.05 |
| 534060 | 60 | AGAGGAGCAACTAGGTCATT | 0.73 |
| 534061 | 61 | GAGGAGCAACTAGGTCATTC | 0.62 |
| 534062 | 62 | AGGAGCAACTAGGTCATTCT | 0.7 |
| 534063 | 63 | GGAGCAACTAGGTCATTCTT | 0.56 |
| 534064 | 64 | GAGCAACTAGGTCATTCTTC | 0.56 |
| 534065 | 65 | AGCAACTAGGTCATTCTTCC | 0.65 |
| 534066 | 66 | GCAACTAGGTCATTCTTCCA | 0.7 |
| 534067 | 67 | CAACTAGGTCATTCTTCCAT | 0.84 |
| 534068 | 68 | ACTAGGTCATTCTTCCATCA | 0.61 |
| 534069 | 69 | CTAGGTCATTCTTCCATCAG | 0.69 |
| 534070 | 70 | TAGGTCATTCTTCCATCAGC | 0.45 |
| 534071 | 71 | GGTCATTCTTCCATCAGCAA | 0.41 |
| 534072 | 72 | GTCATTCTTCCATCAGCAAG | 0.33 |
| 534073 | 73 | TCATTCTTCCATCAGCAAGT | 0.42 |
| 534074 | 74 | CATTCTTCCATCAGCAAGTA | 0.41 |
| 534075 | 75 | ATTCTTCCATCAGCAAGTAC | 0.7 |
| 534076 | 76 | TTCTTCCATCAGCAAGTACT | 0.52 |
| 534077 | 77 | TCTTCCATCAGCAAGTACTT | 0.47 |
| 534078 | 78 | CTTCCATCAGCAAGTACTTA | 0.6 |
| 534079 | 79 | TTCCATCAGCAAGTACTTAT | 0.61 |
| 534080 | 80 | TCCATCAGCAAGTACTTATG | 0.5 |
| 534081 | 81 | CCATCAGCAAGTACTTATGA | 0.61 |
| 534082 | 82 | CATCAGCAAGTACTTATGAT | 0.69 |
| 534083 | 83 | ATCAGCAAGTACTTATGATG | 0.76 |
| 534084 | 84 | AGCAAGTACTTATGATGAGT | 0.59 |
| 534085 | 85 | GCAAGTACTTATGATGAGTT | 0.56 |
| 534086 | 86 | CAAGTACTTATGATGAGTTC | 0.52 |
| 534087 | 87 | AAGTACTTATGATGAGTTCT | 0.51 |
| 534088 | 88 | AGTACTTATGATGAGTTCTC | 0.48 |
| 534089 | 89 | GTACTTATGATGAGTTCTCT | 0.51 |
| 534090 | 90 | ACTTATGATGAGTTCTCTTG | 0.5 |
| 534091 | 91 | CTTATGATGAGTTCTCTTGT | 0.53 |
| 534092 | 92 | TGATGAGTTCTCTTGTGAGT | 0.58 |
| 534093 | 93 | TGAGTTCTCTTGTGAGTTAA | 0.63 |
| 534094 | 94 | AGTTCTCTTGTGAGTTAAGT | 0.58 |
| 534095 | 95 | GTTCTCTTGTGAGTTAAGTC | 0.37 |
| 534096 | 96 | TCTTGTGAGTTAAGTCAAAA | 0.71 |
| 534097 | 97 | CTTGTGAGTTAAGTCAAAAC | 0.98 |
| 534098 | 98 | TTGTGAGTTAAGTCAAAACC | 0.79 |
| 534099 | 99 | TGTGAGTTAAGTCAAAACCC | 0.61 |
| 534100 | 100 | TGAGTTAAGTCAAAACCCGT | 0.87 |
| 534101 | 101 | GAGTTAAGTCAAAACCCGTA | 0.76 |
| 534102 | 102 | AGTTAAGTCAAAACCCGTAT | 0.49 |
| 534103 | 103 | GTTAAGTCAAAACCCGTATT | 0.66 |
| 534104 | 104 | AAGTCAAAACCCGTATTTCT | 0.85 |
| 534105 | 105 | AGTCAAAACCCGTATTTCTA | 0.61 |
| 534106 | 106 | GTCAAAACCCGTATTTCTAA | 0.61 |
| 534107 | 107 | CAAAACCCGTATTTCTAAAG | 0.81 |
| 534108 | 108 | AAAACCCGTATTTCTAAAGT | 0.9 |
| 534109 | 109 | AAACCCGTATTTCTAAAGTT | 0.9 |
| 534110 | 110 | AACCCGTATTTCTAAAGTTA | 0.88 |

**[Table 8]**

| Compound ID | SEQ ID NO | Sequence (5'to 3') | Relative expression level |
|---|---|---|---|
| 534111 | 111 | ACCCGTATTTCTAAAGTTAT | 0.64 |
| 534112 | 112 | CCCGTATTTCTAAAGTTATG | 0.66 |
| 534113 | 113 | CCGTATTTCTAAAGTTATGG | 0.81 |
| 534114 | 114 | CGTATTTCTAAAGTTATGGA | 0.78 |
| 534115 | 115 | TTCTAAAGTTATGGATCTTC | 0.8 |
| 534116 | 116 | CTAAAGTTATGGATCTTCTG | 0.71 |
| 534117 | 117 | AGTTATGGATCTTCTGTTCC | 0.66 |
| 534118 | 118 | GTTATGGATCTTCTGTTCCC | 0.6 |
| 534119 | 119 | TTATGGATCTTCTGTTCCCC | 0.56 |
| 534120 | 120 | TGTTCCCCAAAATGAATGGC | 0.9 |
| 534121 | 121 | GTTCCCCAAAATGAATGGCT | 0.77 |
| 534122 | 122 | TCCCCAAAATGAATGGCTTG | 0.85 |
| 534123 | 123 | ATGGCTTGATTTACGTGGTA | 0.58 |
| 534124 | 124 | TGGCTTGATTTACGTGGTAT | 0.61 |
| 534125 | 125 | GGCTTGATTTACGTGGTATT | 0.67 |
| 534126 | 126 | GCTTGATTTACGTGGTATTA | 0.72 |
| 534127 | 127 | CTTGATTTACGTGGTATTAC | 0.92 |
| 534128 | 128 | TTGATTTACGTGGTATTACT | 0.9 |
| 534129 | 129 | CAGCCAATATAGCACTGGCA | 0.97 |
| 534130 | 130 | GAACATCCAAAGTGTCATCA | 0.81 |
| 534131 | 131 | TCCAAAGTGTCATCATGAGA | 0.6 |
| 534132 | 132 | AGCTTATCTCGAACCCTGTT | 0.72 |
| 534133 | 133 | GCTTATCTCGAACCCTGTTA | 0.72 |
| 534134 | 134 | CTGGTTTCACCAAACCGTCT | 1.01 |
| 534135 | 135 | TGGTTTCACCAAACCGTCTC | 0.82 |
| 534136 | 136 | GTTCCACACCGTCCAAAATT | 0.71 |
| 534137 | 137 | TTCCACACCGTCCAAAATTT | 0.89 |
| 534138 | 138 | TGTCCATCAGCCTCCAGTTC | 0.67 |
| 534139 | 139 | TGATTCTGTAGTTGCCATCC | 0.89 |
| 534140 | 140 | GATTCTGTAGTTGCCATCCA | 0.84 |
| 534141 | 141 | GTCAACATTCTTGTTAGTCT | 0.61 |
| 534142 | 142 | TCAACATTCTTGTTAGTCTA | 0.68 |
| 534143 | 143 | GAAACTTCTCTCGGGTCATA | 0.66 |
| 534144 | 144 | CACTCAGACGGTCCAGCATC | 0.74 |
| 534145 | 145 | ACTCAGACGGTCCAGCATCA | 0.89 |
| 534146 | 146 | CTATCTCCCAGGCCTAAAAT | 1.14 |
| 534147 | 147 | ATCTCCCAGGCCTAAAATAT | 0.91 |
| 534148 | 148 | GACGGCATGCTCAAACACCT | 0.69 |
| 534149 | 149 | ACGGCATGCTCAAACACCTC | 0.86 |
| 534150 | 150 | ATCAGAGTCAAGTGGTCATA | 0.8 |
| 534151 | 151 | AAGAGCTGCATCACACGCTC | 0.96 |
| 534152 | 152 | GAGCTGCATCACACGCTCAT | 1.04 |
| 534153 | 153 | CCCGGCACATCAGAAGTTTT | 0.83 |
| 534154 | 154 | GAAACATATTGCAGCTCTAA | 0.96 |
| 534155 | 155 | AAACATATTGCAGCTCTAAG | 0.86 |
| 534156 | 156 | TTGAGATTCGTCGGAACACA | 0.94 |
| 534157 | 157 | TGAGATTCGTCGGAACACAT | 0.87 |
| 534158 | 158 | CACTCTTGGGCCTCCATTAA | 1.03 |
| 534159 | 159 | ACTCTTGGGCCTCCATTAAA | 0.95 |
| 534160 | 160 | GCCCCGTTCCATCATAGCAT | 0.96 |
| 534161 | 161 | CGTTCCATCATAGCATGCAA | 0.87 |
| 534162 | 162 | CCTCAAACATGCCTTTCACG | 0.96 |
| 534163 | 163 | CTCAAACATGCCTTTCACGT | 0.92 |
| 534164 | 164 | CCCCAAAGTACAAACGAGAT | 0.84 |
| 534165 | 165 | CAAAGTACAAACGAGATGCC | 0.98 |
| 534166 | 166 | GCTCACACATGTTCTTCAGA | 0.51 |
| 534167 | 167 | GTGAATGAGACGTCCCACCA | 0.91 |
| 534168 | 168 | TATCAATTCTTGCAATGAGC | 0.67 |
| 534169 | 169 | ATCAATTCTTGCAATGAGCT | 0.65 |
| 534170 | 170 | GACCATAATCAAACCATAAG | 0.92 |
| 534171 | 171 | TCAAACCATAAGGTGAGAAC | 1.05 |
| 534172 | 172 | GCATGACGCAGTTTCTTCTT | 0.81 |
| 534173 | 173 | CATGACGCAGTTTCTTCTTC | 0.81 |
| 534174 | 174 | CTGTAGTACTGCAGCACTTT | 0.86 |
| 534175 | 175 | CAGCTGCCACTCTCCAAGTT | 0.98 |
| 534176 | 176 | AGCTGCCACTCTCCAAGTTT | 0.75 |
| 534177 | 177 | GCTGCCACTCTCCAAGTTTC | 0.67 |
| 534178 | 178 | CTGCCACTCTCCAAGTTTCA | 0.88 |
| 534179 | 179 | CCACTCTCCAAGTTTCAGGA | 0.55 |
| 534180 | 180 | CACTCTCCAAGTTTCAGGAA | 0.81 |
| 534181 | 181 | CTGCATGGTCTGGACAAAAT | 0.79 |
| 534182 | 182 | GATCAACTCCCAGGAGCAAC | 0.88 |
| 534183 | 183 | AACTCCCAGGAGCAACACTA | 1.02 |
| 534184 | 184 | ACTCCCAGGAGCAACACTAA | 0.89 |
| 534185 | 185 | TACTTGAGCCAGGTTCTCAT | 0.99 |
| 534186 | 186 | CACCATAAGGATTTTCTGCC | 1.16 |
| 534187 | 187 | GATTTTCTGCCAGTCCTCTA | 0.85 |
| 534188 | 188 | TTCTGCCAGTCCTCTACGAT | 1 |
| 534189 | 189 | TCTGCCAGTCCTCTACGATA | 0.85 |
| 534190 | 190 | CTGCCAGTCCTCTACGATAC | 0.79 |
| 534191 | 191 | CCAGTCCTCTACGATACGCT | 0.8 |
| 534192 | 192 | TGCAGTCTCTCCCACCAGAT | 0.66 |
| 534193 | 193 | ACAAGTTTGTACTGGATAAC | 0.97 |
| 534194 | 194 | CAAGTTTGTACTGGATAACC | 1.01 |
| 534195 | 195 | AAGTTTGTACTGGATAACCT | 0.92 |
| 534196 | 196 | AGTTTGTACTGGATAACCTC | 0.89 |
| 534197 | 197 | GTTTGTACTGGATAACCTCC | 0.84 |
| 534198 | 198 | TTTGTACTGGATAACCTCCT | 0.98 |
| 534199 | 199 | TTGTACTGGATAACCTCCTC | 1.01 |
| 534200 | 200 | AGCATGTGGCAAGAAACCAT | 1.06 |
| 534201 | 201 | ATATGCCCGACTGTAACTCT | 0.87 |
| 534202 | 202 | TATGCCCGACTGTAACTCTC | 0.89 |
| 534203 | 203 | ATGCCCGACTGTAACTCTCT | 0.73 |
| 534204 | 204 | TGCCCGACTGTAACTCTCTC | 0.66 |
| 534205 | 205 | GCCCGACTGTAACTCTCTCC | 0.92 |
| 534206 | 206 | CCCGACTGTAACTCTCTCCT | 0.97 |
| 534207 | 207 | CAGTTAATTCAGCATCCAGC | 0.83 |
| 534208 | 208 | AGTTAATTCAGCATCCAGCA | 0.83 |
| 534209 | 209 | CAGTGCCAGCACAGCTCTAT | 0.85 |
| 534210 | 210 | AGTGCCAGCACAGCTCTATA | 0.94 |
| 534211 | 211 | GCCAGCACAGCTCTATAAAA | 0.84 |
| 534212 | 212 | CCAGCACAGCTCTATAAAAT | 0.91 |
| 534213 | 213 | GCTCTATAAAATGCCCCATC | 0.85 |
| 534214 | 214 | CTATAAAATGCCCCATCATG | 0.95 |
| 534215 | 215 | GATCATACAGGTGTATTCTT | 0.74 |
| 534216 | 216 | ATCATACAGGTGTATTCTTC | 0.89 |
| 534217 | 217 | TCATACAGGTGTATTCTTCC | 0.8 |
| 534218 | 218 | CATACAGGTGTATTCTTCCA | 0.68 |
| 534219 | 219 | TATTCTTCCATGCTGTCCCA | 0.92 |
| 534220 | 220 | ATTCTTCCATGCTGTCCCAC | 0.91 |

**[Table 9]**

| Compound ID | SEQ ID NO | Sequence (5'to 3') | Relative expression level |
|---|---|---|---|
| 534221 | 221 | TTTCTCATACCAGGTAGCCT | 0.68 |
| 534222 | 222 | CCAAAGTGTTTCATGGCATA | 0.75 |
| 534223 | 223 | GTTTCATGGCATATTCTAAC | 0.83 |
| 534224 | 224 | ATGGCATATTCTAACACTCC | 0.7 |
| 534225 | 225 | TGGCATATTCTAACACTCCG | 0.52 |
| 534226 | 226 | GCATATTCTAACACTCCGGC | 0.64 |
| 534227 | 227 | CATATTCTAACACTCCGGCC | 0.67 |
| 534228 | 228 | ATATTCTAACACTCCGGCCG | 0.72 |
| 534229 | 229 | ATTCTAACACTCCGGCCGCT | 0.71 |
| 534230 | 230 | GGCTGCTGTAGCTTATTATT | 0.61 |
| 534231 | 231 | TAAGTCTTAACTTCTCATCA | 0.69 |
| 534232 | 232 | CAGCAGAACAATGCCATTGT | 0.77 |
| 534233 | 233 | AGCAGAACAATGCCATTGTC | 0.87 |
| 534234 | 234 | GAACAATGCCATTGTCATCT | 0.81 |
| 534235 | 235 | GTGTTCCATGAATTCAGCCA | 0.7 |
| 534236 | 236 | CATGAATTCAGCCAAGTTTA | 0.72 |
| 534237 | 237 | ATGCTTCTGATGAGCTCATC | 0.59 |
| 534238 | 238 | TGCTTCTGATGAGCTCATCC | 0.54 |
| 534239 | 239 | AGCTCATCCTGTTGATCTTC | 0.63 |
| 534240 | 240 | GACACAAATGCAGCATTGAA | 0.7 |
| 534241 | 241 | ACACAAATGCAGCATTGAAG | 0.66 |
| 534242 | 242 | CACAAATGCAGCATTGAAGA | 0.66 |
| 534243 | 243 | AATGCAGCATTGAAGAGATC | 0.68 |
| 534244 | 244 | CTCAGCCGTCTCAGCCATTC | 0.53 |
| 534245 | 245 | TCAGCCGTCTCAGCCATTCC | 0.67 |
| 534246 | 246 | AGCCATTCCAGCCAGTCATC | 0.67 |
| 534247 | 247 | GCCATTCCAGCCAGTCATCT | 0.67 |
| 534248 | 248 | CCATTCCAGCCAGTCATCTT | 0.76 |
| 534249 | 249 | CATTCCAGCCAGTCATCTTT | 0.66 |
| 534250 | 250 | TGGTAAATCAAAGGATCCTC | 0.68 |
| 534251 | 251 | GGTAAATCAAAGGATCCTCC | 0.6 |
| 534252 | 252 | GTAAATCAAAGGATCCTCCT | 0.75 |
| 534253 | 253 | TAAATCAAAGGATCCTCCTC | 0.8 |
| 534254 | 254 | AATCAAAGGATCCTCCTCTT | 0.72 |
| 534255 | 255 | ATCAAAGGATCCTCCTCTTC | 0.77 |
| 534256 | 256 | AAGGATCCTCCTCTTCATCA | 0.72 |
| 534257 | 257 | TCCTCTTCATCAGCAAGTGT | 0.56 |
| 534258 | 258 | CACATCATAGCGCTGATGAT | 0.8 |
| 534259 | 259 | ACATCATAGCGCTGATGATT | 0.81 |
| 534260 | 260 | GGTGTCGCACCAGAACTTTA | 0.67 |
| 534261 | 261 | GTGTCGCACCAGAACTTTAT | 0.55 |
| 534262 | 262 | TGTCGCACCAGAACTTTATT | 0.59 |
| 534263 | 263 | GTCGCACCAGAACTTTATTC | 0.49 |
| 534264 | 264 | TCGCACCAGAACTTTATTCA | 0.68 |
| 534265 | 265 | CGCACCAGAACTTTATTCAC | 0.68 |
| 534266 | 266 | AGAACTTTATTCACCATTGG | 0.55 |
| 534267 | 267 | GGTCCAGTGTTCGAACAATA | 0.44 |
| 534268 | 268 | GGCATAGTCAGTGAAATCCA | 0.49 |
| 534269 | 269 | CTTCAGGGGCATCAAACAAC | 0.77 |
| 534270 | 270 | TTCAGGGGCATCAAACAACT | 0.65 |
| 534271 | 271 | TCAGGGGCATCAAACAACTT | 0.92 |
| 534272 | 272 | ATCCAGGTTGGCGCCAAACA | 0.64 |
| 534273 | 273 | AGCTTAAATTCACCCCCAAG | 0.66 |
| 534274 | 274 | GCTTAAATTCACCCCCAAGA | 0.67 |
| 534275 | 275 | CTTAAATTCACCCCCAAGAG | 0.73 |
| 534276 | 276 | TAAATTCACCCCCAAGAGCT | 0.74 |
| 534277 | 277 | AAATTCACCCCCAAGAGCTA | 0.79 |
| 534278 | 278 | GCTCAATGAGAAGAATGATC | 0.61 |
| 534279 | 279 | CTATTTCATCCATATAAGGT | 0.73 |
| 534280 | 280 | TATTTCATCCATATAAGGTC | 0.71 |
| 534281 | 281 | ATTTCATCCATATAAGGTCT | 0.72 |
| 534282 | 282 | TTCATCCATATAAGGTCTGA | 0.66 |
| 534283 | 283 | TCATCCATATAAGGTCTGAT | 0.69 |
| 534284 | 284 | AAGGACACCAACATTCCCAG | 0.61 |
| 534285 | 285 | GGCAGGAACTGCACACATTT | 0.51 |
| 534286 | 286 | AACTGCACACATTTGAGTCC | 0.63 |
| 534287 | 287 | ATCCGCATCAGGGCCACCAT | 0.65 |
| 534288 | 288 | ACACAGCTGGGTAGAACTCA | 0.63 |
| 534289 | 289 | CACAGCTGGGTAGAACTCAT | 0.75 |
| 534290 | 290 | ACAGCTGGGTAGAACTCATC | 0.72 |
| 534291 | 291 | CAGCTGGGTAGAACTCATCC | 0.68 |
| 534292 | 292 | AGCTGGGTAGAACTCATCCA | 0.76 |
| 534293 | 293 | CATTTCACTAGTGCTATAGT | 0.74 |
| 534294 | 294 | TTTCACTAGTGCTATAGTCA | 0.75 |
| 534295 | 295 | GACTGGTCTATCATGCCAAT | 0.65 |
| 534296 | 296 | GTCTATCATGCCAATGTTCA | 0.55 |
| 534297 | 297 | AAGCCCCTAAAAGCCCTAAC | 0.63 |
| 534298 | 298 | CCTAAAAGCCCTAACACACG | 0.74 |
| 534299 | 299 | CTAAAAGCCCTAACACACGG | 0.73 |
| 534300 | 300 | TAAAAGCCCTAACACACGGA | 0.7 |
| 534301 | 301 | AAAAGCCCTAACACACGGAT | 0.67 |
| 534302 | 302 | AAAGCCCTAACACACGGATG | 0.7 |
| 534303 | 303 | TCAGAAAATTCAGTAGCACC | 0.67 |
| 534304 | 304 | TACTTCCTGTAGGGCTCTAC | 0.67 |
| 534305 | 305 | ACTTCCTGTAGGGCTCTACT | 0.79 |
| 534306 | 306 | TTCCTGTAGGGCTCTACTAC | 0.67 |
| 534307 | 307 | TCCTGTAGGGCTCTACTACA | 0.82 |
| 534308 | 308 | CTGTAGGGCTCTACTACATA | 0.66 |
| 534309 | 309 | ACCAACTGTCCCAGGGTCCA | 0.62 |
| 534310 | 310 | CAACTGTCCCAGGGTCCACA | 0.7 |
| 534311 | 311 | ACACTAACCATGAGCAGAAA | 0.79 |
| 534312 | 312 | CTCATTTCCAGGCCACTAAC | 0.74 |
| 534313 | 313 | CCTATTGTTGCCAGGACATT | 0.66 |
| 534314 | 314 | CTATTGTTGCCAGGACATTA | 0.68 |
| 534315 | 315 | TATTGTTGCCAGGACATTAT | 0.8 |
| 534316 | 316 | ATTGTTGCCAGGACATTATT | 0.81 |
| 534317 | 317 | TTGCCAGGACATTATTGATC | 0.76 |
| 534318 | 318 | GACATTATTGATCACACCTG | 0.64 |
| 534319 | 319 | ATCAGGGTCTGGATCTTTCA | 0.48 |
| 534320 | 320 | TTCAGAATAGGCTCCATGTA | 0.84 |
| 534321 | 321 | GCACTCTGCTCTTTGATTCT | 0.8 |
| 534322 | 322 | TCTTTGATTCTTCCAATCCC | 0.66 |
| 534323 | 323 | TTTGATTCTTCCAATCCCAC | 0.78 |
| 534324 | 324 | GCCAGCTCCCGGATCTCAAA | 0.8 |
| 534325 | 325 | CCAGCTCCCGGATCTCAAAC | 0.79 |
| 534326 | 326 | CAGCTCCCGGATCTCAAACA | 0.85 |
| 534327 | 327 | AGCTCCCGGATCTCAAACAC | 0.8 |
| 534328 | 328 | GCTCCCGGATCTCAAACACC | 0.84 |
| 534329 | 329 | CTCCCGGATCTCAAACACCT | 1.09 |
| 534330 | 330 | TCCCGGATCTCAAACACCTG | 0.84 |

**[Table 10]**

| Compound ID | SEQ ID NO | Sequence (5'to 3') | Relative expression level |
|---|---|---|---|
| 534331 | 331 | GGATCTCAAACACCTGGTCA | 0.72 |
| 534332 | 332 | ATCCGCACAGTGGCGAACAA | 0.92 |
| 534333 | 333 | TCCGCACAGTGGCGAACAAA | 0.75 |
| 534334 | 334 | CCGCACAGTGGCGAACAAAT | 0.89 |
| 534335 | 335 | CGCACAGTGGCGAACAAATT | 0.87 |
| 534336 | 336 | CAAATTCAAAGCTGCCAAGC | 0.81 |
| 534337 | 337 | AAATTCAAAGCTGCCAAGCG | 0.79 |
| 534338 | 338 | AATTCAAAGCTGCCAAGCGT | 0.88 |
| 534339 | 339 | ATTCAAAGCTGCCAAGCGTT | 0.66 |
| 534340 | 340 | TCCAGCAGCTCCTTGATATC | 0.71 |
| 534341 | 341 | TCCAGCACGCGAGGCAAATA | 0.54 |
| 534342 | 342 | CAGCACGCGAGGCAAATAGA | 0.74 |
| 534343 | 343 | AGCACGCGAGGCAAATAGAC | 0.87 |
| 534344 | 344 | GCACGCGAGGCAAATAGACC | 0.83 |
| 534345 | 345 | CACGCGAGGCAAATAGACCT | 0.94 |
| 534346 | 346 | ACGCGAGGCAAATAGACCTT | 0.28 |
| 534347 | 347 | CGCGAGGCAAATAGACCTTA | 0.34 |
| 534348 | 348 | GCGAGGCAAATAGACCTTAA | 0.77 |
| 534349 | 349 | CGAGGCAAATAGACCTTAAA | 0.85 |
| 534350 | 350 | GAGGCAAATAGACCTTAAAC | 0.75 |
| 534351 | 351 | AGGCAAATAGACCTTAAACT | 0.59 |
| 534352 | 352 | GGCAAATAGACCTTAAACTC | 0.56 |
| 534353 | 353 | GCAAATAGACCTTAAACTCA | 0.7 |
| 534354 | 354 | CAAATAGACCTTAAACTCAG | 0.8 |
| 534355 | 355 | AAATAGACCTTAAACTCAGA | 0.75 |
| 534356 | 356 | AATAGACCTTAAACTCAGAC | 0.75 |
| 534357 | 357 | ATAGACCTTAAACTCAGACC | 0.6 |
| 534358 | 358 | TAGACCTTAAACTCAGACCT | 0.74 |
| 534359 | 359 | ACCTTAAACTCAGACCTCAC | 0.79 |
| 534360 | 360 | CCTTAAACTCAGACCTCACA | 0.9 |
| 534361 | 361 | CTCAGACCTCACAGCCACAG | 0.87 |
| 534362 | 362 | TCAGACCTCACAGCCACAGA | 0.6 |
| 534363 | 363 | CAGACCTCACAGCCACAGAA | 0.56 |
| 534364 | 364 | AGACCTCACAGCCACAGAAA | 0.62 |
| 534365 | 365 | GACCTCACAGCCACAGAAAG | 0.71 |
| 534366 | 366 | ACCTCACAGCCACAGAAAGT | 0.86 |
| 534367 | 367 | CCTCACAGCCACAGAAAGTA | 1.03 |
| 534368 | 368 | TCACAGCCACAGAAAGTAGC | 0.8 |
| 534369 | 369 | CACAGCCACAGAAAGTAGCC | 0.77 |
| 534370 | 370 | CAGCCACAGAAAGTAGCCCC | 0.78 |
| 534371 | 371 | AGCCACAGAAAGTAGCCCCA | 0.93 |
| 534372 | 372 | GCCACAGAAAGTAGCCCCAG | 0.78 |
| 534373 | 373 | CCACAGAAAGTAGCCCCAGG | 0.84 |
| 534374 | 374 | AGAAAGTAGCCCCAGGGCTT | 0.76 |
| 534375 | 375 | AAGGCCGCTGTACGTTCCTT | 0.55 |
| 534376 | 376 | AGGCCGCTGTACGTTCCTTC | 0.71 |
| 534377 | 377 | GGCCGCTGTACGTTCCTTCT | 0.74 |
| 534378 | 378 | GCCGCTGTACGTTCCTTCTC | 0.62 |
| 534379 | 379 | CCGCTGTACGTTCCTTCTCC | 0.56 |
| 534380 | 380 | CGCTGTACGTTCCTTCTCCT | 0.52 |
| 534381 | 381 | GCTGTACGTTCCTTCTCCTT | 0.32 |
| 534382 | 382 | CTGTACGTTCCTTCTCCTTC | 0.59 |
| 534383 | 383 | TGTACGTTCCTTCTCCTTCT | 0.47 |
| 534384 | 384 | TCTTGACACAGCTTAGGACA | 0.61 |
| 534385 | 385 | CTTGACACAGCTTAGGACAT | 0.61 |
| 534386 | 386 | AGGCACCAGTAGTACACATA | 0.7 |
| 534387 | 387 | GGCACCAGTAGTACACATAA | 0.99 |
| 534388 | 388 | AACACCTGACCAGCTACATT | 1.01 |
| 534389 | 389 | ACACCTGACCAGCTACATTT | 0.86 |
| 534390 | 390 | CACCTGACCAGCTACATTTC | 0.87 |
| 534391 | 391 | AGCCAAGCGGGGCAACAAAT | 0.88 |
| 534392 | 392 | GCCAAGCGGGGCAACAAATT | 0.88 |
| 534393 | 393 | CCAAGCGGGGCAACAAATTA | 0.95 |
| 534394 | 394 | CAAGCGGGGCAACAAATTAA | 0.95 |
| 534395 | 395 | GCAACAAATTAAGGATTGTC | 0.67 |
| 534396 | 396 | ATCAGCGAGTTCTTGCTATT | 0.49 |
| 534397 | 397 | TCAGCGAGTTCTTGCTATTC | 0.35 |
| 534398 | 398 | CAGCGAGTTCTTGCTATTCC | 0.27 |
| 534399 | 399 | AGCGAGTTCTTGCTATTCCT | 0.34 |
| 534400 | 400 | CGAGTTCTTGCTATTCCTGC | 0.37 |
| 534401 | 401 | GAGTTCTTGCTATTCCTGCA | 0.25 |
| 534402 | 402 | GTTCTTGCTATTCCTGCATT | 0.51 |
| 534403 | 403 | CTTGCTATTCCTGCATTTCA | 0.34 |
| 534404 | 404 | TGCTATTCCTGCATTTCAGC | 0.64 |
| 534405 | 405 | GCTATTCCTGCATTTCAGCA | 0.58 |
| 534406 | 406 | TATTCCTGCATTTCAGCACC | 0.69 |
| 534407 | 407 | ATTCCTGCATTTCAGCACCC | 0.78 |
| 534408 | 408 | CCTGCATTTCAGCACCCACT | 0.59 |
| 534409 | 409 | TCCATTTCTTCTCTCAGACG | 0.5 |
| 534410 | 410 | CCTCCTAATACTCAGGCCAA | 0.91 |
| 534411 | 411 | CTCCTAATACTCAGGCCAAA | 0.84 |
| 534412 | 412 | TCCTAATACTCAGGCCAAAC | 0.94 |
| 534413 | 413 | GATCCGATCATCCCGATTCA | 0.68 |
| 534414 | 414 | ATCCGATCATCCCGATTCAT | 0.5 |
| 534415 | 415 | TCATCCCGATTCATGCCCTT | 0.6 |
| 534416 | 416 | ATCCCGATTCATGCCCTTCT | 0.41 |
| 534417 | 417 | CGATTCATGCCCTTCTCTTT | 0.5 |
| 534418 | 418 | TTGGCCAAGGTCTCATCAAA | 0.79 |
| 534419 | 419 | TCTGCATCTCCTTCGGCTCA | 0.71 |
| 534420 | 420 | CTGCATCTCCTTCGGCTCAC | 0.64 |
| 534421 | 421 | AGGGCTCGCTTCACCTCAAA | 0.76 |
| 534422 | 422 | GGGCTCGCTTCACCTCAAAT | 0.74 |
| 534423 | 423 | GGCTCGCTTCACCTCAAATT | 0.5 |
| 534424 | 424 | GCTCGCTTCACCTCAAATTC | 0.53 |
| 534425 | 425 | TCGCTTCACCTCAAATTCCA | 0.7 |
| 534426 | 426 | CGCTTCACCTCAAATTCCAC | 0.76 |
| 534427 | 427 | GATGCCACCTTTCCTCTCAT | 0.73 |
| 534428 | 428 | CCTTTCCTCTCATTGGCATC | 0.63 |
| 534429 | 429 | CATCTGAGCTGGAAACCAAT | 0.76 |
| 534430 | 430 | ATCTGAGCTGGAAACCAATT | 0.86 |
| 534431 | 431 | TCTGAGCTGGAAACCAATTC | 0.72 |
| 534432 | 432 | CTGAGCTGGAAACCAATTCA | 0.69 |
| 534433 | 433 | CTCAGCTGCCGCCGCCAGCA | 0.78 |
| 534434 | 434 | TCAGCTGCCGCCGCCAGCAC | 0.99 |
| 534435 | 435 | CACCGCCCGCCTTCCCCGCT | 0.93 |
| 534436 | 436 | ACCGCCCGCCTTCCCCGCTG | 0.77 |
| 534437 | 437 | CCGCCCGCCTTCCCCGCTGT | 0.77 |
| 534438 | 438 | CGCCCGCCTTCCCCGCTGTC | 0.68 |
| 534439 | 439 | CGCCTTCCCCGCTGTCCTCT | 0.66 |

### Example 2: Inhibition of MTOR in SNB-19 Cells by 5-10-5 2'-MOE Gapmers (multiple doses)

The 5-10-5 2'-MOE gapmer modified oligonucleotide prepared in Example 1 was tested at various doses in SNB-19 cells.

SNB-19 cells, cultured at a density of 10,000 cells/well, were treated with modified oligonucleotides at concentrations of 0.02, 0.06, 0.19, 0.56, 1.67, 5, and 15 µM by free uptake. Total RNA was extracted from the cells after 24 hours of treatment. The mRNA expression levels of the MTOR gene relative to RPL13, one of the housekeeping genes, by quantitative real-time PCR.. The mRNA levels were measured using the human RPL13 primer-probe set and human MTOR primer-probe set 3 used in Example 1. The results represent the relative expression level of MTOR mRNA compared to untreated control cells.

IC₅₀ value was calculated using the absolute IC₅₀ formula in GraphPad Prism, version 10 (GraphPad Software). The table below shows the expression levels of human MTOR mRNA following treatment with 5-10-5 2'-MOE gapmer-modified oligonucleotides. The results in Table 11 show the relative MTOR mRNA levels at 15 µM and the corresponding IC₅₀ values for the modified oligonucleotides.

**[Table 11]**

| Compound ID | SEQ ID NO | 15µM | IC₅₀(µM) |
|---|---|---|---|
| 534010 | 10 | 0.29 | 1.34 |
| 534011 | 11 | 0.17 | 0.53 |
| 534012 | 12 | 0.12 | 0.11 |
| 534013 | 13 | 0.11 | 0.92 |
| 534014 | 14 | 0.31 | 5.37 |
| 534028 | 28 | 0.31 | 3.39 |
| 534032 | 32 | 0.23 | 0.77 |
| 534035 | 35 | 0.32 | 1.81 |
| 534071 | 71 | 0.19 | 0.96 |
| 534095 | 95 | 0.21 | 1.17 |
| 534102 | 102 | 0.20 | 1.30 |
| 534105 | 105 | 0.32 | 3.21 |
| 534106 | 106 | 0.24 | 1.83 |
| 534118 | 118 | 0.27 | 1.09 |
| 534319 | 319 | 0.27 | 2.63 |
| 534396 | 396 | 0.29 | 2.47 |
| 534397 | 397 | 0.33 | 2.05 |
| 534398 | 398 | 0.19 | 1.30 |
| 534399 | 399 | 0.20 | 1.40 |
| 534400 | 400 | 0.20 | 1.21 |
| 534401 | 401 | 0.22 | 1.09 |
| 534402 | 402 | 0.17 | 1.56 |
| 534403 | 403 | 0.30 | 2.36 |
| 534409 | 409 | 0.23 | 1.18 |
| 534414 | 414 | 0.35 | 3.41 |
| 534416 | 416 | 0.32 | 3.72 |
| 534423 | 423 | 0.35 | 1.28 |
| 534424 | 424 | 0.38 | 8.42 |

### Example 3: Inhibition of MTOR in SNB-19 Cells by oligonucleotides having mixed backbone chemistry (single dose)

Additional gapmers were designed based on the sequences of the oligonucleotides disclosed in the experiment described above. The oligonucleotides were designed as 5-10-5 2'-MOE. The 5-10-5 2'-MOE gapmer was 20 nucleotide in length, wherein the central gap segment consists of 10 2'-deoxyribonucleosides and was flanked by wing segments in the 5' and 3' directions, each containing five (5) nucleotides. Each nucleoside within the 5' wing segment and the 3' wing segment has a 2'-MOE modification. The internucleoside linkages throughout each gapmer was phosphodiester or phosphorothioate linkages. The internucleoside linkage of each oligonucleotide was indicated in the backbone column, wherein 'o' indicated a phosphodiester linkage and 's' indicated a phosphorothioate linkage. All cytosine residues throughout each gapmer was 5-methylcytosine. Each gapmer listed in the table below was targeted to the human MTOR transcript sequence disclosed herein (Ensembl transcript ID: ENST00000361445.9).

In Table 12 below, SEQ ID NO corresponds to the nucleic acid sequence listed in Tables 1 to 4, provided that, in the corresponding nucleic acid sequences of Tables 1 to 4, the cytosine (C) residues are replaced with 5-methylcytosine and the internucleoside linkages include a PO bond instead of a PS bond.

**[Table 12]**

| Compound ID | SEQ ID NO | Sequence(5'to 3') | Internucleoside linkage |
|---|---|---|---|
| 574001 | 11 | GGCTACACTTTATACTTTGT | sossssssssssssssoss |
| 574002 | 11 | GGCTACACTTTATACTTTGT | sosssssssssssssooss |
| 574003 | 11 | GGCTACACTTTATACTTTGT | soossssssssssssooss |
| 574004 | 11 | GGCTACACTTTATACTTTGT | sososssssssssssosos |
| 574005 | 11 | GGCTACACTTTATACTTTGT | sooosssssssssssooss |
| 574006 | 11 | GGCTACACTTTATACTTTGT | sooosssssssssssooos |
| 574007 | 11 | GGCTACACTTTATACTTTGT | soooossssssssssooss |
| 574008 | 12 | GCTACACTTTATACTTTGTG | sossssssssssssssoss |
| 574009 | 12 | GCTACACTTTATACTTTGTG | sosssssssssssssooss |
| 574010 | 12 | GCTACACTTTATACTTTGTG | sosssssssssssssooss |
| 574011 | 12 | GCTACACTTTATACTTTGTG | sososssssssssssosos |
| 574012 | 12 | GCTACACTTTATACTTTGTG | sooosssssssssssooss |
| 574013 | 12 | GCTACACTTTATACTTTGTG | sooosssssssssssooos |
| 574014 | 12 | GCTACACTTTATACTTTGTG | soooossssssssssooss |
| 574015 | 28 | ACCAGACCTTCCCTGTGTTC | sossssssssssssssoss |
| 574016 | 28 | ACCAGACCTTCCCTGTGTTC | sosssssssssssssooss |
| 574017 | 28 | ACCAGACCTTCCCTGTGTTC | soossssssssssssooss |
| 574018 | 28 | ACCAGACCTTCCCTGTGTTC | sososssssssssssosos |
| 574019 | 28 | ACCAGACCTTCCCTGTGTTC | sooosssssssssssooss |
| 574020 | 28 | ACCAGACCTTCCCTGTGTTC | sooosssssssssssooos |
| 574021 | 28 | ACCAGACCTTCCCTGTGTTC | sooosssssssssssooss |
| 574022 | 95 | GTTCTCTTGTGAGTTAAGTC | sossssssssssssssoss |
| 574023 | 95 | GTTCTCTTGTGAGTTAAGTC | sosssssssssssssooss |
| 574024 | 95 | GTTCTCTTGTGAGTTAAGTC | soossssssssssssooss |
| 574025 | 95 | GTTCTCTTGTGAGTTAAGTC | sososssssssssssosos |
| 574026 | 95 | GTTCTCTTGTGAGTTAAGTC | sooosssssssssssooss |
| 574027 | 95 | GTTCTCTTGTGAGTTAAGTC | sooosssssssssssooos |
| 574028 | 95 | GTTCTCTTGTGAGTTAAGTC | sooosssssssssssooss |
| 574029 | 102 | AGTTAAGTCAAAACCCGTAT | sossssssssssssssoss |
| 574030 | 102 | AGTTAAGTCAAAACCCGTAT | sosssssssssssssooss |
| 574031 | 102 | AGTTAAGTCAAAACCCGTAT | soossssssssssssooss |
| 574032 | 102 | AGTTAAGTCAAAACCCGTAT | sososssssssssssosos |
| 574033 | 102 | AGTTAAGTCAAAACCCGTAT | sooosssssssssssooss |
| 574034 | 102 | AGTTAAGTCAAAACCCGTAT | sooosssssssssssooos |
| 574035 | 102 | AGTTAAGTCAAAACCCGTAT | sooosssssssssssooss |
| 574036 | 105 | AGTCAAAACCCGTATTTCTA | sossssssssssssssoss |
| 574037 | 105 | AGTCAAAACCCGTATTTCTA | sosssssssssssssooss |
| 574038 | 105 | AGTCAAAACCCGTATTTCTA | soossssssssssssooss |
| 574039 | 105 | AGTCAAAACCCGTATTTCTA | sososssssssssssosos |
| 574040 | 105 | AGTCAAAACCCGTATTTCTA | sooosssssssssssooss |
| 574041 | 105 | AGTCAAAACCCGTATTTCTA | sooosssssssssssooos |
| 574042 | 105 | AGTCAAAACCCGTATTTCTA | sooosssssssssssooss |
| 574043 | 106 | GTCAAAACCCGTATTTCTAA | sossssssssssssssoss |
| 574044 | 106 | GTCAAAACCCGTATTTCTAA | sosssssssssssssooss |
| 574045 | 106 | GTCAAAACCCGTATTTCTAA | soossssssssssssooss |
| 574046 | 106 | GTCAAAACCCGTATTTCTAA | sososssssssssssosos |
| 574047 | 106 | GTCAAAACCCGTATTTCTAA | sooosssssssssssooss |
| 574048 | 106 | GTCAAAACCCGTATTTCTAA | sooosssssssssssooos |
| 574049 | 106 | GTCAAAACCCGTATTTCTAA | sooosssssssssssooss |
| 574050 | 319 | ATCAGGGTCTGGATCTTTCA | sosssssssssssssooss |
| 574051 | 319 | ATCAGGGTCTGGATCTTTCA | sosssssssssssssooss |
| 574052 | 319 | ATCAGGGTCTGGATCTTTCA | soossssssssssssooss |
| 574053 | 319 | ATCAGGGTCTGGATCTTTCA | sososssssssssssosos |
| 574054 | 319 | ATCAGGGTCTGGATCTTTCA | sooosssssssssssooss |
| 574055 | 319 | ATCAGGGTCTGGATCTTTCA | sooosssssssssssooos |
| 574056 | 319 | ATCAGGGTCTGGATCTTTCA | soooossssssssssooss |
| 574057 | 396 | ATCAGCGAGTTCTTGCTATT | sossssssssssssssoss |
| 574058 | 396 | ATCAGCGAGTTCTTGCTATT | sosssssssssssssooss |
| 574059 | 396 | ATCAGCGAGTTCTTGCTATT | soossssssssssssooss |
| 574060 | 396 | ATCAGCGAGTTCTTGCTATT | sososssssssssssosos |
| 574061 | 396 | ATCAGCGAGTTCTTGCTATT | sooosssssssssssooss |
| 574062 | 396 | ATCAGCGAGTTCTTGCTATT | sooosssssssssssooos |
| 574063 | 396 | ATCAGCGAGTTCTTGCTATT | soooossssssssssooss |
| 574064 | 398 | CAGCGAGTTCTTGCTATTCC | sossssssssssssssoss |
| 574065 | 398 | CAGCGAGTTCTTGCTATTCC | sosssssssssssssooss |
| 574066 | 398 | CAGCGAGTTCTTGCTATTCC | soossssssssssssooss |
| 574067 | 398 | CAGCGAGTTCTTGCTATTCC | sososssssssssssosos |
| 574068 | 398 | CAGCGAGTTCTTGCTATTCC | sooosssssssssssooss |
| 574069 | 398 | CAGCGAGTTCTTGCTATTCC | sooosssssssssssooos |
| 574070 | 398 | CAGCGAGTTCTTGCTATTCC | soooossssssssssooss |
| 574071 | 399 | AGCGAGTTCTTGCTATTCCT | sossssssssssssssoss |
| 574072 | 399 | AGCGAGTTCTTGCTATTCCT | sosssssssssssssooss |
| 574073 | 399 | AGCGAGTTCTTGCTATTCCT | soossssssssssssooss |
| 574074 | 399 | AGCGAGTTCTTGCTATTCCT | sososssssssssssosos |
| 574075 | 399 | AGCGAGTTCTTGCTATTCCT | sooosssssssssssooss |
| 574076 | 399 | AGCGAGTTCTTGCTATTCCT | sooosssssssssssooos |
| 574077 | 399 | AGCGAGTTCTTGCTATTCCT | sooosssssssssssooss |
| 574078 | 409 | TCCATTTCTTCTCTCAGACG | sosssssssssssssoss |
| 574079 | 409 | TCCATTTCTTCTCTCAGACG | sossssssssssssooss |
| 574080 | 409 | TCCATTTCTTCTCTCAGACG | soossssssssssssooss |
| 574081 | 409 | TCCATTTCTTCTCTCAGACG | sososssssssssssosos |
| 574082 | 409 | TCCATTTCTTCTCTCAGACG | sooosssssssssssooss |
| 574083 | 409 | TCCATTTCTTCTCTCAGACG | sooosssssssssssooos |
| 574084 | 409 | TCCATTTCTTCTCTCAGACG | soooossssssssssooss |
| 574085 | 414 | ATCCGATCATCCCGATTCAT | sosssssssssssssoss |
| 574086 | 414 | ATCCGATCATCCCGATTCAT | sosssssssssssssooss |
| 574087 | 414 | ATCCGATCATCCCGATTCAT | soossssssssssssooss |
| 574088 | 414 | ATCCGATCATCCCGATTCAT | sososssssssssssosos |
| 574089 | 414 | ATCCGATCATCCCGATTCAT | sooosssssssssssooss |
| 574090 | 414 | ATCCGATCATCCCGATTCAT | sooosssssssssssooos |
| 574091 | 414 | ATCCGATCATCCCGATTCAT | soooossssssssssooss |
| 574092 | 416 | ATCCCGATTCATGCCCTTCT | sossssssssssssssoss |
| 574093 | 416 | ATCCCGATTCATGCCCTTCT | sosssssssssssssooss |
| 574094 | 416 | ATCCCGATTCATGCCCTTCT | soossssssssssssooss |
| 574095 | 416 | ATCCCGATTCATGCCCTTCT | sososssssssssssosos |
| 574096 | 416 | ATCCCGATTCATGCCCTTCT | sooosssssssssssooss |
| 574097 | 416 | ATCCCGATTCATGCCCTTCT | sooosssssssssssooos |
| 574098 | 416 | ATCCCGATTCATGCCCTTCT | soooossssssssssooss |
| 574099 | 403 | CTTGCTATTCCTGCATTTCA | sossssssssssssssoss |
| 574100 | 403 | CTTGCTATTCCTGCATTTCA | sosssssssssssssooss |
| 574101 | 403 | CTTGCTATTCCTGCATTTCA | soossssssssssssooss |
| 574102 | 403 | CTTGCTATTCCTGCATTTCA | sososssssssssssosos |
| 574103 | 403 | CTTGCTATTCCTGCATTTCA | sooosssssssssssooss |
| 574104 | 403 | CTTGCTATTCCTGCATTTCA | sooosssssssssssooos |
| 574105 | 403 | CTTGCTATTCCTGCATTTCA | soooossssssssssooss |
| 574106 | 13 | CTACACTTTATACTTTGTGC | sossssssssssssssoss |
| 574107 | 13 | CTACACTTTATACTTTGTGC | sosssssssssssssooss |
| 574108 | 13 | CTACACTTTATACTTTGTGC | soossssssssssssooss |
| 574109 | 13 | CTACACTTTATACTTTGTGC | sososssssssssssosos |
| 574110 | 13 | CTACACTTTATACTTTGTGC | sooosssssssssssooss |
| 574111 | 13 | CTACACTTTATACTTTGTGC | sooosssssssssssooos |
| 574112 | 13 | CTACACTTTATACTTTGTGC | soooossssssssssooss |
| 574113 | 14 | TACACTTTATACTTTGTGCA | sossssssssssssssoss |
| 574114 | 14 | TACACTTTATACTTTGTGCA | sosssssssssssssooss |
| 574115 | 14 | TACACTTTATACTTTGTGCA | soossssssssssssooss |
| 574116 | 14 | TACACTTTATACTTTGTGCA | sososssssssssssosos |
| 574117 | 14 | TACACTTTATACTTTGTGCA | sooosssssssssssooss |
| 574118 | 14 | TACACTTTATACTTTGTGCA | sooosssssssssssooos |
| 574119 | 14 | TACACTTTATACTTTGTGCA | soooossssssssssooss |

SNB-19 cells, cultured at a density of 7,000 cells/well, were treated with 5 µM of modified oligonucleotides by free uptake. Total RNA was extracted from the cells after 24 hours of treatment. The mRNA expression level of the MTOR gene relative to RPL13, one of the housekeeping genes, was measured by quantitative real-time PCR. mRNA levels were measured using the human RPL13 primer-probe set and human MTOR primer-probe set 3 as described in Example 1. The results represent the relative expression level of human MTOR mRNA compared to untreated control cells.

**[Table 13]**

| Compound ID | Relative expression level of MTOR | Compound ID | Relative expression level of MTOR | Compound ID | Relative expression level of MTOR | Compound ID | Relative expression level of MTOR |
|---|---|---|---|---|---|---|---|
| 574001 | 0.17 | 574026 | 0.43 | 574051 | 0.3 | 574076 | 0.45 |
| 574002 | 0.2 | 574027 | 0.3 | 574052 | 0.31 | 574077 | 0.57 |
| 574003 | 0.22 | 574028 | 0.34 | 574053 | 0.35 | 574078 | 0.35 |
| 574004 | 0.19 | 574029 | 0.28 | 574054 | 0.45 | 574079 | 0.47 |
| 574005 | 0.22 | 574030 | 0.44 | 574055 | 0.45 | 574080 | 0.42 |
| 574006 | 0.27 | 574031 | 0.4 | 574056 | 0.52 | 574081 | 0.45 |
| 574007 | 0.23 | 574032 | 0.44 | 574057 | 0.42 | 574082 | 0.43 |
| 574008 | 0.24 | 574033 | 0.49 | 574058 | 0.55 | 574083 | 0.55 |
| 574009 | 0.2 | 574034 | 0.5 | 574059 | 0.54 | 574084 | 0.54 |
| 574010 | 0.21 | 574035 | 0.51 | 574060 | 0.63 | 574085 | 0.54 |
| 574011 | 0.28 | 574036 | 0.53 | 574061 | 0.67 | 574086 | 0.52 |
| 574012 | 0.39 | 574037 | 0.44 | 574062 | 0.75 | 574087 | 0.69 |
| 574013 | 0.43 | 574038 | 0.55 | 574063 | 0.68 | 574088 | 0.79 |
| 574014 | 0.43 | 574039 | 0.45 | 574064 | 0.24 | 574089 | 0.77 |
| 574015 | 0.4 | 574040 | 0.62 | 574065 | 0.27 | 574090 | 0.72 |
| 574016 | 0.5 | 574041 | 0.72 | 574066 | 0.33 | 574091 | 0.92 |
| 574017 | 0.55 | 574042 | 0.55 | 574067 | 0.38 | 574092 | 0.51 |
| 574018 | 0.52 | 574043 | 0.4 | 574068 | 0.48 | 574093 | 0.44 |
| 574019 | 0.53 | 574044 | 0.37 | 574069 | 0.43 | 574094 | 0.62 |
| 574020 | 0.65 | 574045 | 0.43 | 574070 | 0.53 | 574095 | 0.45 |
| 574021 | 0.6 | 574046 | 0.45 | 574071 | 0.28 | 574096 | 0.59 |
| 574022 | 0.36 | 574047 | 0.35 | 574072 | 0.3 | 574097 | 0.68 |
| 574023 | 0.38 | 574048 | 0.42 | 574073 | 0.3 | 574098 | 0.63 |
| 574024 | 0.3 | 574049 | 0.38 | 574074 | 0.38 | ** | ** |
| 574025 | 0.39 | 574050 | 0.29 | 574075 | 0.4 | ** | ** |

### Example 4: Inhibition of MTOR in A-431 Cells by 5-10-5 MOE gapmers having mixed backbone chemistry (single dose)

A-431 cells, cultured at a density of 7,000 cells/well, were treated with 5 µM of modified oligonucleotides by free uptake. Total RNA was extracted from the cells after 24 hours of treatment. The mRNA expression level of the MTOR gene relative to RPL13, one of the housekeeping genes, was measured by quantitative real-time PCR. mRNA expression levels were measured using the human RPL13 primer-probe set and human MTOR primer-probe set 3 described in Example 1. The results represent the relative expression level of human MTOR mRNA compared to untreated control cells.

**[Table 14]**

| Compound ID | Relative expression level of MTOR | Compound ID | Relative expression level of MTOR | Compound ID | Relative expression level of MTOR |
|---|---|---|---|---|---|
| 574001 | 0.22 | 574034 | 0.19 | 574067 | 0.15 |
| 574002 | 0.22 | 574035 | 0.17 | 574068 | 0.25 |
| 574003 | 0.15 | 574036 | 0.24 | 574069 | 0.24 |
| 574004 | 0.15 | 574037 | 0.28 | 574070 | 0.32 |
| 574005 | 0.18 | 574038 | 0.26 | 574071 | 0.15 |
| 574006 | 0.19 | 574039 | 0.24 | 574072 | 0.14 |
| 574007 | 0.21 | 574040 | 0.37 | 574073 | 0.12 |
| 574008 | 0.14 | 574041 | 0.37 | 574074 | 0.12 |
| 574009 | 0.11 | 574042 | 0.31 | 574075 | 0.18 |
| 574010 | 0.15 | 574043 | 0.23 | 574076 | 0.19 |
| 574011 | 0.17 | 574044 | 0.18 | 574077 | 0.31 |
| 574012 | 0.15 | 574045 | 0.15 | 574078 | 0.23 |
| 574013 | 0.13 | 574046 | 0.19 | 574079 | 0.28 |
| 574014 | 0.17 | 574047 | 0.15 | 574080 | 0.24 |
| 574015 | 0.31 | 574048 | 0.21 | 574081 | 0.3 |
| 574016 | 0.39 | 574049 | 0.15 | 574082 | 0.3 |
| 574017 | 0.44 | 574050 | 0.11 | 574083 | 0.31 |
| 574018 | 0.44 | 574051 | 0.15 | 574084 | 0.28 |
| 574019 | 0.48 | 574052 | 0.09 | 574085 | 0.36 |
| 574020 | 0.53 | 574053 | 0.14 | 574086 | 0.3 |
| 574021 | 0.48 | 574054 | 0.19 | 574087 | 0.37 |
| 574022 | 0.2 | 574055 | 0.19 | 574088 | 0.39 |
| 574023 | 0.19 | 574056 | 0.23 | 574089 | 0.45 |
| 574024 | 0.13 | 574057 | 0.22 | 574090 | 0.46 |
| 574025 | 0.16 | 574058 | 0.31 | 574091 | 0.49 |
| 574026 | 0.19 | 574059 | 0.31 | 574092 | 0.17 |
| 574027 | 0.17 | 574060 | 0.36 | 574093 | 0.23 |
| 574028 | 0.23 | 574061 | 0.37 | 574094 | 0.3 |
| 574029 | 0.15 | 574062 | 0.47 | 574095 | 0.14 |
| 574030 | 0.23 | 574063 | 0.49 | 574096 | 0.29 |
| 574031 | 0.17 | 574064 | 0.12 | 574097 | 0.33 |
| 574032 | 0.22 | 574065 | 0.11 | 574098 | 0.35 |
| 574033 | 0.18 | 574066 | 0.21 | ** | ** |

### Example 5: Inhibition of MTOR in SNB-19 Cells by 5-10-5 MOE gapmers having mixed backbone chemistry (multiple doses)

5-10-5 2'-MOE gapmer modified oligonucleotides having the mixed backbone disclosed in the experiment described above were tested in SNB-19 cells at various doses. SNB-19 cells cultured at a density of 10,000 cells/well were treated by free uptake with modified oligonucleotides at concentrations of 0.02, 0.06, 0.19, 0.56, 1.67, 5, and 15 µM. Total RNA was extracted from the cells after 24 hours of treatment.

The mRNA expression level of the MTOR gene for RPL13, one of the housekeeping genes, was measured by quantitative real-time PCR. The mRNA levels were measured using the human RPL13 primer-probe set and human MTOR primer-probe set 3 as described in Example 1 above.

The results represent the relative MTOR mRNA levels compared to untreated control cells. IC₅₀ was calculated using the "absolute IC₅₀" formula in GraphPad Prism, version 10 (GraphPad Software). The results in Table 15 show the relative MTOR mRNA levels at 15 µM and the corresponding IC₅₀ values for the modified oligonucleotides.

**[Table 15]**

| Compound ID | 15µM | IC₅₀(µM) | Compound ID | 15µM | IC₅₀(µM) |
|---|---|---|---|---|---|
| 574001 | 0.12 | 0.35 | 574051 | 0.21 | 1.35 |
| 574002 | 0.17 | 0.73 | 574052 | 0.23 | 4.59 |
| 574003 | 0.18 | 1.04 | 574053 | 0.22 | 1.55 |
| 574004 | 0.15 | 0.47 | 574054 | 0.29 | 3.09 |
| 574005 | 0.19 | 0.91 | 574055 | 0.26 | 3.34 |
| 574006 | 0.14 | 1.35 | 574056 | 0.38 | 10.62 |
| 574007 | 0.18 | 1.01 | 574057 | 0.28 | 0.73 |
| 574008 | 0.18 | 0.82 | 574064 | 0.16 | 0.5 |
| 574009 | 0.23 | 1.02 | 574065 | 0.15 | 0.87 |
| 574010 | 0.18 | 1 | 574066 | 0.25 | 2.11 |
| 574011 | 0.16 | 0.87 | 574067 | 0.24 | 1.84 |
| 574012 | 0.27 | 2.6 | 574071 | 0.17 | 0.77 |
| 574013 | 0.24 | 4.18 | 574072 | 0.15 | 0.94 |
| 574014 | 0.24 | 2.71 | 574073 | 0.26 | 2.35 |
| 574022 | 0.19 | 1.52 | 574074 | 0.32 | 3.46 |
| 574023 | 0.19 | 1.02 | 574075 | 0.42 | 9.19 |
| 574024 | 0.21 | 1.24 | 574078 | 0.27 | 2.01 |
| 574025 | 0.23 | 1.35 | 574086 | 0.41 | 6.72 |
| 574026 | 0.26 | 2.6 | 574092 | 0.35 | 2.49 |
| 574027 | 0.29 | 2.31 | 574095 | 0.25 | 1.34 |
| 574028 | 0.31 | 2.66 | 574099 | 0.3 | 5.33 |
| 574029 | 0.2 | 1.34 | 574100 | 0.37 | 4.25 |
| 574030 | 0.22 | 1.43 | 574101 | 0.43 | 8.92 |
| 574031 | 0.21 | 1.59 | 574102 | 0.38 | 5.62 |
| 574032 | 0.3 | 2.02 | 574103 | 0.5 | >15 |
| 574033 | 0.27 | 3.28 | 574104 | 0.55 | >15 |
| 574034 | 0.32 | 5.46 | 574105 | 0.52 | >15 |
| 574035 | 0.29 | 3.87 | 574106 | 0.26 | 2.24 |
| 574036 | 0.27 | 2.04 | 574107 | 0.3 | 1.91 |
| 574037 | 0.3 | 3.94 | 574108 | 0.29 | 3.65 |
| 574038 | 0.27 | 4.76 | 574109 | 0.27 | 2 |
| 574039 | 0.25 | 1.94 | 574110 | 0.25 | 3.35 |
| 574043 | 0.29 | 3.26 | 574111 | 0.38 | 7.64 |
| 574044 | 0.24 | 1.4 | 574112 | 0.37 | 5.87 |
| 574045 | 0.36 | 5.64 | 574113 | 0.34 | 3.87 |
| 574046 | 0.3 | 3.39 | 574114 | 0.47 | 9.82 |
| 574047 | 0.29 | 4.18 | 574115 | 0.36 | 3.75 |
| 574048 | 0.24 | 3.24 | 574116 | 0.31 | 3.4 |
| 574049 | 0.29 | 3.94 | 574117 | 0.43 | 5.67 |
| 574050 | 0.25 | 2.3 | 574118 | 0.41 | 5.42 |

### Example 6: Inhibition of MTOR in A-431Cell by 5-10-5 2'-MOE gapmers having mixed backbone chemistry (multiple doses)

The 5-10-5 2'-MOE gapmer-modified oligonucleotides having the mixed backbone disclosed in the experiment described above were tested in A-431 cells at various concentrations. A-431 cells cultured at a density of 10,000 cells/well were treated by free uptake with modified oligonucleotides at concentrations of 0.02, 0.06, 0.19, 0.56, 1.67, 5, and 15 µM. Total RNA was extracted from the cells after 24 hours of treatment.

The mRNA expression level of the MTOR gene was measured relative to RPL13, one of the housekeeping genes, by quantitative real-time PCR. The mRNA levels were measured using the human RPL13 primer-probe set and human MTOR primer-probe set 3 as described in Example 1 above.

The results in Table 16 show the relative MTOR mRNA levels at 15 µM as compared with the untreated control cells, and the corresponding IC₅₀ value. IC₅₀ value was calculated using the absolute IC₅₀ formula in GraphPad Prism, version 10 (GraphPad Software).

**[Table 16]**

| Compound ID | 15µM | IC₅₀(µM) | Compound ID | 15µM | IC₅₀(µM) |
|---|---|---|---|---|---|
| 574001 | 0.19 | 0.15 | 574051 | 0.19 | 0.24 |
| 574002 | 0.16 | 0.09 | 574052 | 0.21 | 0.62 |
| 574003 | 0.15 | 0.1 | 574053 | 0.19 | 0.39 |
| 574004 | 0.14 | 0.07 | 574054 | 0.19 | 0.85 |
| 574005 | 0.15 | 0.08 | 574055 | 0.19 | 0.61 |
| 574006 | 0.17 | 0.1 | 574056 | 0.29 | 1.38 |
| 574007 | 0.14 | 0.13 | 574057 | 0.19 | 0.19 |
| 574008 | 0.14 | 0.07 | 574064 | 0.14 | 0.24 |
| 574009 | 0.1 | 0.04 | 574065 | 0.14 | 0.22 |
| 574010 | 0.15 | 0.07 | 574066 | 0.21 | 0.44 |
| 574011 | 0.12 | 0.04 | 574067 | 0.2 | 0.32 |
| 574012 | 0.17 | 0.2 | 574071 | 0.11 | 0.08 |
| 574013 | 0.11 | 0.21 | 574072 | 0.14 | 0.06 |
| 574014 | 0.13 | 0.16 | 574073 | 0.13 | 0.1 |
| 574022 | 0.14 | 0.11 | 574074 | 0.13 | 0.12 |
| 574023 | 0.16 | 0.15 | 574075 | 0.19 | 0.19 |
| 574024 | 0.15 | 0.16 | 574078 | 0.13 | 0.09 |
| 574025 | 0.17 | 0.1 | 574086 | 0.32 | 0.85 |
| 574026 | 0.19 | 0.35 | 574092 | 0.29 | 1.1 |
| 574027 | 0.2 | 0.44 | 574095 | 0.16 | 0.48 |
| 574028 | 0.21 | 0.67 | 574099 | 0.14 | 0.27 |
| 574029 | 0.17 | 0.46 | 574100 | 0.18 | 0.47 |
| 574030 | 0.11 | 0.32 | 574101 | 0.18 | 0.55 |
| 574031 | 0.11 | 0.28 | 574102 | 0.18 | 0.62 |
| 574032 | 0.22 | 0.36 | 574103 | 0.24 | 0.96 |
| 574033 | 0.25 | 0.51 | 574104 | 0.23 | 1.66 |
| 574034 | 0.23 | 0.88 | 574105 | 0.36 | 3.08 |
| 574035 | 0.23 | 0.82 | 574106 | 0.13 | 0.15 |
| 574036 | 0.41 | 4.45 | 574107 | 0.15 | 0.13 |
| 574037 | 0.25 | 1.84 | 574108 | 0.18 | 0.16 |
| 574038 | 0.25 | 1.07 | 574109 | 0.15 | 0.11 |
| 574039 | 0.25 | 0.56 | 574110 | 0.17 | 0.21 |
| 574043 | 0.23 | 0.87 | 574111 | 0.21 | 0.52 |
| 574044 | 0.22 | 0.43 | 574112 | 0.18 | 0.46 |
| 574045 | 0.23 | 0.75 | 574113 | 0.27 | 0.55 |
| 574046 | 0.25 | 0.47 | 574114 | 0.3 | 0.51 |
| 574047 | 0.15 | 0.52 | 574115 | 0.26 | 0.45 |
| 574048 | 0.18 | 0.91 | 574116 | 0.26 | 0.46 |
| 574049 | 0.23 | 0.68 | 574117 | 0.24 | 0.21 |
| 574050 | 0.19 | 0.21 | 574118 | 0.34 | 0.83 |

### Example 7: Inhibition of MTOR in SNB-19 Cells by LNA oligonucleotides (single dose)

LNA-modified oligonucleotides targeting human MTOR RNA were designed, and tested for their in vitro effects on MTOR mRNA. The modified oligonucleotides in Table 17 below had phosphorothioate (PS) internucleoside linkages (PS linkages) and contained no modified bases. The sequences in Table 17 are shown in the 5' to 3' direction, and 'D' represents the 2'-deoxyribosyl sugar moiety and 'L' represents the LNA sugar moiety in the sugar moiety expressions,

**[Table 17]**

| Compound ID | SEQ ID NO | Sequence (5' to 3') | Sugar moiety |
|---|---|---|---|
| 536001 | 106 | GTCAAAACCCGTATTTCTAA | LDDLLLDDDDDDDDDLLDLL |
| 536002 | 106 | GTCAAAACCCGTATTTCTAA | LLDLLLDDDDDDDDDLLDLL |
| 536003 | 106 | GTCAAAACCCGTATTTCTAA | LDDLLLDDDDDDDDLLLDLL |
| 536004 | 106 | GTCAAAACCCGTATTTCTAA | LLDLLLDDDDDDDDLLLDLL |
| 536005 | 106 | GTCAAAACCCGTATTTCTAA | LDDLLDDDDDDDDDDLLDLL |
| 536006 | 106 | GTCAAAACCCGTATTTCTAA | LLDLLDDDDDDDDDDLLDLL |
| 536007 | 106 | GTCAAAACCCGTATTTCTAA | LDDLLDDDDDDDDDLLLDLL |
| 536008 | 106 | GTCAAAACCCGTATTTCTAA | LLDLLDDDDDDDDDLLLDLL |
| 536009 | 106 | GTCAAAACCCGTATTTCTAA | LDDLLLDDDDDDDDDDLDLL |
| 536010 | 106 | GTCAAAACCCGTATTTCTAA | LLDLLLDDDDDDDDDDLDLL |
| 536011 | 106 | GTCAAAACCCGTATTTCTAA | LDDLLLDDDDDDDDLDLDLL |
| 536012 | 106 | GTCAAAACCCGTATTTCTAA | LLDLLLDDDDDDDDLDLDLL |
| 536013 | 106 | GTCAAAACCCGTATTTCTAA | LDDLLDDDDDDDDDDDLDLL |
| 536014 | 106 | GTCAAAACCCGTATTTCTAA | LLDLLDDDDDDDDDDDLDLL |
| 536015 | 106 | GTCAAAACCCGTATTTCTAA | LDDLLDDDDDDDDDLDLDLL |
| 536016 | 106 | GTCAAAACCCGTATTTCTAA | LLDLLDDDDDDDDDLDLDLL |
| 536017 | 106 | GTCAAAACCCGTATTTCTAA | LDLLLLDDDDDDDDDLLDLL |
| 536018 | 106 | GTCAAAACCCGTATTTCTAA | LDLLLLDDDDDDDDLLLDLL |
| 536019 | 106 | GTCAAAACCCGTATTTCTAA | LLLLLLDDDDDDDDDLLDLL |
| 536020 | 106 | GTCAAAACCCGTATTTCTAA | LDLLLDDDDDDDDDDLLDLL |
| 536021 | 106 | GTCAAAACCCGTATTTCTAA | LDLLLDDDDDDDDDLLLDLL |
| 536022 | 106 | GTCAAAACCCGTATTTCTAA | LLLLLDDDDDDDDDDLLDLL |
| 536023 | 106 | GTCAAAACCCGTATTTCTAA | LLLLLDDDDDDDDDLLLDLL |
| 536024 | 106 | GTCAAAACCCGTATTTCTAA | LDLLLLDDDDDDDDDDLDLL |
| 536025 | 106 | GTCAAAACCCGTATTTCTAA | LDLLLLDDDDDDDDLDLDLL |
| 536026 | 106 | GTCAAAACCCGTATTTCTAA | LLLLLLDDDDDDDDDDLDLL |
| 536027 | 106 | GTCAAAACCCGTATTTCTAA | LDLLLDDDDDDDDDDDLDLL |
| 536028 | 106 | GTCAAAACCCGTATTTCTAA | LLLLLLDDDDDDDDLDLDLL |
| 536029 | 106 | GTCAAAACCCGTATTTCTAA | LDLLLDDDDDDDDDLDLDLL |
| 536030 | 106 | GTCAAAACCCGTATTTCTAA | LLLLLDDDDDDDDDDDLDLL |
| 536031 | 106 | GTCAAAACCCGTATTTCTAA | LLLLLDDDDDDDDDLDLDLL |
| 536032 | 106 | GTCAAAACCCGTATTTCTAA | LDDLLLDDDDDDDDDLLLLL |
| 536033 | 106 | GTCAAAACCCGTATTTCTAA | LLDLLLDDDDDDDDDLLLLL |
| 536034 | 106 | GTCAAAACCCGTATTTCTAA | LDDLLLDDDDDDDDLLLLLL |
| 536035 | 106 | GTCAAAACCCGTATTTCTAA | LDLLLLDDDDDDDDDLLLLL |
| 536036 | 409 | TCCATTTCTTCTCTCAGACG | LDLLLLDDDDDDDDDLLLLL |
| 536037 | 409 | TCCATTTCTTCTCTCAGACG | LDLLLLDDDDDDDDDLLDLL |
| 536038 | 409 | TCCATTTCTTCTCTCAGACG | LDLLLLDDDDDDDDLLLDLL |
| 536039 | 409 | TCCATTTCTTCTCTCAGACG | LDDLLLDDDDDDDDDLLLLL |
| 536040 | 409 | TCCATTTCTTCTCTCAGACG | LDDLLLDDDDDDDDDLLDLL |
| 536041 | 409 | TCCATTTCTTCTCTCAGACG | LDDLLDDDDDDDDDDLLLLL |
| 536042 | 409 | TCCATTTCTTCTCTCAGACG | LDDLLLDDDDDDDDLLLLLL |
| 536043 | 409 | TCCATTTCTTCTCTCAGACG | LDDLLLDDDDDDDDLLLDLL |
| 536044 | 409 | TCCATTTCTTCTCTCAGACG | LDLDLLDDDDDDDDDLLLLL |
| 536045 | 409 | TCCATTTCTTCTCTCAGACG | LDLDLLDDDDDDDDDLLDLL |
| 536046 | 409 | TCCATTTCTTCTCTCAGACG | LDLDLDDDDDDDDDDLLLLL |
| 536047 | 409 | TCCATTTCTTCTCTCAGACG | LDLDLLDDDDDDDDLLLLLL |
| 536048 | 409 | TCCATTTCTTCTCTCAGACG | LDDLLLDDDDDDDDLDLLLL |
| 536049 | 409 | TCCATTTCTTCTCTCAGACG | LLLLLLDDDDDDDDDLLDLL |
| 536050 | 409 | TCCATTTCTTCTCTCAGACG | LDDDLLDDDDDDDDDLLLLL |
| 536051 | 409 | TCCATTTCTTCTCTCAGACG | LLDLLLDDDDDDDDDLLLLL |
| 536052 | 409 | TCCATTTCTTCTCTCAGACG | LLDLLLDDDDDDDDDLLDLL |
| 536053 | 409 | TCCATTTCTTCTCTCAGACG | LLDLLLDDDDDDDDLLLDLL |
| 536054 | 409 | TCCATTTCTTCTCTCAGACG | LLLDLLDDDDDDDDDLLLLL |
| 536055 | 409 | TCCATTTCTTCTCTCAGACG | LLLDLLDDDDDDDDDLLDLL |
| 536056 | 409 | TCCATTTCTTCTCTCAGACG | LLLDLDDDDDDDDDDLLLLL |
| 536057 | 409 | TCCATTTCTTCTCTCAGACG | LLLDLLDDDDDDDDLLLDLL |
| 536058 | 409 | TCCATTTCTTCTCTCAGACG | LDLLDLDDDDDDDDDLLLLL |
| 536059 | 409 | TCCATTTCTTCTCTCAGACG | LDLLDLDDDDDDDDLLLLLL |
| 536060 | 409 | TCCATTTCTTCTCTCAGACG | LDDLLLDDDDDDDDDLDLLL |
| 536061 | 409 | TCCATTTCTTCTCTCAGACG | LDLDLLDDDDDDDDDLDLLL |
| 536062 | 409 | TCCATTTCTTCTCTCAGACG | LLLDLLDDDDDDDDDDLLLL |
| 536063 | 409 | TCCATTTCTTCTCTCAGACG | LLLDLLDDDDDDDDLDLLLL |
| 536064 | 409 | TCCATTTCTTCTCTCAGACG | LDDLDLDDDDDDDDDLLLLL |
| 536065 | 409 | TCCATTTCTTCTCTCAGACG | LDDLDLDDDDDDDDDLLDLL |
| 536066 | 409 | TCCATTTCTTCTCTCAGACG | LDDLDLDDDDDDDDLLLLLL |
| 536067 | 409 | TCCATTTCTTCTCTCAGACG | LDDLDDDDDDDDDDDLLLLL |
| 536068 | 409 | TCCATTTCTTCTCTCAGACG | LDLDDLDDDDDDDDDLLLLL |
| 536069 | 409 | TCCATTTCTTCTCTCAGACG | LDLDDLDDDDDDDDLLLLLL |
| 536070 | 409 | TCCATTTCTTCTCTCAGACG | LDLDDLDDDDDDDDDLLDLL |
| 536071 | 416 | ATCCCGATTCATGCCCTTCT | LDLDLLDDDDDDDDDLLDLL |
| 536072 | 416 | ATCCCGATTCATGCCCTTCT | LLLDLLDDDDDDDDDLLDLL |
| 536075 | 416 | ATCCCGATTCATGCCCTTCT | LDLDLDDDDDDDDDDLLDLL |
| 536076 | 416 | ATCCCGATTCATGCCCTTCT | LLLDLDDDDDDDDDDLLDLL |
| 536077 | 416 | ATCCCGATTCATGCCCTTCT | LDLDLDDDDDDDDDLLLDLL |
| 536078 | 416 | ATCCCGATTCATGCCCTTCT | LLLDLDDDDDDDDDLLLDLL |
| 536079 | 416 | ATCCCGATTCATGCCCTTCT | LDLLLLDDDDDDDDDLLDLL |
| 536082 | 416 | ATCCCGATTCATGCCCTTCT | LDLLLDDDDDDDDDDLLDLL |
| 536083 | 416 | ATCCCGATTCATGCCCTTCT | LLLLLDDDDDDDDDDLLDLL |
| 536084 | 416 | ATCCCGATTCATGCCCTTCT | LDLLLDDDDDDDDDLLLDLL |
| 536086 | 416 | ATCCCGATTCATGCCCTTCT | LDDDLLDDDDDDDDDLLDLL |
| 536087 | 416 | ATCCCGATTCATGCCCTTCT | LLDDLLDDDDDDDDDLLDLL |
| 536088 | 416 | ATCCCGATTCATGCCCTTCT | LDDDLLDDDDDDDDLLLDLL |
| 536089 | 416 | ATCCCGATTCATGCCCTTCT | LLDDLLDDDDDDDDLLLDLL |
| 536090 | 416 | ATCCCGATTCATGCCCTTCT | LDDDLDDDDDDDDDDLLDLL |
| 536091 | 416 | ATCCCGATTCATGCCCTTCT | LLDDLDDDDDDDDDDLLDLL |
| 536092 | 416 | ATCCCGATTCATGCCCTTCT | LDDDLDDDDDDDDDLLLDLL |
| 536093 | 416 | ATCCCGATTCATGCCCTTCT | LLDDLDDDDDDDDDLLLDLL |
| 536094 | 416 | ATCCCGATTCATGCCCTTCT | LDDDLLDDDDDDDDDDLDLL |
| 536095 | 416 | ATCCCGATTCATGCCCTTCT | LLDDLLDDDDDDDDDDLDLL |
| 536096 | 416 | ATCCCGATTCATGCCCTTCT | LDDDLLDDDDDDDDLDLDLL |
| 536097 | 416 | ATCCCGATTCATGCCCTTCT | LLDDLLDDDDDDDDLDLDLL |
| 536098 | 416 | ATCCCGATTCATGCCCTTCT | LDDDLDDDDDDDDDDDLDLL |
| 536099 | 416 | ATCCCGATTCATGCCCTTCT | LLDDLDDDDDDDDDDDLDLL |
| 536100 | 416 | ATCCCGATTCATGCCCTTCT | LDDDLDDDDDDDDDLDLDLL |
| 536101 | 416 | ATCCCGATTCATGCCCTTCT | LLDDLDDDDDDDDDLDLDLL |
| 536102 | 416 | ATCCCGATTCATGCCCTTCT | LDDLLLDDDDDDDDDLLDLL |
| 536110 | 13 | CTACACTTTATACTTTGTGC | LDDLLLDDDDDDDDLLLDLL |
| 536111 | 13 | CTACACTTTATACTTTGTGC | LDDLLLDDDDDDDDLLLLLL |
| 536112 | 13 | CTACACTTTATACTTTGTGC | LDDLLLDDDDDDDDDLLDLL |
| 536113 | 13 | CTACACTTTATACTTTGTGC | LDDLLLDDDDDDDDDLLLLL |
| 536115 | 13 | CTACACTTTATACTTTGTGC | LLDLLLDDDDDDDDDLLDLL |
| 536116 | 13 | CTACACTTTATACTTTGTGC | LLDLLLDDDDDDDDDLLLLL |
| 536117 | 13 | CTACACTTTATACTTTGTGC | LDDDLLDDDDDDDDLLLDLL |
| 536118 | 13 | CTACACTTTATACTTTGTGC | LDDDLLDDDDDDDDLLLLLL |
| 536119 | 13 | CTACACTTTATACTTTGTGC | LLDDLLDDDDDDDDLLLDLL |
| 536120 | 13 | CTACACTTTATACTTTGTGC | LLDDLLDDDDDDDDLLLLLL |
| 536121 | 13 | CTACACTTTATACTTTGTGC | LDDDLLDDDDDDDDDLLDLL |
| 536122 | 13 | CTACACTTTATACTTTGTGC | LDDDLLDDDDDDDDDLLLLL |
| 536123 | 13 | CTACACTTTATACTTTGTGC | LLDDLLDDDDDDDDDLLDLL |
| 536124 | 13 | CTACACTTTATACTTTGTGC | LLDDLLDDDDDDDDDLLLLL |
| 536125 | 13 | CTACACTTTATACTTTGTGC | LDLLLLDDDDDDDDLLLDLL |
| 536127 | 13 | CTACACTTTATACTTTGTGC | LDLLLLDDDDDDDDDLLLLL |
| 536128 | 13 | CTACACTTTATACTTTGTGC | LLLLLLDDDDDDDDDLLDLL |
| 536129 | 13 | CTACACTTTATACTTTGTGC | LDDDLLDDDDDDDDDDLDLL |
| 536130 | 13 | CTACACTTTATACTTTGTGC | LDDDLLDDDDDDDDDDLLLL |
| 536131 | 13 | CTACACTTTATACTTTGTGC | LLDDLLDDDDDDDDDDLDLL |
| 536132 | 13 | CTACACTTTATACTTTGTGC | LLDDLLDDDDDDDDDDLLLL |
| 536133 | 13 | CTACACTTTATACTTTGTGC | LDDDLLDDDDDDDDLDLDLL |
| 536134 | 13 | CTACACTTTATACTTTGTGC | LDDDLLDDDDDDDDLDLLLL |
| 536135 | 13 | CTACACTTTATACTTTGTGC | LLDDLLDDDDDDDDLDLDLL |
| 536136 | 13 | CTACACTTTATACTTTGTGC | LLDDLLDDDDDDDDLDLLLL |
| 536137 | 13 | CTACACTTTATACTTTGTGC | LDLLLLDDDDDDDDDDLDLL |
| 536138 | 13 | CTACACTTTATACTTTGTGC | LDLLLLDDDDDDDDDDLLLL |
| 536139 | 13 | CTACACTTTATACTTTGTGC | LLLLLLDDDDDDDDDDLDLL |
| 536140 | 13 | CTACACTTTATACTTTGTGC | LLLLLLDDDDDDDDDDLLLL |
| 536141 | 13 | CTACACTTTATACTTTGTGC | LLDLLLDDDDDDDDLLDDLL |
| 536142 | 13 | CTACACTTTATACTTTGTGC | LLDLLLDDDDDDDDDLDDLL |
| 536143 | 13 | CTACACTTTATACTTTGTGC | LDLDLLDDDDDDDDLLLDLL |
| 536144 | 13 | CTACACTTTATACTTTGTGC | LDLDLLDDDDDDDDLLLLLL |
| 536145 | 13 | CTACACTTTATACTTTGTGC | LDLDLLDDDDDDDDDLLDLL |
| 536146 | 13 | CTACACTTTATACTTTGTGC | LDLDLLDDDDDDDDDLLLLL |
| 536147 | 13 | CTACACTTTATACTTTGTGC | LLLDLLDDDDDDDDLLLDLL |
| 536148 | 13 | CTACACTTTATACTTTGTGC | LLLDLLDDDDDDDDDLLDLL |
| 536149 | 13 | CTACACTTTATACTTTGTGC | LLLDLLDDDDDDDDDLLLLL |
| 536150 | 13 | CTACACTTTATACTTTGTGC | LDLDLLDDDDDDDDDDLDLL |
| 536153 | 14 | TACACTTTATACTTTGTGCA | LLDLLLDDDDDDDDLLLDLL |
| 536154 | 14 | TACACTTTATACTTTGTGCA | LDLLLLDDDDDDDDDLLDLL |
| 536155 | 14 | TACACTTTATACTTTGTGCA | LDLLLLDDDDDDDDLLLDLL |
| 536157 | 14 | TACACTTTATACTTTGTGCA | LDDLLLDDDDDDDDLLLDLL |
| 536160 | 14 | TACACTTTATACTTTGTGCA | LLLLLLDDDDDDDDDLDDLL |
| 536161 | 14 | TACACTTTATACTTTGTGCA | LDDLLLDDDDDDDDDLLLLL |
| 536162 | 14 | TACACTTTATACTTTGTGCA | LDDLLLDDDDDDDDLLLLLL |
| 536163 | 14 | TACACTTTATACTTTGTGCA | LLLLLLDDDDDDDDDDDDLL |
| 536164 | 14 | TACACTTTATACTTTGTGCA | LLDLLLDDDDDDDDDDDDLL |
| 536165 | 14 | TACACTTTATACTTTGTGCA | LLLLLLDDDDDDDDLDDDLL |
| 536166 | 14 | TACACTTTATACTTTGTGCA | LLDLLLDDDDDDDDLDDDLL |
| 536167 | 14 | TACACTTTATACTTTGTGCA | LLLLLDDDDDDDDDDDDDLL |
| 536168 | 14 | TACACTTTATACTTTGTGCA | LLLLLDDDDDDDDDLDDDLL |
| 536169 | 14 | TACACTTTATACTTTGTGCA | LLDLLDDDDDDDDDDDDDLL |
| 536170 | 14 | TACACTTTATACTTTGTGCA | LLDLLDDDDDDDDDLDDDLL |
| 536171 | 14 | TACACTTTATACTTTGTGCA | LDLLLLDDDDDDDDDDDDLL |
| 536172 | 14 | TACACTTTATACTTTGTGCA | LDLLLLDDDDDDDDLDDDLL |
| 536173 | 14 | TACACTTTATACTTTGTGCA | LDLLLDDDDDDDDDDDDDLL |
| 536174 | 14 | TACACTTTATACTTTGTGCA | LDLLLDDDDDDDDDLDDDLL |
| 536175 | 14 | TACACTTTATACTTTGTGCA | LDDLLLDDDDDDDDDDDDLL |
| 536176 | 14 | TACACTTTATACTTTGTGCA | LDDLLLDDDDDDDDLDDDLL |
| 536177 | 14 | TACACTTTATACTTTGTGCA | LDDLLDDDDDDDDDDDDDLL |
| 536178 | 14 | TACACTTTATACTTTGTGCA | LDDLLDDDDDDDDDLDDDLL |
| 536179 | 14 | TACACTTTATACTTTGTGCA | LLDDLLDDDDDDDDDDDDLL |
| 536180 | 14 | TACACTTTATACTTTGTGCA | LLLDLLDDDDDDDDDDDDLL |
| 536181 | 14 | TACACTTTATACTTTGTGCA | LLDDLLDDDDDDDDLDDDLL |
| 536182 | 14 | TACACTTTATACTTTGTGCA | LLLDLLDDDDDDDDLDDDLL |
| 536183 | 14 | TACACTTTATACTTTGTGCA | LLLDLDDDDDDDDDDDDDLL |
| 536184 | 14 | TACACTTTATACTTTGTGCA | LLLDLDDDDDDDDDLDDDLL |
| 536185 | 14 | TACACTTTATACTTTGTGCA | LLLLLLDDDDDDDDDDDLLL |
| 536186 | 14 | TACACTTTATACTTTGTGCA | LLLLLLDDDDDDDDLDDLLL |
| 536187 | 14 | TACACTTTATACTTTGTGCA | LLDLLLDDDDDDDDDDDLLL |
| 536188 | 14 | TACACTTTATACTTTGTGCA | LLDLLLDDDDDDDDLDDLLL |
| 536189 | 14 | TACACTTTATACTTTGTGCA | LLLLLDDDDDDDDDDDDLLL |
| 536190 | 14 | TACACTTTATACTTTGTGCA | LLLLLDDDDDDDDDLDDLLL |
| 536191 | 14 | TACACTTTATACTTTGTGCA | LLDLLDDDDDDDDDDDDLLL |
| 536192 | 14 | TACACTTTATACTTTGTGCA | LLDLLDDDDDDDDDLDDLLL |

SNB-19 cells cultured at a density of 7,000 cells/well were treated with 5 µM of modified oligonucleotides by free uptake. Total RNA was extracted from the cells after 24 hours of treatment. The mRNA expression levels of the MTOR gene relative to RPL13, one of the housekeeping genes, by quantitative real-time PCR.. The mRNA levels were measured using the human RPL13 primer-probe set and human MTOR primer-probe set 3 used in Example 1.

The analysis results represent the relative expression levels of MTOR mRNA compared to untreated control cells. Each gapmer listed in the table below targets the human MTOR transcript disclosed herein (Ensembl Transcript ID: ENST00000361445.9). Table 18 below shows the expression levels of human MTOR mRNA following treatment with LNA-modified oligonucleotides.

**[Table 18]**

| Compound ID | Relative expression level of MTOR | Compound ID | Relative expression level of MTOR | Compound ID | Relative expression level of MTOR |
|---|---|---|---|---|---|
| 536001 | 0.21 | 536050 | 0.31 | 536112 | 0.41 |
| 536002 | 0.17 | 536051 | 0.47 | 536115 | 0.47 |
| 536003 | 0.22 | 536052 | 0.36 | 536117 | 0.25 |
| 536004 | 0.2 | 536053 | 0.45 | 536119 | 0.25 |
| 536005 | 0.25 | 536054 | 0.49 | 536121 | 0.14 |
| 536006 | 0.13 | 536055 | 0.41 | 536122 | 0.41 |
| 536007 | 0.21 | 536056 | 0.5 | 536123 | 0.16 |
| 536008 | 0.17 | 536057 | 0.56 | 536129 | 0.18 |
| 536009 | 0.15 | 536058 | 0.29 | 536130 | 0.25 |
| 536010 | 0.13 | 536059 | 0.41 | 536131 | 0.16 |
| 536011 | 0.17 | 536060 | 0.15 | 536132 | 0.31 |
| 536012 | 0.25 | 536061 | 0.25 | 536133 | 0.1 |
| 536013 | 0.23 | 536062 | 0.48 | 536134 | 0.32 |
| 536014 | 0.13 | 536063 | 0.43 | 536135 | 0.13 |
| 536015 | 0.27 | 536064 | 0.16 | 536136 | 0.32 |
| 536016 | 0.14 | 536065 | 0.16 | 536137 | 0.42 |
| 536017 | 0.13 | 536066 | 0.25 | 536141 | 0.31 |
| 536018 | 0.23 | 536067 | 0.14 | 536142 | 0.14 |
| 536019 | 0.23 | 536068 | 0.21 | 536143 | 0.39 |
| 536020 | 0.16 | 536069 | 0.4 | 536145 | 0.25 |
| 536021 | 0.18 | 536070 | 0.18 | 536148 | 0.39 |
| 536022 | 0.17 | 536071 | 0.23 | 536150 | 0.18 |
| 536023 | 0.21 | 536072 | 0.45 | 536161 | 0.5 |
| 536024 | 0.18 | 536075 | 0.24 | 536163 | 0.34 |
| 536025 | 0.22 | 536076 | 0.34 | 536164 | 0.12 |
| 536026 | 0.22 | 536077 | 0.4 | 536166 | 0.21 |
| 536027 | 0.17 | 536078 | 0.43 | 536167 | 0.3 |
| 536028 | 0.29 | 536079 | 0.37 | 536168 | 0.33 |
| 536029 | 0.2 | 536082 | 0.3 | 536169 | 0.15 |
| 536030 | 0.18 | 536083 | 0.36 | 536170 | 0.14 |
| 536031 | 0.2 | 536084 | 0.44 | 536171 | 0.23 |
| 536032 | 0.15 | 536086 | 0.17 | 536172 | 0.28 |
| 536033 | 0.15 | 536087 | 0.24 | 536173 | 0.17 |
| 536034 | 0.26 | 536088 | 0.24 | 536174 | 0.19 |
| 536035 | 0.19 | 536089 | 0.37 | 536175 | 0.13 |
| 536036 | 0.46 | 536090 | 0.22 | 536176 | 0.11 |
| 536037 | 0.3 | 536091 | 0.2 | 536177 | 0.16 |
| 536038 | 0.42 | 536092 | 0.22 | 536178 | 0.12 |
| 536039 | 0.24 | 536093 | 0.34 | 536179 | 0.19 |
| 536040 | 0.21 | 536094 | 0.16 | 536180 | 0.31 |
| 536041 | 0.2 | 536095 | 0.17 | 536181 | 0.16 |
| 536042 | 0.51 | 536096 | 0.18 | 536182 | 0.39 |
| 536043 | 0.34 | 536097 | 0.3 | 536183 | 0.23 |
| 536044 | 0.42 | 536098 | 0.22 | 536184 | 0.28 |
| 536045 | 0.28 | 536099 | 0.19 | 536187 | 0.2 |
| 536046 | 0.22 | 536100 | 0.25 | 536188 | 0.41 |
| 536047 | 0.43 | 536101 | 0.24 | 536191 | 0.13 |
| 536048 | 0.25 | 536102 | 0.22 | 536192 | 0.21 |
| 536049 | 0.51 | ** | ** | ** | ** |

### Example 8: Inhibition of MTOR in SH-SY5Y Cells by LNA oligonucleotides (single dose)

SH-SY5Y cells cultured at a density of 40,000 cells/well were transfected with 5 µM of modified oligonucleotides using electroporation. After a treatment period of 24 hours, total RNA was extracted from the cells.

The mRNA expression level of the MTOR gene for RPL13, one of the housekeeping genes, was measured by quantitative real-time PCR. The mRNA levels were measured using the human RPL13 primer-probe set and human MTOR primer-probe set 3 used in Example 1 above.

The analysis results represent the relative expression levels of MTOR mRNA compared to untreated control cells. Each gapmer listed in the table below targets the human MTOR transcript disclosed herein (Ensembl Transcript ID: ENST00000361445.9). Table 19 below shows the expression levels of human MTOR mRNA following treatment with LNA-modified oligonucleotides.

**[Table 19]**

| Compoun d ID | Relative expressio n level of MTOR | Compoun d ID | Relative expressio n level of MTOR | Compoun d ID | Relative expressio n level of MTOR | Compoun d ID | Relative expressio n level of MTOR |
|---|---|---|---|---|---|---|---|
| 536001 | 0.44 | 536041 | 0.28 | 536097 | 0.39 | 536148 | 0.35 |
| 536002 | 0.38 | 536042 | 0.36 | 536098 | 0.38 | 536149 | 0.43 |
| 536003 | 0.43 | 536043 | 0.38 | 536099 | 0.4 | 536150 | 0.38 |
| 536004 | 0.6 | 536044 | 0.32 | 536100 | 0.33 | 536153 | 0.2 |
| 536005 | 0.46 | 536045 | 0.55 | 536101 | 0.34 | 536154 | 0.46 |
| 536006 | 0.55 | 536046 | 0.53 | 536102 | 0.35 | 536155 | 0.45 |
| 536007 | 0.42 | 536047 | 0.63 | 536110 | 0.38 | 536157 | 0.46 |
| 536008 | 0.24 | 536048 | 0.51 | 536111 | 0.35 | 536160 | 0.31 |
| 536009 | 0.39 | 536049 | 0.58 | 536112 | 0.3 | 536161 | 0.16 |
| 536010 | 0.34 | 536050 | 0.24 | 536113 | 0.43 | 536162 | 0.28 |
| 536011 | 0.31 | 536051 | 0.44 | 536115 | 0.34 | 536163 | 0.21 |
| 536012 | 0.5 | 536052 | 0.36 | 536116 | 0.39 | 536164 | 0.39 |
| 536013 | 0.52 | 536053 | 0.46 | 536118 | 0.5 | 536165 | 0.2 |
| 536014 | 0.33 | 536054 | 0.6 | 536119 | 0.36 | 536166 | 0.29 |
| 536015 | 0.37 | 536055 | 0.39 | 536120 | 0.44 | 536167 | 0.16 |
| 536016 | 0.29 | 536056 | 0.45 | 536121 | 0.39 | 536168 | 0.24 |
| 536017 | 0.33 | 536057 | 0.53 | 536123 | 0.25 | 536169 | 0.23 |
| 536018 | 0.34 | 536058 | 0.36 | 536124 | 0.49 | 536170 | 0.16 |
| 536019 | 0.4 | 536059 | 0.53 | 536125 | 0.37 | 536171 | 0.35 |
| 536020 | 0.27 | 536060 | 0.19 | 536127 | 0.36 | 536172 | 0.18 |
| 536021 | 0.35 | 536061 | 0.28 | 536128 | 0.38 | 536173 | 0.12 |
| 536022 | 0.33 | 536062 | 0.33 | 536129 | 0.28 | 536174 | 0.22 |
| 536023 | 0.34 | 536063 | 0.72 | 536130 | 0.31 | 536175 | 0.18 |
| 536024 | 0.39 | 536064 | 0.22 | 536131 | 0.2 | 536176 | 0.24 |
| 536025 | 0.32 | 536065 | 0.27 | 536132 | 0.24 | 536177 | 0.24 |
| 536026 | 0.35 | 536066 | 0.38 | 536133 | 0.4 | 536178 | 0.17 |
| 536027 | 0.31 | 536067 | 0.24 | 536134 | 0.31 | 536179 | 0.22 |
| 536028 | 0.32 | 536068 | 0.3 | 536135 | 0.36 | 536180 | 0.17 |
| 536029 | 0.24 | 536069 | 0.48 | 536136 | 0.3 | 536181 | 0.17 |
| 536030 | 0.26 | 536070 | 0.35 | 536137 | 0.28 | 536182 | 0.15 |
| 536031 | 0.32 | 536071 | 0.42 | 536138 | 0.41 | 536183 | 0.33 |
| 536032 | 0.22 | 536075 | 0.38 | 536139 | 0.34 | 536184 | 0.23 |
| 536033 | 0.21 | 536086 | 0.29 | 536140 | 0.3 | 536185 | 0.12 |
| 536034 | 0.25 | 536087 | 0.31 | 536141 | 0.4 | 536186 | 0.24 |
| 536035 | 0.41 | 536088 | 0.29 | 536142 | 0.3 | 536187 | 0.14 |
| 536036 | 0.33 | 536090 | 0.33 | 536143 | 0.34 | 536188 | 0.17 |
| 536037 | 0.25 | 536091 | 0.39 | 536144 | 0.41 | 536189 | 0.21 |
| 536038 | 0.39 | 536094 | 0.21 | 536145 | 0.25 | 536190 | 0.23 |
| 536039 | 0.2 | 536095 | 0.25 | 536146 | 0.31 | 536191 | 0.12 |
| 536040 | 0.24 | 536096 | 0.37 | 536147 | 0.34 | 536192 | 0.15 |

### Example 9: Inhibition of MTOR in SNB-19 Cells by LNA oligonucleotides (multiple doses)

The LNA-modified oligonucleotides prepared in Example 7 were tested in SNB-19 cells at various concentrations. SNB-19 cells cultured at a density of 10,000 cells/well were treated by free uptake at 0.02, 0.06, 0.19, 0.56, 1.67, 5, and 15 µM of concentrations of the modified oligonucleotides. Total RNA was extracted from the cells after 24 hours of treatment. The mRNA expression level of the MTOR gene for RPL13, one of the housekeeping genes, was measured by quantitative real-time PCR. The mRNA levels were measured using the human RPL13 primer-probe set (forward primer sequence: ATTTCTGTGGATCCGAGGAGG, reverse primer sequence: GCCAGTTTCAGTTCTTCAGCAGA, probe sequence: AGTACCGCTCCAAACTCATCCTCTTCC) and human MTOR primer-probe set 3 (forward primer sequence: GAGATCCAGGCTACCTGGT, reverse primer sequence: GGTGGAGTTGACCCCATTC, probe sequence: ATGGACACCAACAAGGACGACCCAGA) used in Example 1 above.

The results in Table 20 show the relative MTOR mRNA levels at 15 µM as compared with untreated control cells and the corresponding IC₅₀ values for the modified oligonucleotides. IC₅₀ value was calculated using the absolute IC₅₀ formula in GraphPad Prism, version 10 (GraphPad Software).

**[Table 20]**

| Compound ID | 15µM | IC₅₀(µM) |
|---|---|---|
| 536008 | 0.12 | 0.32 |
| 536010 | 0.12 | 0.4 |
| 536011 | 0.23 | 1.17 |
| 536014 | 0.15 | 0.32 |
| 536016 | 0.18 | 0.39 |
| 536017 | 0.19 | 0.61 |
| 536018 | 0.31 | 0.83 |
| 536020 | 0.16 | 0.37 |
| 536021 | 1.1 | 0.25 |
| 536022 | 0.14 | 0.24 |
| 536023 | 0.19 | 0.46 |
| 536025 | 0.27 | 0.59 |
| 536026 | 0.14 | 0.72 |
| 536027 | 0.15 | 0.44 |
| 536029 | 0.17 | 0.38 |
| 536030 | 0.15 | 0.51 |
| 536031 | 0.15 | 0.62 |
| 536032 | 0.16 | 0.31 |
| 536033 | 0.14 | 0.27 |
| 536034 | 0.23 | 0.47 |
| 536060 | 0.2 | 0.37 |
| 536086 | 0.22 | 0.5 |
| 536087 | 0.26 | 2.57 |
| 536090 | 0.33 | 0.57 |
| 536094 | 0.12 | 0.48 |
| 536095 | 0.35 | 0.06 |
| 536101 | 0.34 | 2.04 |
| 536170 | 0.1 | 0.17 |
| 536173 | 0.12 | 0.19 |
| 536175 | 0.12 | 0.11 |
| 536178 | 0.11 | 0.09 |
| 536181 | 0.09 | 0.12 |
| 536187 | 0.11 | 0.26 |
| 536191 | 0.1 | 0.23 |
| 536192 | 0.19 | 0.25 |

### Example 10: Inhibition of intracellular MTOR by siRNA and C16-siRNA (multiple doses)

Lipophilic moieties are well known to improve the intracellular uptake of oligonucleotides and siRNA and their delivery to various organs, including the central nervous system (CNS). SNB-19 cells and SK-N-AS cells were treated with siRNA targeting human MTOR RNA, in which C16 (Palmitic acid) was conjugated to the sense strand of each siRNA, and MTOR mRNA expression levels were measured.

In Table 21 below, duplexes D-598701, D-598702, D-598703, and D-598704 have no C16 conjugate on their sense strands, while D-598705, D-598706, D-598707, and D-598708 have C16 conjugates bound to their sense strands. The target nucleobase sequence of human MTOR mRNA to which each siRNA complementarily binds (Ensembl transcript ID No. ENST00000361445.9) is shown in the target sequence column of Table 14 below, and the start point and end point indicate the nucleobase positions within the human MTOR transcript sequence (Ensembl transcript ID No. ENST00000361445.9) as provided by Ensembl.

**[Table 21]**

| duplex | Name of oligonucleotide | strand | Sequence of oligonucleotide | SE Q ID N O | Start point | End point | Target sequence (SEQ ID NO) |
|---|---|---|---|---|---|---|---|
| D-598 701 | 598001 | sense | AUUCUAUCACUAG CGUGACTT | 45 6 | N/A | N/A | N/A |
| | 598002 | antis ense | GUCACGCUAGUGA UAGAAUTT | 45 7 | | | |
| D-598 702 | 598003 | sense | GAAUGUUGACCAA UGCUAUTT | 45 8 | 147, 717 | 147, 735 | GAATGTTGACC AATGCTAT (464) |
| | 598004 | antis ense | AUAGCAUUGGUCA ACAUUCTT | 45 9 | | | |
| D-598 703 | 598005 | sense | GGUCUGAGUUUAA GGUCUATT | 46 0 | 19,3 60 | 19,3 78 | GGTCTGAGTTTA AGGTCTA (465) |
| | 598006 | antis ense | UAGACCUUAAACU CAGACCTT | 46 1 | | | |
| D-598 704 | 598007 | sense | GCCGCAUUGUCUC UAUCAATT | 46 2 | 49,1 34 | 49,1 52 | GCCGCATTGTCT CTATCAA (466) |
| | 598008 | antis ense | UUGAUAGAGACAA UGCGGCTT | 46 3 | | | |
| D-598 705 | 598009 | sense | AUUCUAUCACUAG CGUGACTT | 45 6 | N/A | N/A | N/A |
| | 598002 | antis ense | GUCACGCUAGUGA UAGAAUTT | 45 7 | | | |
| D-598 706 | 598010 | sense | GAAUGUUGACCAA UGCUAUTT | 45 8 | 147, 717 | 147, 735 | GAATGTTGACC AATGCTAT (464) |
| | 598004 | antis ense | AUAGCAUUGGUCA ACAUUCTT | 45 9 | | | |
| D-598 707 | 598011 | sense | GGUCUGAGUUUAA GGUCUATT | 46 0 | 19,3 60 | 19,3 78 | GGTCTGAGTTTA AGGTCTA (465) |
| | 598006 | antis ense | UAGACCUUAAACU CAGACCTT | 46 1 | | | |
| D-598 708 | 598012 | sense | GCCGCAUUGUCUC UAUCAATT | 46 2 | 49,1 34 | 49,1 52 | GCCGCATTGTCT CTATCAA (466) |

SNB-19 and SK-N-AS cells, cultured at densities of 8,000 cells/well and 20,000 cells/well, respectively, were transfected with 0.3, 3, and 30 nM concentrations of unmodified siRNA and C16 conjugated siRNA using Lipofectamine^{™} RNAiMAX Transfection Reagent (Thermo Fisher Scientific). Total RNA was extracted from the cells after 24 hours of treatment.

The mRNA expression level of the MTOR gene for RPL13, one of the housekeeping genes, was measured by quantitative real-time PCR. The mRNA expression levels were measured using the human RPL13 primer-probe set and human MTOR primer-probe set 3 used in Example 1 above.

**[Table 22]**

| Compound | SNB-19 | | | SK-N-AS | | |
|---|---|---|---|---|---|---|
| Duplex | 0.3nM | 3nM | 30nM | 0.3nM | 3nM | 30nM |
| D-598701 | 1.23 | 1.10 | 0.96 | 0.90 | 0.89 | 0.95 |
| D-598702 | 0.52 | 0.35 | 0.29 | 0.41 | 0.36 | 0.28 |
| D-598703 | 0.45 | 0.29 | 0.27 | 0.35 | 0.26 | 0.28 |
| D-598704 | 0.46 | 0.36 | 0.25 | 0.37 | 0.27 | 0.21 |
| D-598705 | 1.09 | 1.27 | 0.82 | 0.89 | 0.73 | 0.77 |
| D-598706 | 0.48 | 0.35 | 0.35 | 0.24 | 0.47 | 0.24 |
| D-598707 | 0.57 | 0.45 | 0.36 | 0.34 | 0.31 | 0.25 |
| D-598708 | 0.80 | 0.58 | 0.41 | 0.51 | 0.44 | 0.32 |

The experimental results show the relative expression levels of MTOR mRNA compared to untreated control cells. As shown in Table 22, MTOR mRNA levels were reduced in a dose-dependent manner in modified siRNA-treated cells.

### Example 11: Tolerability of modified oligonucleotides complementary to human MTOR at a dose of 600 µg in mice

The modified oligonucleotide used in this experiment had the mixed backbone prepared in Example 3, with the cytosine residue modified to 5-mC, and was a 5-10-5 2'-MOE gapmer. This gapmer was 20 nucleosides in length, wherein the central gap segment consists of ten(10) 2'-deoxynucleosides, and the 3' and 5' wings each consist of five (5) 2'-MOE nucleosides. The tolerability of the oligonucleotide was evaluated by administering the modified oligonucleotide described above to mice.

Specifically, wild-type C57/Bl6 mice were each administered a single dose of 600 µg of the oligonucleotides listed in the table below via intracerebroventricular intraventricular administration. Each treatment group consisted of three mice. A group of mice administered DPBS served as a negative control. Three hours after administration, mice were evaluated according to seven criteria, namely, whether the mouse: (1) was bright, alert, and responsive; (2) Standing or hunched without stimulation; (3) showed any movement without stimuli; (4) Demonstrated forward movement after it was lifted; (5) demonstrated any movement after it was lifted; (6) Responded to tail pinching; and (7) exhibited regular breathing. For each criterion, a sub-score of 0 was assigned if the mouse exhibited normal behavior for that criterion, and a sub-score of 1 was assigned otherwise. The sub-scores for all seven criteria were then summed for each mouse to yield a Functional Observational Battery (FOB) score, and the FOB scores were averaged within each treatment group. The results are presented in Table 23 below. "n.d." indicates that no measurement was obtained because the administered subject died.

**[Table 23]**

| Compound ID | score | Compound ID | score |
|---|---|---|---|
| DPBS | 0 | 574065 | 3 |
| 574001 | 2.3 | 574066 | 0 |
| 574003 | 1.5 | 574067 | 0 |
| 574004 | 1.5 | 574071 | 1 |
| 574006 | 0.5 | 574072 | 0 |
| 574007 | 0 | 574078 | 6 |
| 574022 | n.d. | 574100 | 2.7 |
| 574023 | 6 | 574101 | 2.7 |
| 574024 | 5.3 | 574102 | 1.7 |
| 574025 | 4.7 | 574106 | 1.3 |
| 574029 | 5.3 | 574107 | 0 |
| 574030 | 3 | 574108 | 0 |
| 574031 | 2.3 | 574109 | 0.3 |
| 574044 | 3.3 | 574110 | 0.7 |
| 574050 | 6 | 574111 | 0 |
| 574051 | 6 | 574112 | 0.3 |
| 574064 | 3 | 574117 | 0.7 |

Using brain tissue collected at necropsy 8 weeks after intracerebroventricular (ICV) administration, mRNA levels of Iba1 (a microglial marker) and Gfap (an astrocytic marker) were measured in the cerebellum. Because both Iba1 and Gfap are CNS inflammatory markers, elevated levels of either marker indicate lower tolerability of the oligonucleotide in mice. The mRNA expression levels of the Iba1 and Gfap genes were measured relative to Hprt, a housekeeping gene, by quantitative real-time PCR. mRNA levels were measured using the mouse Hprt primer set (Forward Primer Sequence: TCAGTCAACGGGGGACATAAA (SEQ ID NO: 476); Reverse Primer Sequence: GGGGCTGTACTGCTTAACCAG (SEQ ID NO: 477)), the Gfap primer set (Forward Primer Sequence: AACCTGGCTGCGTATAGA (SEQ ID NO: 478); Reverse Primer Sequence: CGAACTTCCTCCTCATAGAT (SEQ ID NO: 479)), and the Iba1 primer set (Forward Primer Sequence: ATCAACAAGCAATTCCTC (SEQ ID NO: 480); Reverse Primer Sequence: ATATCTCCATTTCCATTCAG (SEQ ID NO: 481)). "n.d." indicates that no measurement was obtained because the administered subject died prior to the measurement.

**[Table 24]**

| Compound ID | Cerebellum Gfap | Cerebellum Iba1 | Compound ID | Cerebellum Gfap | Cerebellum Iba1 |
|---|---|---|---|---|---|
| DPBS | 1 | 1 | 574065 | 2.28 | 1.72 |
| 574001 | 0.79 | 1.02 | 574066 | 1.33 | 1.29 |
| 574003 | 0.91 | 0.87 | 574067 | 1.74 | 1.75 |
| 574004 | 1.32 | 1.54 | 574071 | 4.37 | 3.82 |
| 574006 | 0.87 | 1.32 | 574072 | 4.1 | 3.62 |
| 574007 | 0.82 | 1.16 | 574078 | 0.79 | 0.69 |
| 574022 | n.d. | n.d. | 574100 | 0.92 | 1.13 |
| 574023 | n.d. | n.d. | 574101 | 1.12 | 1.45 |
| 574024 | 0.76 | 0.69 | 574102 | 1.03 | 1.3 |
| 574025 | 0.98 | 0.69 | 574106 | 0.87 | 1.3 |
| 574029 | 0.84 | 0.55 | 574107 | 0.86 | 1.13 |
| 574030 | 0.78 | 0.6 | 574108 | 0.97 | 1.12 |
| 574031 | 0.77 | 0.62 | 574109 | 0.95 | 1.11 |
| 574044 | 1.86 | 1.39 | 574110 | 0.88 | 1.04 |
| 574050 | 1.04 | 0.86 | 574111 | 1.14 | 1.39 |
| 574051 | 0.73 | 0.77 | 574112 | 0.97 | 1.1 |
| 574064 | 1.48 | 1.33 | 574117 | 0.91 | 1.12 |

### Example 12: Tolerability of modified oligonucleotides complementary to human MTOR at a dose of 3 mg in rats

The tolerability of the modified oligonucleotides described above was evaluated by testing them in rats. Sprague-Dawley rats each received a single intrathecal injection of 3 mg of the oligonucleotides listed in the table below. Each treatment group consisted of three rats, and a group receiving PBS served as a negative control. At 3 hours and 8 weeks after administration, gait and alertness were evaluated for each rat.

For the gait evaluation, each animal was assigned a score according to the following criteria: -2 points for ataxic gait or excessive swaying; -1 point for dragging of the limbs or splayed limbs; 0 points for normal gait with body support on all four limbs; 1 point for walking on the toes; and 2 points for walking with a hunched back. For the alertness evaluation, each animal was assigned a score according to the following criteria: 1 point for anesthesia or coma; 2 points for markedly reduced alertness, with only slight movement of the head or body; 3 points for reduced alertness, with periods of immobility and slight exploratory walking; 4 points for a normal state, with alertness and exploratory walking behavior; 5 points for increased alertness, with excitement, tension, sudden dashing, or rigidity; and 6 points for markedly increased alertness, with high vigilance and excitement and running around the surroundings. The scores assigned in the gait and alertness evaluations were summed for each mouse and averaged within each treatment group. The results are presented in Table 25.

**[Table 25]**

| Compound ID | Gait status for 3 hours | Arousal status for 3 hours | Gait status for 8 weeks | Arousal status for 8 weeks |
|---|---|---|---|---|
| DPBS | 0 | 4 | 0 | 4 |
| 574001 | -0.7 | 2.7 | 0 | 4 |
| 574004 | -0.7 | 4 | 0 | 4 |
| 574007 | -0.7 | 3.7 | 0 | 4 |
| 574031 | -1 | 3 | 0 | 4 |
| 574066 | -0.7 | 3.3 | 0 | 4 |
| 574106 | 0 | 4 | 0 | 4 |
| 574107 | 0 | 4 | 0 | 4 |
| 574108 | 0 | 4 | 0 | 4 |
| 574109 | 0 | 4 | 0 | 4 |
| 574110 | 0 | 4 | 0 | 4 |

Using brain tissue collected at necropsy 8 weeks after intrathecal (IT) administration, mRNA levels of Iba1 (a microglial marker) and Gfap (an astrocytic marker) were measured in the cerebral cortex and the cerebellum. Because both Iba1 and Gfap are CNS inflammatory markers, elevated levels of either marker indicate lower tolerability of the oligonucleotide in rat. The mRNA expression levels of the Iba1 and Gfap genes were measured relative to Gapdh, a housekeeping gene, by quantitative real-time PCR. mRNA levels were measured using the rat Gapdh primer-probe set (forward primer sequence: TGCTCCTCCCTGTTCTAGAGACA (SEQ No. 482), reverse primer sequence: CACCGACCTTCACCATCTTGT (SEQ No. 483), probe sequence: CCGCATCTTCTTGTGCAGTGCCAG (SEQ No. 484)), the Gfap primer-probe set (Thermo Fisher, Assay ID Rn01253033_m1), and the rat Iba1 primer-probe set (forward primer sequence: AGGAGAAAAACAAAGAACACCAGAA (SEQ No. 485), reverse primer sequence: CAATTAGGGCAACTCAGAAATAGCT (SEQ No. 486), probe sequence: CCAACTGGTCCCCCAGCCAAGA (SEQ No. 487)). The results are presented in Table 26 below.

**[Table 26]**

| Compound ID | Cerebral cortex Gfap | Cerebral cortex Iba1 | cerebellum Gfap | cerebellum Iba1 |
|---|---|---|---|---|
| DPBS | 1.00 | 1.00 | 1.00 | 1.00 |
| 574001 | 1.32 | 1.29 | 1.05 | 1.64 |
| 574004 | 1.1 | 1.12 | 0.88 | 0.9 |
| 574007 | 0.69 | 0.92 | 0.68 | 0.81 |
| 574031 | 1.13 | 1.29 | 0.84 | 1.13 |
| 574066 | 0.98 | 1.23 | 0.59 | 0.98 |
| 574106 | 0.97 | 1.13 | 1.02 | 1.66 |
| 574107 | 0.93 | 1.41 | 0.61 | 1.2 |
| 574108 | 0.78 | 1.38 | 0.77 | 1.04 |
| 574109 | 0.66 | 0.98 | 0.67 | 1.03 |
| 574110 | 0.98 | 1.49 | 0.63 | 1.2 |

### Example 13: Inhibition of MTOR in SH-SY5Y Cells by 5-10-5 MOE gapmers having mixed backbone chemistry (multiple doses):

The modified oligonucleotides of the Example were tested at various concentrations in SH-SY5Y human cells. Cells were seeded at a density of 3,700 cells per well and transfected by electroporation with modified oligonucleotides at concentrations of 0.02, 0.06, 0.19, 0.56, 1.67, 5, and 15 µM. After 24 hours of treatment, total RNA was extracted from the cells.

The mRNA expression level of the MTOR gene were measured relative to RPL13, one of the housekeeping genes, was measured by quantitative real-time PCR. The mRNA levels were measured using the human RPL13 primer-probe set and human MTOR primer-probe set 3 used in Example 1.

The results in Table 27 show the relative MTOR mRNA levels at 15 µM as compared with untreated control cells and the corresponding IC₅₀ value. The IC₅₀ was calculated using the "absolute IC₅₀" formula with Prism10 software.

**[Table 27]**

| Compound ID | 15µM | IC₅₀(µM) |
|---|---|---|
| 574001 | 0.17 | 0.83 |
| 574004 | 0.11 | 0.88 |
| 574007 | 0.16 | 0.66 |
| 574031 | 0.15 | 1.35 |
| 574066 | 0.06 | 0.48 |
| 574106 | 0.16 | 0.43 |
| 574107 | 0.12 | 0.53 |
| 574108 | 0.14 | 0.57 |
| 574109 | 0.17 | 0.43 |
| 574110 | 0.13 | 0.39 |

### Example 14: Inhibition of MTOR in human neuronal cells by 5-10-5 MOE gapmers having mixed backbone chemistry (multiple doses)

Selected modified oligonucleotides complementary to human MTOR were tested for their effects on MTOR mRNA levels in human neuronal cells via in vitro by free uptake. Human embryonic stem cell (ESC)-derived neuronal cells were seeded at a density of 30,000 cells per well. The cultured human neuronal cells were treated with modified oligonucleotides at concentrations of 0.39, 0.78, 1.56, 3.12, 6.25, 12.5, 25, and 50 µM for 3 days. After 3 days, the medium was replaced with modified oligonucleotide-free medium,, and 6 days later, total RNA was extracted from the cells.

The mRNA expression level of the MTOR gene was measured relative to HPRT, one of the housekeeping genes, by quantitative real-time PCR. The mRNA level was measured using the human HPRT primer-probe set and human MTOR primer-probe set 1 used in Example 1 above.

The results in Table 28 show the relative MTOR mRNA levels at 15 µM as compared with untreated control cells and the corresponding IC₅₀ value. The IC₅₀ was calculated using the "absolute IC₅₀" formula with Prism10 software.

**[Table 28]**

| Compound ID | 15µM | IC₅₀(µM) |
|---|---|---|
| 574007 | 0.24 | 1.78 |
| 574107 | 0.20 | 1.77 |
| 574109 | 0.27 | 2.07 |

### Example 15: Inhibition of MTOR expression and activity in human neurons harboring MTOR mutation by modified oligonucleotides complementary to human MTOR

To determine whether modified oligonucleotides complementary to human MTOR inhibit MTOR expression and activity in a hyperactive MTOR signaling pathway, human embryonic stem cell (ESC)-derived neuronal cells harboring the MTOR p.Leu2427Pro mutation found in patients with FCD type II were utilized. Human embryonic stem cells, genome-edited using CRISPR/Cas9 technology to introduce the MTOR p.Leu2427Pro (c.7280T>C) mutation, were differentiated into neuronal cells and seeded at a density of 400,000 cells/well. Cultured human neuronal cells were treated with modified oligonucleotides (Compound IDs 574120, 574007, 574107, and 574109 in Table 30 below) at concentrations of 5 µM, 10 µM, and 50 µM for 3 days. After 3 days, the medium was replaced with oligonucleotide-free medium, and protein was extracted from the cells 21 days after the medium change. Compound ID 574120, a non-targeting oligonucleotide presented in Table 29, was used as a negative control.

**[Table 29]**

| Compound ID | SEQ ID NO | Sequence | Sugar moiety | Internucleoside linkage |
|---|---|---|---|---|
| 574120 | 449 | CCTATAGGACTATCCAGGA A | eeeeeddddddddddeeee e | SOOOOSSSSSSSSSSSOOS S |

As shown in FIG. 1, the expression level of MTOR protein (Cell Signaling Technology, Cat. No. 2983S) relative to α-tubulin (Cell Signaling Technology, Cat. No. 3873S), a housekeeping protein, was analyzed by Western blotting to assess MTOR expression. The expression level of p-4EBP1 protein (Cell Signaling Technology, Cat. No. 2855S) relative to total 4EBP1 protein (Cell Signaling Technology, Cat. No. 9644S) was also analyzed by Western blotting, as an indicator of MTOR signaling pathway activity.

The results in Tables 30 and 31 show the relative levels of MTOR protein and p-4EBP1 protein as compared to untreated control cells. As exemplified in the table below, MTOR protein and p-4EBP1 protein levels were reduced in a dose-dependent manner in cells treated with the modified oligonucleotides. "N/A" indicates that the corresponding condition was not evaluated.

**[Table 30]**

| Compound ID | Relative expression level of MTOR protein | | |
|---|---|---|---|
| | 5µM | 10µM | 50µM |
| 574120 | N/A | N/A | 1.07 |
| 574007 | 0.48 | N/A | 0.20 |
| 574107 | 0.52 | 0.41 | 0.18 |
| 574109 | 0.48 | 0.29 | 0.14 |

**[Table 31]**

| Compound ID | Relative expression level of p-4EBP1/4EBP1 | | |
|---|---|---|---|
| | 5µM | 10µM | 50µM |
| 574120 | N/A | N/A | 0.91 |
| 574007 | 0.73 | N/A | 0.33 |
| 574107 | 0.52 | 0.52 | 0.33 |
| 574109 | 0.58 | 0.45 | 0.38 |

### Example 16: Evaluation of Efficacy of 5-10-5 2'-MOE gapmers in human FCD Type II neurospheres

The modified oligonucleotide used in this study is the 5-10-5 2'-MOE gapmer having PS internucleoside linkages and unmodified nucleobases, as prepared in Example 1, in which the cytosine residues were further modified to 5-methylcytosine. This gapmer was 20 nucleosides in length, wherein the central gap segment consists of ten(10) 2'-deoxynucleosides, and the 3' and 5' wings each consist of five (5) 2'-MOE nucleosides.

Neuronal spheres were differentiated over a total of 42 days from stem cells harboring the MTOR mutation (c.7280T>C) associated with FCD type II, as well as from normal stem cells. Embryoid bodies (EB) were treated with 10 µM SB431542 and 5 µM dorsomorphin for 4 days, followed by induction of differentiation into early neuroectoderm and subsequently into neural rosettes (NR) over 8 days. Only neural rosettes were selected and differentiated into three-dimensional neural precursor cells (NPCs) over 9 days. The three-dimensional neural precursor cells were then cultured in a differentiation medium capable of inducing maturation into neuronal cells, yielding neuronal spheroids after 21 days.

Total RNA was extracted from the differentiated wild-type and MTOR-mutant neural spheres following treatment with the modified oligonucleotides described above, at concentrations of 2 µM and 5 µM, by free uptake for 10 days. The mRNA expression levels of the MTOR gene relative to RPL13, a housekeeping gene, were quantified by quantitative real-time PCR (qRT-PCR) based on the TaqMan method. Quantification was performed using the human RPL13 primer-probe set described in Example 1 and the human MTOR primer-probe set (Thermo Fisher Scientific, Cat. No. 4331182, Assay ID: Hs00234508_m1). The relative MTOR mRNA expression levels were analyzed by comparison with the PBS-treated group of wild-type-derived neural spheres not treated with the modified oligonucleotides. The relative MTOR mRNA expression levels resulting from this analysis are shown in Table 32 below. In Table 32, "n.d." indicates that the inhibition level was not determined, and "WT" neuronal cell indicates wild type neuronal cell.

**[Table 32]**

| Compound ID | SEQ ID NO | WT neuronal cell | | | FCD type II neuronal cell | | |
|---|---|---|---|---|---|---|---|
| | | 0µM | 2µM | 5µM | 0µM | 2µM | 5µM |
| 534442 | 12 | 1.00 | 0.47 | 0.34 | 0.94 | 0.47 | 0.36 |
| 534455 | 398 | 1.00 | 0.27 | n.d. | 0.94 | 0.29 | n.d. |

### Example 17: Efficacy of modified oligonucleotides complementary to human MTOR against abnormal neuronal firing in human neurons harboring an MTOR mutation

Similar to the epilepsy observed in patients with mTORopathies such as FCD type II and TSC, the firing rate in human neuronal cells with a hyperactivated MTOR signaling pathway is abnormally elevated. Accordingly, using human embryonic stem cell-derived neuronal cells harboring the MTOR p.Leu2427Pro mutation used in Example [X] above, the weighted mean firing rate (WMFR) and burst frequency were analyzed using a multi-electrode array (MEA) system, in order to confirm the efficacy of modified oligonucleotides complementary to human MTOR.

On day 3 of differentiation from human embryonic stem cells into neuronal cells, 100,000 human neuronal cells and 16,700 rat cortical astrocytes were co-cultured per well in a CytoView MEA 48-well plate (M768-tMEA-48W; Axion Biosystems), and the medium was changed every 3-4 days. On day 27 of differentiation, neuronal cells were treated with the modified oligonucleotides (Compound IDs 574120, 574007, 574107, and 574109 in Table 30) at a concentration of 50 µM for 3 days, after which the medium was replaced with medium free of the modified oligonucleotides. Cells treated with PBS and with a non-targeting oligonucleotide (Compound ID 574120) were used as negative controls. The WMFR and burst frequency were analyzed by MEA system recording every 3-4 days from day 13 to day 48 of neuronal cell differentiation.

As shown in FIG. 2 and FIG. 3, the WMFR and burst frequency of MTOR p.Leu2427Pro mutant neurons, which were elevated relative to normal neurons on day 27 of differentiation, were decreased to levels comparable to normal neurons by day 48 of differentiation, following treatment with modified oligonucleotides complementary to human MTOR (Compound IDs 574007, 574107, and 574109).

### Example 18: Efficacy of modified oligonucleotides complementary to human MTOR against hypertrophic morphology in human neurons harboring MTOR mutation

It is well established that, in brain tissue from patients with FMCD (Focal Malformation of Cortical Development) caused by hyperactivation of the mTOR signaling pathway, such as in FCD type II, HME, and TSC, neurons with the mutations exhibit an enlarged neuronal size relative to normal neurons. To determine whether modified oligonucleotides complementary to human MTOR were effective against this hypertrophic morphology, human embryonic stem cell (ESC)-derived neuronal cells harboring the MTOR p.Leu2427Pro mutation found in patients with FCD type II were utilized.

Human embryonic stem cells harboring the MTOR p.Leu2427Pro (c.7280T>C) mutation, introduced using CRISPR/Cas9 technology, were differentiated into neuronal cells and seeded at a density of 140,000 cells/well. The cultured human neuronal cells were treated with the modified oligonucleotides (Compound IDs 574120, 574007, 574107, and 574109 in Table 30) at a concentration of 50 µM for 4 days. Cells treated with PBS and with the non-targeting oligonucleotide (NT ASO; Compound ID 574120) were used as negative control groups.

After 4 days, the medium was replaced with medium free of the modified oligonucleotides, and 10 days thereafter, the cells were immunostained for the neuronal marker MAP2 (Sigma, M1406) to analyze cell soma size. For each test group, the soma size of 51 to 81 neurons was measured.

As shown in FIG. 4, the results demonstrated that the hypertrophic morphology, which was increased relative to normal neurons, was restored to normal levels following treatment with modified oligonucleotides complementary to human MTOR (Compound IDs 574007, 574107, and 574109).

### Example 19: Inhibition of MTOR in non-human primate cell line CO31 by 5-10-5 MOE gapmers with mixed backbone chemistry (multiple doses)

The modified oligonucleotides described above are complementary to the Cynomolgus monkey MTOR gene sequence. The modified oligonucleotides were tested at various concentrations in CO31 Cynomolgus monkey fibroblast cells. Cells were seeded at a density of 12,000 cells/well and transfected by electroporation with the modified oligonucleotides at concentrations of 0.02, 0.06, 0.19, 0.56, 1.67, 5, and 15 µM. After 24 hours of treatment, total RNA was extracted from the cells.

The mRNA expression level of the MTOR gene was measured relative to RPL13, a housekeeping gene, by quantitative real-time PCR (qRT-PCR). mRNA levels were measured using the RPL13 primer-probe set and MTOR primer-probe set 3 used in Example 1 above.

The results in Table 33 show the relative MTOR mRNA levels and IC₅₀ values at 15 µM, as compared with untreated control cells, and the corresponding IC₅₀ values. IC₅₀ values were calculated using the absolute IC₅₀ formula in GraphPad Prism, version 10 (GraphPad Software).

**[Table 33]**

| Compound ID | 15µM | IC₅₀ (µM) |
|---|---|---|
| 574001 | 0.18 | 1.70 |
| 574004 | 0.17 | 1.46 |
| 574007 | 0.08 | 1.65 |
| 574031 | 0.16 | 1.80 |
| 574066 | 0.06 | 1.24 |
| 574106 | 0.22 | 0.92 |
| 574107 | 0.18 | 0.75 |
| 574108 | 0.14 | 1.01 |
| 574109 | 0.24 | 1.04 |
| 574110 | 0.21 | 0.76 |

### Example 20: Potency of modified oligonucleotides complementary to human MTOR in non-human primates

The modified oligonucleotides described above were further evaluated for potency in non-human primates (NHPs). For each compound, two Cynomolgus monkeys received an intrathecal bolus injection of 40 mg of the respective compound on Day 1 and Day 29 of the study. Two weeks after the Day 29 administration, the relative MTOR mRNA expression levels in the cerebral cortex and spinal cord regions were analyzed.

The mRNA expression level of the MTOR gene was measured relative to UBC, a housekeeping gene, by quantitative real-time PCR (qRT-PCR). mRNA levels were measured using the non-human primate UBC primer-probe set (Thermo Fisher, Assay ID: Mf02798368_m1) and the non-human primate MTOR primer-probe set (Thermo Fisher, Assay ID: Mf01042402_m1). Brain tissue from untreated monkeys was used as a control, and the relative MTOR mRNA expression levels are shown in Table 34 below. As shown in the table below, the modified oligonucleotides reduced MTOR mRNA levels in the cerebral cortex and spinal cord regions as compared to the control.

**[Table 34]**

| Compound ID | Frontal cortex | Motor cortex | Temporal cortex | Lumbar spinal cord |
|---|---|---|---|---|
| 574007 | 0.36 | 0.40 | 0.34 | 0.42 |
| 574107 | 0.41 | 0.49 | 0.39 | 0.31 |
| 574109 | 0.32 | 0.49 | 0.31 | 0.13 |

## Claims

1. An oligomeric compound for modulating the activity and/or expression level of MTOR, comprising a modified oligonucleotide comprising 8 to 80 linked nucleosides, wherein the modified oligonucleotide is complementary to an MTOR nucleic acid sequence, wherein the nucleic acid sequence of the modified oligonucleotide has at least 80% sequence identity to a sequence complementary to target region within an MTOR transcript, and wherein the modified oligonucleotide comprises at least one modification selected from the group consisting of a modified sugar moiety, a modified internucleoside linkage and a modified nucleobase.

2. The oligomeric compound of claim 1, wherein the target region comprises a nucleic acid sequence selected from the group consisting of SEQ ID NOs: 440 to 446.

3. The oligomeric compound of claim 2, wherein the nucleic acid sequence of the modified oligonucleotide comprises 12, 13, 14, 15, 16, 17, 18, 19, 20, 21, or 22 contiguous nucleobases from a nucleic acid sequence of SEQ ID NOs: 1 to 19, SEQ ID NOs: 27 to 36, SEQ ID NOs: 68 to 81, SEQ ID NOs: 396 to 403, or SEQ ID NOs: 414 to 417, and wherein the modified oligonucleotide comprises at least one modification selected from the group consisting of a modified sugar moiety, a modified internucleoside linkage and a modified nucleobase.

4. The oligomeric compound of claim 1, wherein the nucleic acid sequence of the modified oligonucleotide comprises 12, 13, 14, 15, 16, 17, 18, 19, 20, 21, or 22 contiguous nucleobases from a nucleic acid sequence of SEQ ID NOs: 447 to 453, and wherein the modified oligonucleotide comprises at least one modification selected from the group consisting of a modified sugar moiety, a modified internucleoside linkage and a modified nucleobase.

5. The oligomeric compound of claim 1, wherein the nucleic acid sequence of the modified oligonucleotide comprises an oligonucleotide consisting of 14 to 80, for example 14 to 30, or 14 to 22, specifically 14, 15, 16, 17, 18, 19, 20, 21, or 22 contiguous linked nucleosides comprising the nucleic acid sequence of SEQ ID NO: 454, and wherein the modified oligonucleotide comprises at least one modification selected from the group consisting of a modified sugar moiety, a modified internucleoside linkage and a modified nucleobase.

6. The oligomeric compound of claim 1, wherein the nucleic acid sequence of the modified oligonucleotide comprises an oligonucleotide consisting of 9 to 80, for example 12 to 30, or 12 to 22, specifically 12, 13, 14, 15, 16, 17, 18, 19, or 20 contiguous linked nucleosides comprising the nucleic acid sequence of SEQ ID NO: 455, and wherein the modified oligonucleotide comprises at least one modification selected from the group consisting of a modified sugar moiety, a modified internucleoside linkage and a modified nucleobase.

7. The oligomeric compound of claim 2, wherein the nucleic acid sequence of the modified oligonucleotide comprises 12, 13, 14, 15, 16, 17, 18, 19, or 20 contiguous nucleobase selected from at least a nucleic acid sequence which is selected from the group consisting of SEQ ID NOs: 10, 11, 12, 13, 14, 28, 32, 35, 71, 396, 397, 398, 399, 400, 401, 402, 403, 414, and 416.

8. The oligomeric compound of claim 2, wherein the nucleic acid sequence of the modified oligonucleotide is SEQ ID NO: 10, 11, 12, 13, or 14.

9. The oligomeric compound of any one of claims 1 to 8, wherein the modified oligonucleotide comprises:
a 5'-wing region consisting of 1 to 7 linked 5'-region nucleosides;
a central gap region consisting of 6 to 10 linked central region nucleosides; and
a 3'-wing region consisting of 1 to 7 linked 3'-region nucleosides.

10. The oligomeric compound of any one of claims 1 to 9, wherein the modified oligonucleotide comprises at least one modified nucleoside comprising a modified sugar moiety.

11. The oligomeric compound of any one of claims 1 to 10, wherein the modified oligonucleotide comprises at least one modified nucleoside comprising a modified bicyclic sugar moiety having a 2'-4' bridge and at least one modified nucleoside comprising a modified non-bicyclic sugar moiety.

12. The oligomeric compound of any one of claims 1 to 11, wherein the modified sugar moiety comprises at least one selected from the group consisting of:
a modified non-bicyclic sugar moiety including a 2'-MOE sugar moiety and a 2'-OMe sugar moiety; and
a modified bicyclic sugar moiety including a 2',4'-bridge selected from -O-CH₂- and -O-CH(CH₃)-.

13. The oligomeric compound of claim 11, wherein the modified oligonucleotide comprises a 5'-wing region, a central gap region, and a 3'-wing region, wherein at least one nucleoside selected from the group consisting of the 5'-wing and 3'-wing comprises a modified bicyclic sugar moiety and a non-modified bicyclic sugar moiety, and wherein the modified bicyclic sugar moiety is a modified bicyclic sugar moiety having a 2'-4' bridge.

14. The oligomeric compound of any one of claims 1 to 13, wherein the modified oligonucleotide comprises at least one modified internucleoside linkage.

15. The oligomeric compound of any one of claims 1 to 14, wherein the modified oligonucleotide comprises at least one internucleoside linkage selected from the group consisting of a phosphorothioate internucleoside linkage, a mesylphosphoramidate internucleoside linkage, and a phosphodiester internucleoside linkage.

16. The oligomeric compound of any one of claims 1 to 15, wherein the modified oligonucleotide comprises a modified bicyclic sugar moiety comprising a 2',4'-bridge and a 2'-MOE sugar moiety in the nucleic acid sequence of SEQ ID NO: 13, 14, 106, 409, or 416, and at least one modified internucleoside linkage comprises a phosphorothioate internucleoside linkage.

17. The oligomeric compound of any one of claims 1 to 15, wherein the modified oligonucleotide comprises at least one 2'-MOE sugar moiety in the nucleic acid sequence of SEQ ID NO: 11, 12, 13, 14, 28, 95, 102, 105, 106, 319, 396, 398, 399, 403, 409, 414, or 416, and comprises at least one internucleoside linkage selected from the group consisting of phosphorothioate internucleoside linkage and phosphodiester internucleoside linkage.

18. The oligomeric compound of any one of claims 1 to 17, wherein the modified oligonucleotide comprises a modified nucleobase.

19. The oligomeric compound of claim 18, wherein the modified nucleobase is 5-methylcytosine.

20. The oligomeric compound of claim 13, wherein each nucleoside of the central gap region is a 2'-β-D-deoxynucleoside.

21. The oligomeric compound of any one of claims 1 to 20, further comprising a conjugate group.

22. A composition for reducing or inhibiting an activity or expression level of MTOR, comprising the oligomeric compound of any one of claims 1 to 21.

23. A pharmaceutical composition for the prevention, amelioration, or treatment of a disease or disorder associated with MTOR, comprising the oligomeric compound of any one of claims 1 to 22.

24. The pharmaceutical composition of claim 23, wherein the composition further comprises a pharmaceutically acceptable carrier or diluent.

25. The pharmaceutical composition of claim 24, wherein the pharmaceutically acceptable diluent is artificial cerebrospinal fluid (aCSF) or phosphate-buffered saline (PBS).

26. The pharmaceutical composition of any one of claims 23 to 25, wherein the disease or disorder associated with MTOR is cancer, renal disease, a neurodevelopmental disorder, or a neurodegenerative disease.

27. The pharmaceutical composition of claim 26, wherein the disease or disorder is Focal Cortical Dysplasia (FCD), Hemimegalencephaly (HME), Tuberous Sclerosis Complex (TSC), Megalencephaly (MEG), Cowden Syndrome, PIK3CA-Related Overgrowth Spectrum (PROS), Bannayan-Riley-Ruvalcaba Syndrome (BRRS), Lhermitte-Duclos Disease (LDD), MCAP (Megalencephaly-Polydactyly-Polymicrogyria-Hydrocephalus), MPPH (Megalencephaly-Polymicrogyria-Hydrocephalus), Smith-Kingsmore Syndrome (SKS), Polyhydramnios, Megalencephaly, and Symptomatic Epilepsy Syndrome (PMSE), Familial Intellectual Disability-Macrocephaly Syndrome, Proteus Syndrome, Fragile X Syndrome (FXS), Autism Spectrum Disorder (ASD), Familial Focal Epilepsy with Variable Foci (FFEVF), Autosomal Dominant Nocturnal Frontal Lobe Epilepsy (ADNFLE), Familial Mesial Temporal Lobe Epilepsy (FMTLE), Temporal Lobe Epilepsy (TLE), Infantile-Onset Developmental and Epileptic Encephalopathies (DEE), Alzheimer's Disease (AD), Parkinson's Disease (PD), Huntington's Disease (HD), Amyotrophic Lateral Sclerosis (ALS), Lung Cancer, Colorectal Cancer, Gastric Cancer, Renal Cell Carcinoma, Bladder Cancer, Prostate Cancer, Breast Cancer, Ganglioma, Glioblastoma, Dysembryoplastic Neuroepithelial Tumor (DNET), Subependymal Giant Cell Astrocytomas (SGCAs/SEGAs), Polycystic Kidney Disease (PKD), or seizures or epilepsy associated with these diseases or disorders.
